# EUROPEAN PATENT SPECIFICATION

(11) **EP 2 925 780 B1**
(45) Date of publication and mention of the grant of the patent: **18.10.2017**
(21) Application number: 13795771.8
(22) Date of filing: 26.11.2013
(51) Int. Cl.: C07K 16/28, C07K 16/30, A61K 47/00, C07K 16/46, C07K 14/74, C07K 14/045, A61K 39/00

(54) **REMOVAL OF CANCER CELLS BY CIRCULATING VIRUS-SPECIFIC CYTOTOXIC T-CELLS USING CANCER CELL TARGETED MHC CLASS I COMPRISING MULTI-FUNCTION PROTEINS**
BESEITIGUNG VON KREBSZELLEN DURCH ZIRKULATION VIRUSSPEZIFISCHER CYTOTOXISCHER T-ZELLEN UNTER VERWENDUNG VON AUF KREBSZELLEN GERICHTETE MHC-KLASSE-I MIT MULTIFUNKTIONELLEN PROTEINEN
ÉLIMINATION DE CELLULES CANCÉREUSES PAR LA CIRCULATION DE LYMPHOCYTES T CYTOTOXIQUES SPÉCIFIQUES DU VIRUS À L'AIDE DE CELLULES CANCÉREUSES CIBLES EXPRIMANT LES MOLÉCULES DU CMH DE CLASSE I ET COMPRENANT DES PROTÉINES MULTIFONCTION

(30) Priority: 30.11.2012 EP 12195094
(43) Date of publication of application: 07.10.2015
(73) Proprietor: Roche Glycart AG, 8952 Schlieren (CH)
(72) Inventor: KNOETGEN, Hendrik, 79650 Schopfheim (DE); SCHMITTNAEGEL, Martina, 1004 Lausanne (CH); KLEIN, Christian, 8906 Bonstetten (CH); UMANA, Pablo, CH-8832 Wollerau (CH)
(74) Representative: Burger, Alexander
(86) International application number: PCT/EP2013/074759
(87) International publication number: WO 2014/083004

(56) References cited:
- WO-A1-2012/175508
- WO-A2-99/13095
- WO-A2-02/102299
- WO-A2-2005/099361
- US-A1- 2004 091 488
- KFIR OVED ET AL: "Antibody-mediated targeting of human single-chain class I MHC with covalently linked peptides induces efficient killing of tumor cells by tumor or viral-specific cytotoxic T lymphocytes", CANCER IMMUNOLOGY, IMMUNOTHERAPY, SPRINGER, BERLIN, DE, vol. 54, no. 9, 20 May 2005 (2005-05-20), pages 867-879, XP019333169, ISSN: 1432-0851, DOI: 10.1007/S00262-005-0666-5
- ROBERT B ET AL: "Redirecting anti-viral CTL against cancer cells by surface targeting of monomeric MHC class I-viral peptide conjugated to antibody fragments", CANCER IMMUNITY 20010330 CH, vol. 1, 30 March 2001 (2001-03-30), pages 1-13, XP002697163, ISSN: 1424-9634
- V. CESSON ET AL: "Active Antiviral T-Lymphocyte Response Can Be Redirected against Tumor Cells by Antitumor Antibody x MHC/Viral Peptide Conjugates", CLINICAL CANCER RESEARCH, vol. 12, no. 24, 15 December 2006 (2006-12-15), pages 7422-7430, XP055062873, ISSN: 1078-0432, DOI: 10.1158/1078-0432.CCR-06-1862
- ROBERT B ET AL: "ANTIBODY-CONJUGATED MHC CLASS I TETRAMERS CAN TARGET TUMOR CELLS FOR SPECIFIC LYSIS BY T LYMPHOCYTES", EUROPEAN JOURNAL OF IMMUNOLOGY, WILEY - V C H VERLAG GMBH & CO. KGAA, DE, vol. 30, no. 11, 1 November 2000 (2000-11-01), pages 3165-3170, XP001021944, ISSN: 0014-2980, DOI: 10.1002/1521-4141(200011)30:11<3165::AID-I MMU3165>3.0.CO;2-R cited in the application
- CLAUDIA BLUEMEL ET AL: "Epitope distance to the target cell membrane and antigen size determine the potency of T cell-mediated lysis by BiTE antibodies specific for a large melanoma surface antigen", CANCER IMMUNOLOGY, IMMUNOTHERAPY, SPRINGER, BERLIN, DE, vol. 59, no. 8, 23 March 2010 (2010-03-23) , pages 1197-1209, XP019842190, ISSN: 1432-0851
- TORISU-ITAKURA HITOE ET AL: "Redirected lysis of human melanoma cells by a MCSP/CD3-bispecific BiTE antibody that engages patient-derived T cells.", JOURNAL OF IMMUNOTHERAPY (HAGERSTOWN, MD. : 1997) OCT 2011, vol. 34, no. 8, October 2011 (2011-10), pages 597-605, XP009169634, ISSN: 1537-4513
- GRETEN TIM F ET AL: "Peptide-beta2-microglobulin-MHC fusion molecules bind antigen-specific T cells and can be used for multivalent MHC-Ig complexes", JOURNAL OF IMMUNOLOGICAL METHODS, ELSEVIER SCIENCE PUBLISHERS B.V.,AMSTERDAM, NL, vol. 271, 1 January 2002 (2002-01-01), pages 125-135, XP002693603, ISSN: 0022-1759 cited in the application
- R MOUS ET AL: "Redirection of CMV-specific CTL towards B-CLL via CD20-targeted HLA/CMV complexes", LEUKEMIA, vol. 20, no. 6, 23 March 2006 (2006-03-23) , pages 1096-1102, XP055062917, ISSN: 0887-6924, DOI: 10.1038/sj.leu.2404185

## Description

Herein is reported a multi-function protein comprising an antibody fragment and a MHC class I component and its use for removal of cancer cells by targeted attraction of circulating virus-specific cytotoxic T-cells.

### Background of the Invention

Melanoma chondroitin sulfate proteoglycan (MCSP) is a large transmembrane proteoglycan that is expressed in the majority of melanoma cancers. MCSP is also expressed on other cancers, including glioblastomas, osteosarcomas, chondrosarcomas, some types of ALL and AML, and in basal cell carcinomas. It serves as an early cell surface melanoma progression marker and is involved in stimulating tumor cell proliferation, metastasis, migration, invasion, and angiogenesis (see e.g. Staub, E., et al., FEBS Lett. 527 (2002) 114-118; Campoli, M., et al., Crit. Rev. Immun. 24 (2004) 267-296; Vergilis, I. J., J. Invest. Dermatol. 125 (2005) 526-531; Yang, J., J. Chem. Biol. 165 (2004) 881-891; Luo, W., J. Immunol. 176 (2006) 6046-6054).

The MHC Class I protein consists of an α-chain (α-1 to 3 and a transmembrane domain) and β2-microglobulin. It is polygenic (3 gene loci for MHC-class I protein in the haploid genome) giving rise to six different MHC class I protein α-chains (in humans two HLA-A, two HLA-B, two HLA-C). The MHC is further polymorphic. The human HLA-A allele A*0201 is prevalent in about 30 % to 50 % of the caucasian population (see e.g. Player, M.A., et al., J Immunother. Emphasis Tumor Immunol. 19 (1996) 357-363).

Human cytomegalovirus huCMV (= human herpesvirus 5, HHV-5) is one of the largest human viruses. Its genome comprises around 230,000 bp linear double stranded DNA and encodes more than 160 proteins (see e.g. Davison, A.J., et al., J. Gen. Virol. 84 (2003) 17-28).

The CMV has evolved to become a sublime parasite of the human genome and it is a potent immunogen and triggers strong immune responses from all arms of the immune system. This virus appears to be among the most immunodominant antigens known to the human immune system and stimulates CD8⁺-T-cell responses of unprecedented magnitude.

The CMV "latency" depends on chronic immune suppression of CMV viruses rather than a change in the pattern of viral transcription (see e.g. Moss & Khan, Human Immunology 65 (2004) 456-464).

CD8⁺-T-cell immune responses are not directed evenly against all CMV proteins but are focused. The CMV proteins pp65 and IE-1 are the predominant targets (see e.g. McLaughlin-Taylor, E., et al., J. Med. Virol. 43 (1994) 103-110; Moss & Khan, Human Immunology 65 (2004) 456-464).

The frequency of CMV-specific T-cells is very high with frequencies for individual peptides in the order of up to 1 to 2 % of the total CD8⁺-T-cell repertoire (see e.g. Moss & Khan, Human Immunology supra; Wills, M.R., et al., J. Virol. 70 (1996) 7569-7579).

The CMV-specific CD8⁺-T-cell response increases markedly with age and individual HLA-peptide tetramers frequently stain in excess of 10 % of the total CD8⁺-T-cell pool (see e.g. Khan, N., et al., J. Immunol. 169 (2002) 1984-1992).

The total CD8⁺-T-cell response in healthy elderly donors could constitute approximately 50 % of the CD8⁺-T-cell repertoire.

The enormous CD8⁺-T-cell expansions are often very clonally restricted, and it is estimated that CMV is the cause of at least 30 % of the clonal CD8⁺-T-cell expansions that are seen in peripheral blood with aging. The total CD8⁺-T-cell count is twice as high in CMV-seropositive donors older than age 60 years in comparison to a CMV-seronegative cohort (see e.g. Looney, R.J., et al., Clin. Immunol. 90 (1999) 213-219).

A fusion of soluble HLA and β-2-microglobulin is reported by Mottez, E., et al., Eur. J. Immunol. 21 (1991) 467-471; Godeau, F., et al., J. Biol. Chem. 267 (1992) 24223-24229 and Mage, M.G., et al., Proc. Natl. Acad. Sci. 89 (1992) 10658-10662. A fusion of viral-derived peptide with soluble HLA and β-2-microglobulin is reported by Mottez, E., et al., J. Exp. Med. 181 (1995) 493-502. A fusion of an immunoglobulin heavy chain with soluble HLA and co-expressed β-2-microglobulin is reported by Dal Porto, J., et al., Proc. Natl. Acad. Sci. USA 90 (1993) 6671-6675. A tetrameric multi-function protein of biotinylated peptide-soluble HLA and β-2-microglobulin with streptavidin chemically coupled to a Fab is described by Robert, B., et al., Eur. J. Immun. 30 (2000) 3165-3170. A chemically coupled Fab with a fusion of viral-derived peptide with soluble HLA and β-2-microglobulin is reported by Robert, B., et al., Cancer Immunity 1 (2001) 2. A fusion of a viral-derived peptide with soluble HLA and β-2-microglobulin to a murine monoclonal antibody heavy chain is reported by Greten, T.F., et al., J. Immunol. Methods 271 (2002) 125-135. An E. coli expression of scFv fusions without peptide, in vitro refolding and peptide loading is reported by Lev, A., et al., J. Immunol. 169 (2002) 2988-2996; Lev, A., Proc. Natl. Acad. Sci. 101 (2004) 9051-9056, and Novak, H., et al., Int. J. Cancer 120 (2007) 329-336. The use of biotinylated soluble MHC loaded with peptides and coupled to streptavidin fused Fab or scFv antibodies is reported by Mous, R., et al., Leukemia 20 (2006) 1096-1102.

In WO 2005/099361 are reported MHC class I - peptide-antibody conjugates with modified beta-2-microglobulin. Exemplary conjugates as reported in WO 2005/099361 are obtained by in vitro conjugation of the alpha chain of the MHC-multi-function protein (HLA) or by the co-expression from separate genes in the same cell.

In US 2004/0091488 antigenic constructs of major histocompatibility multi-function protein class I antigens with specific carrier molecules are reported. These reported fusion polypeptides lack the hinge region.

In WO 02/102299 methods and pharmaceutical compositions for immune deception, particularly useful in the treatment of cancer are reported. Antibody-mediated targeting of human single-chain class I MHC with covalently linked peptides induces efficient killing of tumor cells by tumor or viral-specific cytotoxic T lymphocytes are reported by Oved, K., et al. (Immunotherapy 54 (2005) 867-879). Robert, B., et al. (Cancer Immun. 1 (2001) 1-13) report redirecting anti-viral CTL against cancer cells by surface targeting of monomeric MHC class 1-viral peptide conjugated to antibody fragments. Active antiviral T-lymphocyte response can be redirected against tumor cells by antitumor antibody x MHC/viral peptide conjugates (Cresson, V., et al., Clin. Cancer Res. 12 (2006) 7422-7430). Robert, B., et al. (Eur. J. Immunol. 30 (2000) 3165-3170) report antibody-conjugated MHC class I tetramers can target tumor cells for specific lysis by T lymphocytes. Antigenic constructs of major histocompatibility complex class I antigens with specific carrier molecules, the preparation and use thereof are reported in US 2004/0091488. Bluemel, C., et al. (Cancer Immunol. 59 (2010) 1197-1209) report epitope distance to the target cell membrane and antigen size determine the potency of T cell-mediated lysis by BiTE antibodies specific for a large melanoma surface antigen. Redirected lysis of human melanoma cells by a MCSP/CD3-bispecific BiTE antibody that engages patient derived T cells is reported by Torisu-Itakura, H., et al. (J. Immunother. 34 (2011) 597-605). Greten, T., et al. (J. Immunol. Meth. 271 (2002) 125-135) report peptide-beta2-microglobulin-MHC fusion molecules bind antigen-specific T cells and can be used for multivalent MHC-Ig complexes. Redirection of CMV-specific CTL towards B-CLL via CD20-targeted HLA/CMV complexes is reported by Mous, R., et al. (Leukemia 20 (2006) 1096-1102). In WO 2012/175508 removal of target cells by circulating virus-specific cytotoxic T-cells using MHC class I comprising complexes is reported.

### Summary of the Invention

Herein is reported a multi-function protein comprising exactly one antigen presenting domain as first part, one antibody Fc-region as second part, and at least one antigen binding site that is derived from an antibody and that specifically binds to a target antigen as third part.

With the multi-function protein as reported herein existing virus-specific circulating cytotoxic T-cells (T-memory-cells and/or T-effector-cells) of an individual can be directed to cells expressing the target antigen, to which the antibody derived part of the multi-function protein specifically binds to. Thereafter by dressing these cells with a MHC class I complex an acute viral infection by the virus-derived peptide linked to the MHC class I protein multi-function protein is mimicked and cytotoxic cells are attracted resulting in the removal of the targeted cell.

One aspect as reported herein is a multi-function protein, characterized in that it comprises
- exactly one antigen presenting domain,
- exactly one antibody Fc-region, and
- at least one antigen binding site,
   wherein the antigen presenting domain comprises in N- to C-terminal direction
   either
      (i) a T-cell response eliciting peptide,
      (ii) a β2-microglobulin, and
      (iii) the extracellular domains α1, α2, and α3 of a class I MHC molecule selected from the group comprising HLA-A*0201, HLA-A*1101, HLA-A*2402, HLA-A*340101, HLA-C*0304, HLA-C*0401, and HLA-C*0702,
      or
      (i) a T-cell response eliciting peptide,
      (ii) the extracellular domains α1, α2, and α3 of a class I MHC molecule selected from the group comprising HLA-A*0201, HLA-A*1101, HLA-A*2402, HLA-A*340101, HLA-C*0304, HLA-C*0401, and HLA-C*0702, and
      (iii) a β2-microglobulin,
   wherein the antigen binding site binds to melanoma-associated chondroitin sulfate proteoglycan (MCSP).

In one embodiment the multi-function protein is glycosylated.

In one embodiment the antigen presenting domain is a fusion polypeptide comprising in N- to C-terminal direction the listed components. In one embodiment the antigen presenting domain is recombinantly produced as a complete molecule.

In one embodiment the antibody Fc-region comprises a first and second disulfide-linked Fc-region polypeptide, whereby the antigen binding site comprises the first Fc-region polypeptide.

In one embodiment the antigen binding site comprises i) a (cognate) pair of an antibody heavy chain and an antibody light chain, whereby the individual chains can be wild-type chains or modified chains (substituted, mutated or domain exchanged), or ii) a scFv fusion polypeptide comprising in N- to C-terminal direction a scFv antibody fragment and an antibody Fc-region polypeptide, or iii) a scFab fusion polypeptide comprising in N- to C-terminal direction a scFab and an antibody Fc-region polypeptide.

In one embodiment the antibody light chain pairs only with its cognate heavy chain (i.e. the antibody light chain is no common light chain).

In one embodiment i) the antigen presenting domain is linked to the N-terminus of the heavy chain or to the N-terminus of the light chain of the antigen binding site, or ii) the antigen presenting domain is linked to the C-terminus of the heavy chain or to the C-terminus of the light chain of the antigen binding site, or iii) the antigen presenting domain is linked to the N- or C-terminus of the scFv fusion polypeptide, or iv) the antigen presenting domain is linked to the N- or C-terminus of the scFab fusion polypeptide, or iv) the antigen presenting domain is linked to the N- or C-terminus of the second Fc-region polypeptide.

In one embodiment the cancer cell surface antigen is melanoma-associated chondroitin sulfate proteoglycan (MCSP).

In one embodiment the multi-function protein is a covalent multi-function protein.

In one embodiment the T-cell response eliciting peptide is a virus-derived peptide. In one embodiment the T-cell response eliciting peptide is a CD8⁺-T-cell response eliciting peptide.

In one embodiment the virus is selected from adenovirus, human herpesvirus 1, human herpesvirus 2, human herpesvirus 4 (Epstein-Barr virus), hepatitis-B-virus, hepatitis-C-virus, human cytomegalovirus, human immunodeficiency virus, human papillomavirus type 16, human papillomavirus type 18, human papillomavirus type 31, human papillomavirus type 33, human papillomavirus type 35, human papillomavirus type 39, human papillomavirus type 45, human papillomavirus type 51, human papillomavirus type 52, human papillomavirus type 56, human papillomavirus type 58, human papillomavirus type 59, human papillomavirus type 68, human papillomavirus type 73, human papillomavirus type 82, human T-cell lymphotropic virus type I, human influenza A virus, human influenza B virus, vaccinia virus, dengue virus.

In one embodiment the virus-derived peptide is selected from NLVPMVATV (SEQ ID NO: 01), VTEHDTLLY (SEQ ID NO: 02), NTDFRVLEL (SEQ ID NO: 03), CVETMCNEY (SEQ ID NO: 04), VLEETSVML (SEQ ID NO: 05), NLVPMVATV (SEQ ID NO: 06), RIFAELEGV (SEQ ID NO: 07), IIYTRNHEV (SEQ ID NO: 08), VLAELVKQI (SEQ ID NO: 09), AVGGAVASV (SEQ ID NO: 10), TVRSHCVSK (SEQ ID NO: 11), IMREFNSYK (SEQ ID NO: 12), GPISHGHVLK (SEQ ID NO: 13), ATVQGQNLK (SEQ ID NO: 14), VYALPLKML (SEQ ID NO: 15), AYAQKIFKIL (SEQ ID NO: 16), QYDPVAALF (SEQ ID NO: 17), YVKVYLESF (SEQ ID NO: 18), DIYRIFAEL (SEQ ID NO: 19), VFETSGGLVV (SEQ ID NO: 20), KARDHLAVL (SEQ ID NO: 21), QARLTVSGL (SEQ ID NO: 22), KARAKKDEL (SEQ ID NO: 23), QIKVRVDMV (SEQ ID NO: 24), RRRHRQDAL (SEQ ID NO: 25), ARVYEIKCR (SEQ ID NO: 26), KMQVIGDQY (SEQ ID NO: 27), NVRRSWEEL (SEQ ID NO: 28), CPSQEPMSIYVY (SEQ ID NO: 29), KPGKISHIMLDVA (SEQ ID NO: 30), ELRRKMMYM (SEQ ID NO: 31), IPSINVHHY (SEQ ID NO: 32), FEQPTETPP (SEQ ID NO: 33), YAYIYTTYL (SEQ ID NO: 34), QEFFWDANDIY (SEQ ID NO: 35), YEQHKITSY (SEQ ID NO: 36), QEPMSIYVY (SEQ ID NO: 37), SEHPTFTSQY (SEQ ID NO: 38), QAIRETVEL (SEQ ID NO: 39), TRATKMQVI (SEQ ID NO: 40), DALPGPCI (SEQ ID NO: 41), CEDVPSGKL (SEQ ID NO: 42), HERNGFTVL (SEQ ID NO: 43), PTFTSQYRIQGKL (SEQ ID NO: 44), QMWQARLTV (SEQ ID NO: 45), HELLVLVKKAQL (SEQ ID NO: 46), or DDYSNTHSTRYV (SEQ ID NO: 47), SLYNTVATL (SEQ ID NO: 48), GLCTLVAML (SEQ ID NO: 49), GILGFVFTL (SEQ ID NO: 50), STNRQSGRQ (SEQ ID NO: 51), LLFGYPVYV (SEQ ID NO: 52), FAEGFVRAL (SEQ ID NO: 53), LIVIGILIL (SEQ ID NO: 54), or ILHTPGCV (SEQ ID NO: 55), WYAQIQPHW (SEQ ID NO: 56), AFSGVSWTM (SEQ ID NO: 57), ILIGVVITW (SEQ ID NO: 58), MMIPTVVAF (SEQ ID NO: 59), PFPQSNAPI (SEQ ID NO: 60), LLLTLLATV (SEQ ID NO: 61), IVLEHGSCV (SEQ ID NO: 62), LLFKTENGV (SEQ ID NO: 63), PLNEAIMAV (SEQ ID NO: 64), NLVRLQSGV (SEQ ID NO: 65), LVISGLFPV (SEQ ID NO: 66), LLLVAHYAI (SEQ ID NO: 67), LALLAAFKV (SEQ ID NO: 68), VILAGPMPV (SEQ ID NO: 69), HVLGRLITV (SEQ ID NO: 70), or a variant thereof comprising of from 1 to 3 amino acid exchanges, additions, and/or deletions.

In one embodiment the virus-derived peptide is a human cytomegalovirus-derived peptide. In one embodiment the virus-derived peptide has an amino acid sequence selected from the group of SEQ ID NO: 01 to SEQ ID NO: 47. In one embodiment the virus-derived peptide has the amino acid sequence of SEQ ID NO: 01.

In one embodiment the antigen presenting domain comprises
(i) a virus-derived peptide,
(ii) β2-microglobulin, and
(iii) the soluble HLA-A allele A*0201.

In one embodiment the β2-microglobulin is human β2-microglobulin.

In one embodiment the β2-microglobulin is wild-type human β2-microglobulin.

In one embodiment the β2-microglobulin is consisting of the amino acid sequence of SEQ ID NO: 71 or is a functional variant thereof comprising of from 1 to 10 amino acid exchanges, additions, and/or deletions.

In one embodiment the extracellular domain (α1, α2 and α3) of a class I MHC molecule is consisting of the amino acid sequence of SEQ ID NO: 72 or is a functional variant thereof comprising of from 1 to 10 amino acid exchanges, additions, and/or deletions.

In one embodiment the virus-derived peptide is fused to the β2-microglobulin via a first linker peptide.

In one embodiment the virus-derived peptide is fused to the N-terminus of the β2-microglobulin.

In one embodiment the β2-microglobulin is fused to the extracellular domain α1 of a class I MHC molecule via a second linker peptide.

In one embodiment the extracellular domains α3 of a class I MHC molecule is fused to one of the disulfide-linked polypeptide chains via a third linker peptide.

In one embodiment the first, second, and third linker peptide is the same or different.

In one embodiment the first linker peptide, the second linker peptide, and the third linker peptide are selected independently from each other from the amino acid sequences GS (SEQ ID NO: 73), GGS (SEQ ID NO: 74), GSG (SEQ ID NO: 136), GGGS (SEQ ID NO: 75), GGGSGGGS (SEQ ID NO: 76), GGGSGGGSGGGS (SEQ ID NO: 77), GGGSGGGSGGGSGGGS (SEQ ID NO: 78), GGGSGGGSGGGSGGGSGGGS (SEQ ID NO: 79), GGGGS (SEQ ID NO: 80), GGGGSGGGGS (SEQ ID NO: 81), GGGGSGGGGSGGGGS (SEQ ID NO: 82), GGGGSGGGGSGGGGSGGGGS (SEQ ID NO: 83), and GGGGSGGGGSGGGGSGGGGSGGGGS (SEQ ID NO: 84).

In one embodiment of all aspects the first linker peptide comprises the amino acid sequence of SEQ ID NO: 82.

In one embodiment of all aspects the second linker peptide comprises the amino acid sequence of SEQ ID NO: 83.

In one embodiment of all aspects the third linker peptide comprises the amino acid sequence of SEQ ID NO: 73.

In one embodiment the antigen presenting domain comprises in N- to C-terminal direction
(i) a virus-derived peptide that has an amino acid sequence selected from the group comprising SEQ ID NO: 01 to SEQ ID NO: 70,
(ii) a first linker peptide that has an amino acid sequence selected from the group comprising SEQ ID NO: 77, 78, 79, 82, 83, and 84,
(iii) a β2-microglobulin that has an amino acid sequence of SEQ ID NO: 71,
(iv) a second linker peptide that has an amino acid sequence selected from the group comprising SEQ ID NO: 77, 78, 79, 82, 83, and 84,
(v) the extracellular domains α1, α2, and α3 of a class I MHC molecule that has an amino acid sequence of SEQ ID NO: 72, and
(vi) a third linker peptide that has an amino acid sequence selected from the group comprising SEQ ID NO: 73, 77, 78, 79, 82, 83, 84, and 136.

In one embodiment
- the first linker peptide has the amino acid sequence of SEQ ID NO: 82, and/or
- the second linker peptide has the amino acid sequence of SEQ ID NO: 83, and/or
- the third linker peptide has the amino acid sequence of SEQ ID NO: 136.

In one embodiment the multi-function protein is characterized in that the antigen presenting domain comprises in N- to C-terminal direction
(i) a virus-derived peptide that has an amino acid sequence selected from the group comprising SEQ ID NO: 01 to SEQ ID NO: 70,
(ii) a first linker peptide that has an amino acid sequence selected from the group comprising SEQ ID NO: 77, 78, 79, 82, 83, and 84,
(iii) a β2-microglobulin that has an amino acid sequence of SEQ ID NO: 71 or is a functional variant thereof comprising of from 1 to 10 amino acid exchanges, additions, and/or deletions,
(iv) a second linker peptide that has an amino acid sequence selected from the group comprising SEQ ID NO: 77, 78, 79, 82, 83, and 84,
(v) the extracellular domains α1, α2 and α3 of a class I MHC molecule that has an amino acid sequence of SEQ ID NO: 72 or is a functional variant thereof comprising of from 1 to 10 amino acid exchanges, additions, and/or deletions, and
(vi) a third linker peptide that has an amino acid sequence selected from the group comprising SEQ ID NO: 73, 77, 78, 79, 82, 83, 84, and 136,

In one embodiment the antibody Fc-region is selected from an antibody Fc-region of a human antibody of the class IgG or the class IgE.

In one embodiment the antibody Fc-region is selected from an antibody Fc-region of a human antibody of the subclass IgG1, or IgG2, or IgG3, or IgG4.

In one embodiment the antibody Fc-region is of a human antibody of the subclass IgG1 or IgG2 and comprises at least one mutation in E233, L234, L235, G236, D265, D270, N297, E318, K320, K322, A327, P329, A330, and/or P331 (numbering according to the EU index of Kabat).

In one embodiment the antibody Fc-region is of a human antibody of the subclass IgG1 or the subclass IgG2 with the mutations L234A and L235A, and/or the mutations D265A and N297A, and/or contains the PVA236 mutation, and/or contains the mutation P329G.

In one embodiment the antibody Fc-region is of a human antibody of the subclass IgG1 with the mutations L234A and L235A, and/or P329G.

In one embodiment the antibody Fc-region is of a human antibody of the subclass IgG4 with the mutation S228P and/or L235E.

In one embodiment the first and second antibody Fc-region polypeptide is selected independently of each other from the group comprising SEQ ID NO: 87 to 101.

In one embodiment the antibody Fc-region comprises two Fc-region polypeptides with the amino acid sequence of SEQ ID NO: 94.

In one embodiment the antibody Fc-region comprises two Fc-region polypeptides with the amino acid sequence of SEQ ID NO: 100.

In one embodiment the antibody Fc-region comprises two Fc-region polypeptides with the amino acid sequence of SEQ ID NO: 101.

In one embodiment the antibody Fc-region comprises a first Fc-region polypeptide with the amino acid sequence of SEQ ID NO: 89 and a second Fc-region polypeptide with the amino acid sequence of SEQ ID NO: 90.

In one embodiment the antibody Fc-region comprises a first Fc-region polypeptide with the amino acid sequence of SEQ ID NO: 97 and a second Fc-region polypeptide with the amino acid sequence of SEQ ID NO: 98.

In one embodiment the antibody Fc-region comprises a first Fc-region polypeptide with the amino acid sequence of SEQ ID NO: 102 and a second Fc-region polypeptide with the amino acid sequence of SEQ ID NO: 103.

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, and
- at least one antigen binding site, which specifically binds to melanoma-associated chondroitin sulfate proteoglycan (MCSP).

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
- at least one antigen binding site, which specifically binds to melanoma-associated chondroitin sulfate proteoglycan (MCSP).

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
- at least one antigen binding site, which specifically binds to melanoma-associated chondroitin sulfate proteoglycan (MCSP).

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
- at least one antigen binding site, which comprises an antibody light chain variable domain comprising an amino acid sequence from the group of SEQ ID NO: 104 to 106 and an antibody heavy chain variable domain comprising an amino acid sequence from the group of SEQ ID NO: 108 to 110, which specifically binds to melanoma-associated chondroitin sulfate proteoglycan (MCSP).

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
- at least one antigen binding site, which specifically binds to melanoma-associated chondroitin sulfate proteoglycan (MCSP), and which comprises an antibody light chain with a variable domain comprising an amino acid sequence from the group of SEQ ID NO: 104 to 106 and an antibody heavy chain with a variable domain comprising an amino acid sequence from the group of SEQ ID NO: 108 to 110.

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
- two antigen binding sites, which specifically bind to melanoma-associated chondroitin sulfate proteoglycan (MCSP), and which each comprise an antibody light chain with a variable domain comprising an amino acid sequence from the group of SEQ ID NO: 105 to 107 and an antibody heavy chain with a variable domain comprising an amino acid sequence from the group of SEQ ID NO: 110 to 112.

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
- at least one antigen binding site, which specifically binds to melanoma-associated chondroitin sulfate proteoglycan (MCSP), and which comprises an antibody light chain with a variable domain comprising an amino acid sequence from the group of SEQ ID NO: 105 to 107 and an antibody heavy chain with a variable domain comprising an amino acid sequence from the group of SEQ ID NO: 110 to 112, whereby the Fc-region of the antibody heavy chain is one of the disulfide-linked Fc-region polypeptides.

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
- at least one antigen binding site, which specifically binds to melanoma-associated chondroitin sulfate proteoglycan (MCSP), and which comprises an antibody light chain with a variable domain comprising an amino acid sequence from the group of SEQ ID NO: 104 to 106 and an antibody heavy chain with a variable domain comprising an amino acid sequence from the group of SEQ ID NO: 108 to 110, whereby the Fc-region of the antibody heavy chain is one of the disulfide-linked Fc-region polypeptides,
   wherein the antigen presenting domain is linked to the N-terminus of one of the variable domains.

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
- two antigen binding site, which specifically bind to melanoma-associated chondroitin sulfate proteoglycan (MCSP), and which each comprise an antibody light chain with a variable domain comprising an amino acid sequence from the group of SEQ ID NO: 104 to 106 and an antibody heavy chain with a variable domain comprising an amino acid sequence from the group of SEQ ID NO: 108 to 110, whereby the Fc-regions of the antibody heavy chains are the disulfide-linked Fc-region polypeptides,
   wherein the antigen presenting domain is linked to the N-terminus of one of the variable domains.

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
- two antigen binding site, which specifically bind to melanoma-associated chondroitin sulfate proteoglycan (MCSP), and which each comprise an antibody light chain with a variable domain comprising an amino acid sequence from the group of SEQ ID NO: 105 to 107 and an antibody heavy chain with a variable domain comprising an amino acid sequence from the group of SEQ ID NO: 110 to 112, whereby the Fc-regions of the antibody heavy chains are the disulfide-linked Fc-region polypeptides,
   wherein the antigen presenting domain is linked to the N-terminus of one of the heavy chain variable domains.

One aspect as reported herein is a multi-function protein, characterized in that it comprises
- exactly one antigen presenting domain, which comprises in N- to C-terminal direction
   (i) a T-cell response eliciting peptide of SEQ ID NO: 01,
   (ii) a β2-microglobulin of SEQ ID NO: 71, and
   (iii) the extracellular domains α1, α2, and α3 of a class I MHC molecule of SEQ ID NO: 72,
- exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
- two antigen binding site, which specifically bind to melanoma-associated chondroitin sulfate proteoglycan (MCSP), and which each comprise an antibody light chain with a variable domain comprising an amino acid sequence from the group of SEQ ID NO: 105 to 107 and an antibody heavy chain with a variable domain comprising an amino acid sequence from the group of SEQ ID NO: 110 to 112, whereby the Fc-regions of the antibody heavy chains are the disulfide-linked Fc-region polypeptides,
   wherein the antigen presenting domain is linked to the N-terminus of one of the heavy chain variable domains.

One aspect as reported herein is a multi-function protein, characterized in that it comprises
- one polypeptide chain of SEQ ID NO: 117,
- one polypeptide chain of SEQ ID NO: 118,
- two polypeptide chains each of SEQ ID NO: 119.

One aspect as reported herein is a multi-function protein, characterized in that it comprises
- one polypeptide chain of SEQ ID NO: 137,
- one polypeptide chain of SEQ ID NO: 118,
- two polypeptide chains each of SEQ ID NO: 119.

In one embodiment the MCSP binding site comprises an antibody heavy chain variable domain comprising an HVR-H1 comprising the amino acid sequence of SEQ ID NO: 108, an HVR-H2 comprising the amino acid sequence of SEQ ID NO: 109, and an HVR-H3 comprising the amino acid sequence of SEQ ID NO: 110.

In one embodiment the MCSP binding site comprises an antibody light chain variable domain comprising an HVR-L1 comprising the amino acid sequence of SEQ ID NO: 104; an HVR-L2 comprising the amino acid sequence of SEQ ID NO: 105; and an HVR-L3 comprising the amino acid sequence of SEQ ID NO: 106.

In one embodiment the MCSP binding site comprises an antibody heavy chain variable domain comprising an HVR-H1 comprising the amino acid sequence of SEQ ID NO: 108; an HVR-H2 comprising the amino acid sequence of SEQ ID NO: 109; an HVR-H3 comprising the amino acid sequence of SEQ ID NO: 110; and an antibody light chain variable domain comprising an HVR-L1 comprising the amino acid sequence of SEQ ID NO: 104; an HVR-L2 comprising the amino acid sequence of SEQ ID NO: 105; and an HVR-L3 comprising the amino acid sequence of SEQ ID NO: 106.

In one embodiment the MCSP binding site comprises an antibody heavy chain variable domain comprising an HVR-H1 comprising the amino acid sequence of SEQ ID NO: 111, an HVR-H2 comprising the amino acid sequence of SEQ ID NO: 112, and an HVR-H3 comprising the amino acid sequence of SEQ ID NO: 110.

In one embodiment the MCSP binding site comprises an antibody light chain variable domain comprising an HVR-L1 comprising the amino acid sequence of SEQ ID NO: 107; an HVR-L2 comprising the amino acid sequence of SEQ ID NO: 105; and an HVR-L3 comprising the amino acid sequence of SEQ ID NO: 106.

In one embodiment the MCSP binding site comprises an antibody heavy chain variable domain comprising an HVR-H1 comprising the amino acid sequence of SEQ ID NO: 111; an HVR-H2 comprising the amino acid sequence of SEQ ID NO: 112; an HVR-H3 comprising the amino acid sequence of SEQ ID NO: 110; and an antibody light chain variable domain comprising an HVR-L1 comprising the amino acid sequence of SEQ ID NO: 107; an HVR-L2 comprising the amino acid sequence of SEQ ID NO: 105; and an HVR-L3 comprising the amino acid sequence of SEQ ID NO: 106.

In one embodiment the MCSP binding site comprises an antibody heavy chain variable domain having at least 95% sequence identity to the amino acid sequence of SEQ ID NO: 114; and an antibody light chain variable domain having at least 95% sequence identity to the amino acid sequence of SEQ ID NO: 113.

In one embodiment the MCSP binding site comprises an antibody heavy chain variable domain of SEQ ID NO: 114 and an antibody light chain variable domain of SEQ ID NO: 113.

In one embodiment the MCSP binding site comprises SEQ ID NO: 114 and SEQ ID NO: 113.

In one embodiment the MCSP binding site comprises an antibody heavy chain variable domain having at least 95% sequence identity to the amino acid sequence of SEQ ID NO: 116; and an antibody light chain variable domain having at least 95% sequence identity to the amino acid sequence of SEQ ID NO: 115.

In one embodiment the MCSP binding site comprises an antibody heavy chain variable domain of SEQ ID NO: 116; and an antibody light chain variable domain of SEQ ID NO: 115.

In one embodiment the MCSP binding site comprises SEQ ID NO: 116 and SEQ ID NO: 115.

In one aspect, the invention provides multi-function proteins comprising the binding specificity, i.e. HVRs or variable domains, of isolated antibodies that bind to MCSP. In particular, the anti-MCSP antibody binding specificity, i.e. HVRs or variable domains, comprised in the multi-function proteins as provided herein bind to a membrane proximal epitope of human MCSP. As discussed in Staub, E., et al. (FEBS Lett. 527 (2002) 114-118), the membrane proximal region of MCSP is comprised of multiple novel repeated domains, referred to as CSPG repeat domains.

One aspect as reported herein is a nucleic acid encoding the multi-function protein as reported herein.

In one embodiment the nucleic acid comprises two to four expression cassettes comprising structural genes encoding polypeptides with different amino acid sequence.

One aspect as reported herein is a host cell comprising the nucleic acid as reported herein.

One aspect as reported herein is a method of producing a multi-function protein as reported herein comprising culturing the host cell as reported herein so that the multi-function protein is produced.

In one embodiment the multi-function protein is recovered from the cells or the cultivation medium and thereby the multi-function protein is produced.

One aspect as reported herein is a pharmaceutical formulation comprising the multi-function protein as reported herein and optionally a pharmaceutically acceptable carrier.

In one embodiment the pharmaceutical formulation further comprises an additional therapeutic agent.

One aspect as reported herein is the multi-function protein as reported herein for use as a medicament.

One aspect as reported herein is the multi-function protein as reported herein for use in treating cancer.

One aspect as reported herein is the multi-function protein as reported herein for use in attracting virus-specific cytotoxic T-cells of an individual to a target.

One aspect as reported herein is the multi-function protein as reported herein for use in removal cancer cells.

One aspect as reported herein is the use of the multi-function protein as reported herein in the manufacture of a medicament.

In one embodiment the medicament is for treatment of cancer.

In one embodiment the medicament is for attracting virus-specific cytotoxic T-cells of an individual to a target.

In one embodiment the medicament is for removal cancer cells.

One aspect as reported herein is a method of treating an individual having cancer comprising administering to the individual an effective amount of the multi-function protein as reported herein.

In one embodiment the method further comprises administering an additional therapeutic agent to the individual.

One aspect as reported herein is a method of attracting virus-specific cytotoxic T-cells of an individual to a target in an individual comprising administering to the individual an effective amount of the multi-function protein as reported herein to attract virus-specific cytotoxic T-cells of an individual to a target.

One aspect as reported herein is a method of removal cancer cells in an individual comprising administering to the individual an effective amount of the multi-function protein as reported herein to remove/disintegrate cancer cells.

One aspect as reported herein is a method for the recombinant production of a multi-function protein comprising i) a fusion polypeptide of β2-microglobulin and the extracellular domains α1, α2 and α3 of a class I MHC molecule, ii) a pair of disulfide-linked polypeptide chains each comprising an antibody hinge region, and iii) at least one pair of an antibody light chain variable domain and an antibody heavy chain variable domain in a eukaryotic cell, comprising the steps of i) cultivating a eukaryotic cell comprising one or more nucleic acids encoding the multi-function protein, and ii) recovering the multi-function protein from the cell or the cultivation medium, wherein the multi-function protein comprises exactly one fusion polypeptide of β2-microglobulin and the extracellular domains α1, α2 and α3 of a class I MHC molecule.

In one embodiment the multi-function protein comprises exactly one MHC-derived polypeptide or exactly one fusion polypeptide comprising an MHC-derived molecule.

In one embodiment the multi-function protein is obtained with a concentration of 1 mg/ml or more in the cultivation medium. In one embodiment the multi-function protein is obtained with a concentration of 4 mg/ml or more in the cultivation medium.

In one embodiment the eukaryotic cell is a mammalian cell. In one embodiment the mammalian cell is a human embryonic kidney cell, or a chinese hamster ovary cell, or a baby hamster kidney cell, or a mouse myeloma cell.

The following embodiments can be combined with any of the aspects as reported herein. Also any embodiment as reported herein can be combined with any other embodiment or combination of embodiments as reported herein.

### Detailed Description of the Invention

### Short description of the figures

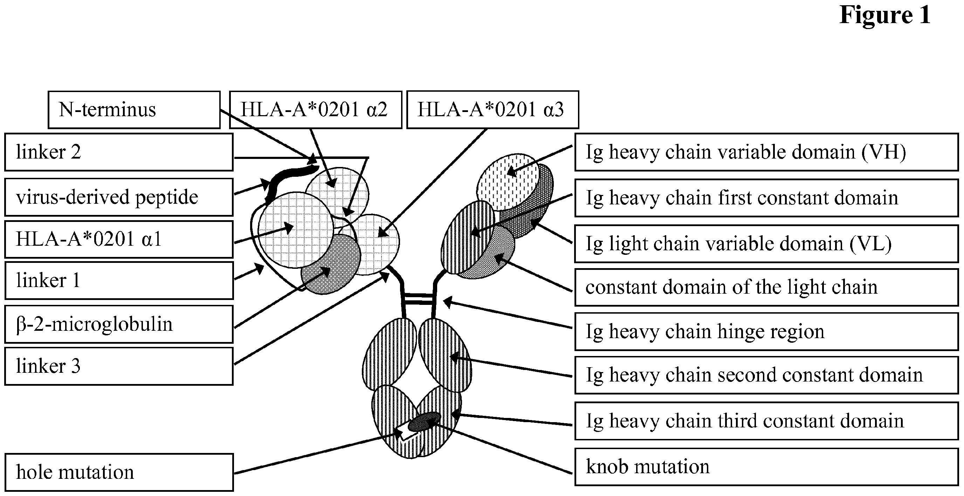

- **Figure 1**: Annotated scheme of an exemplary multi-function protein as reported herein.
- **Figure 2**: Exemplary polypeptides comprised in the multi-function protein as reported herein: fusion polypeptides were N-terminally fused to either an antibody light chain or to an antibody heavy chain hinge region comprising polypeptide.

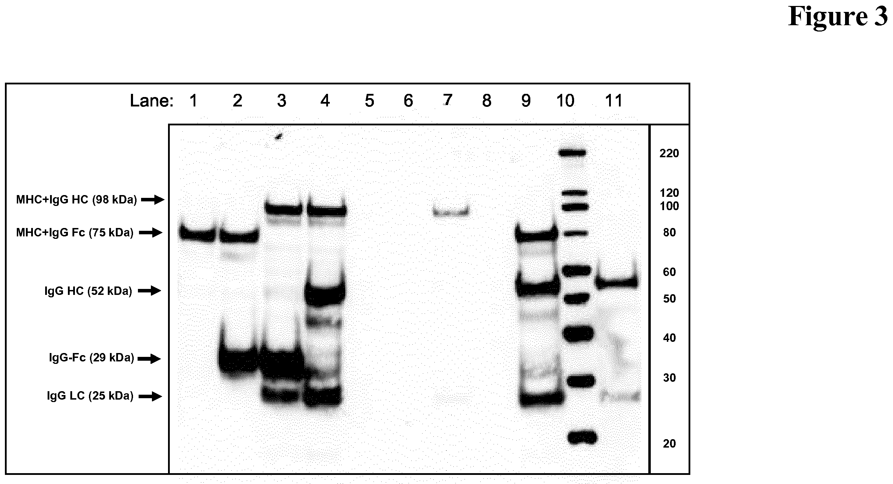

- **Figure 3**: Western blot of a SDS polyacrylamide gel of cell culture supernatant of HEK 293 cells transfected with the corresponding expression plasmids. Staining was performed with peroxidase conjugated polyclonal rabbit anti-human κ-light chain antibody and polyclonal rabbit anti-human IgG antibody conjugated to horseradish peroxidase.
Lanes: 1: two-armed peptide-β2-microglobulin-HLA-A0201-IgG-Fc; 2: one-armed peptide-β2-microglobulin-HLA-A0201-IgG-Fc + IgG-Fc; 3: one-armed peptide-β2-microglobulin-HLA-A0201-IgG-heavy chain + IgG-light chain + IgG-Fc; 4: one-armed peptide-β2-microglobulin-HLA-A0201-IgG-heavy chain + IgG-heavy chain + IgG-light chain; 5: two-armed β2-microglobulin-HLA-A0201-IgG-light chain + IgG-heavy chain; 6: two-armed peptide-β2-microglobulin-HLA-A0201-IgG-light chain + IgG-heavy chain; 7: two-armed peptide-β2-microglobulin-HLA-A0201-IgG-heavy chain + IgG-light chain; 8: two-armed peptide-β2-microglobulin-HLA-A0201-IgG-Fc-scFv; 9: one-armed peptide-β2-microglobulin-HLA-A0201-IgG-Fc + one-armed IgG (heavy and light chain); 10: molecular weight marker; 11: reference standard IgG1 antibody.

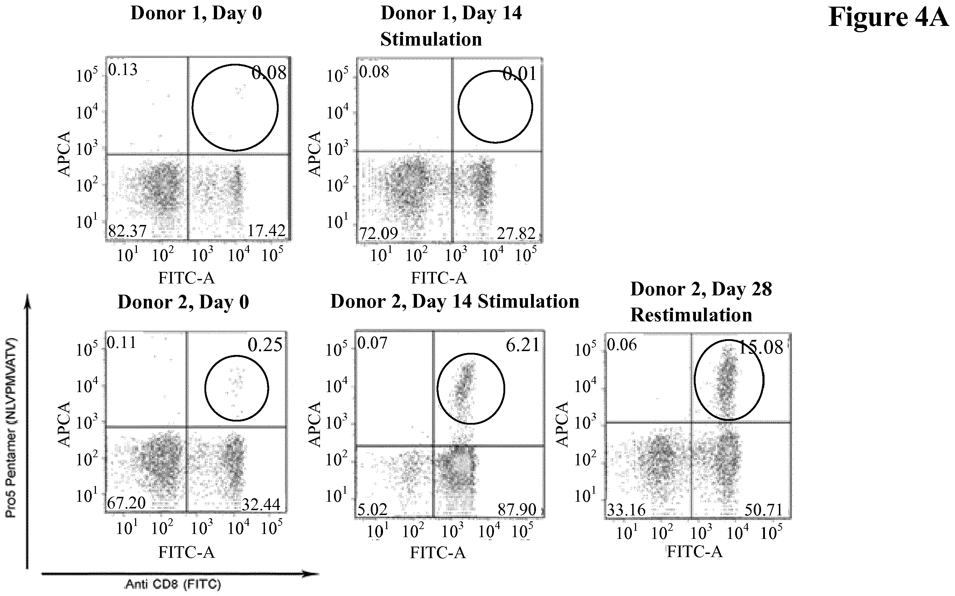

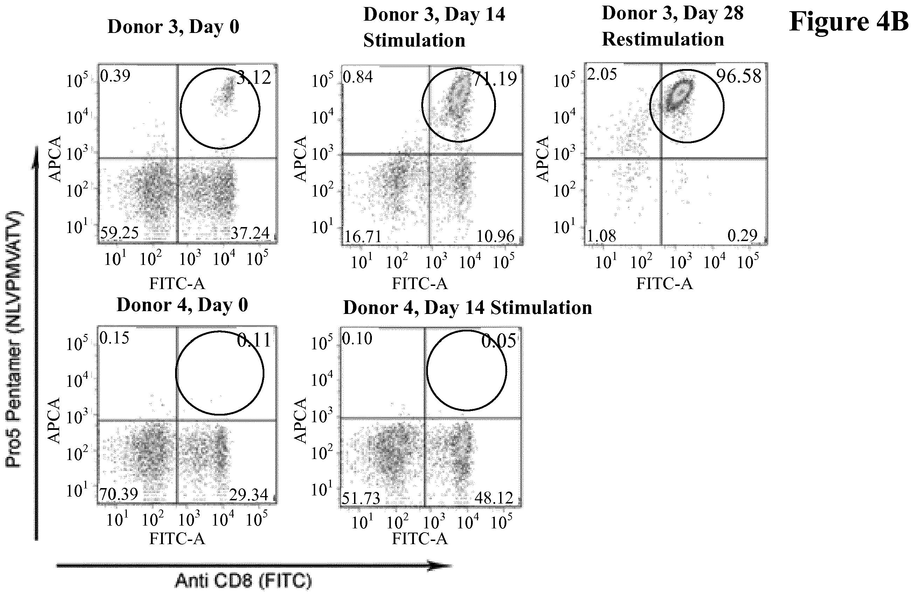

- **Figure 4**: Flow cytometric analysis to determine the number of CMV-specific cytolytic T-cells from different donors before and after in vitro stimulation with specific peptide: Analysis of 4 human donor derived peripheral blood lymphocytes (PBLs); anti-CD8 antibody conjugated to FITC label staining (BD, Cat. No. 345772) combined with Pro5 pentamer APC (ProImmune, Cat. No. F008-4A-E) stained TCR recognizing MHC-class I (HLA-A*0201) loaded with CMV-derived peptide (NLVPMVATV, SEQ ID NO: 01); circle: CMV-specific CD8⁺-T-cells; A: Donor 1; B: Donor 3.

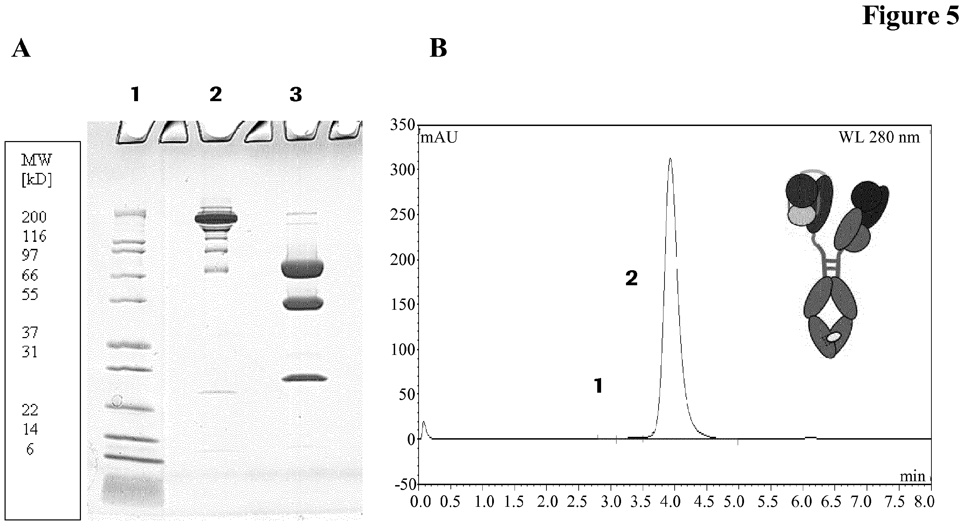

- **Figure 5**: A: SDS-PAGE gel with Coomassie staining: lane 1: molecular weight standard, lane 2: one-armed peptide-β2-microglobulin-HLA-A0201-IgG-Fc + one-armed IgG (heavy and light chain), non-reducing conditions; lane 3: one-armed peptide-β2-microglobulin-HLA-A0201-IgG-Fc + one-armed IgG multi-function protein (heavy and light chain), reducing conditions.
B: Size exclusion chromatography chromatogram; 1: high molecular weight forms (0.7 area %); 2: monomeric multi-function protein (99.3 area %). C: schematic molecule.
- **Figure 6**: A:
   a) SDS-PAGE gel with Coomassie staining after protein A HPLC and SEC; non-reducing conditions; lane 1: molecular weight standard, lane 2: peptide-β2-microglobulin-HLA-A0201-HC + LC + IgG-Fc, lane 3: peptide-β2-microglobulin-HLA-A0201-HC + LC + one-armed IgG (heavy and light chain).
   b): SDS-PAGE gel with Coomassie staining after protein A HPLC and SEC; reducing conditions; lane 1: molecular weight standard, lane 2: peptide-β2-microglobulin-HLA-A0201-HC + LC + IgG-Fc, lane 3: peptide-β2-microglobulin-HLA-A0201-HC + LC + one-armed IgG (heavy and light chain).
B:
   a) Size exclusion chromatography chromatogram of peptide-β2-microglobulin-HLA-A0201-HC + LC + IgG-Fc; 1: high molecular weight forms (1.9 area %); 2: monomeric multi-function protein (98.1 area %).
   b) Size exclusion chromatography chromatogram of peptide-β2-microglobulin-HLA-A0201-HC + LC + one-armed IgG (heavy and light chain); 1: high molecular weight forms (2.1 area %); 2: monomeric multi-function protein (97.9 area %)
C: schematic molecules 2: peptide-β2-microglobulin-HLA-A0201-HC + LC + IgG-Fc, 3: peptide-β2-microglobulin-HLA-A0201-HC + LC + one-armed IgG.

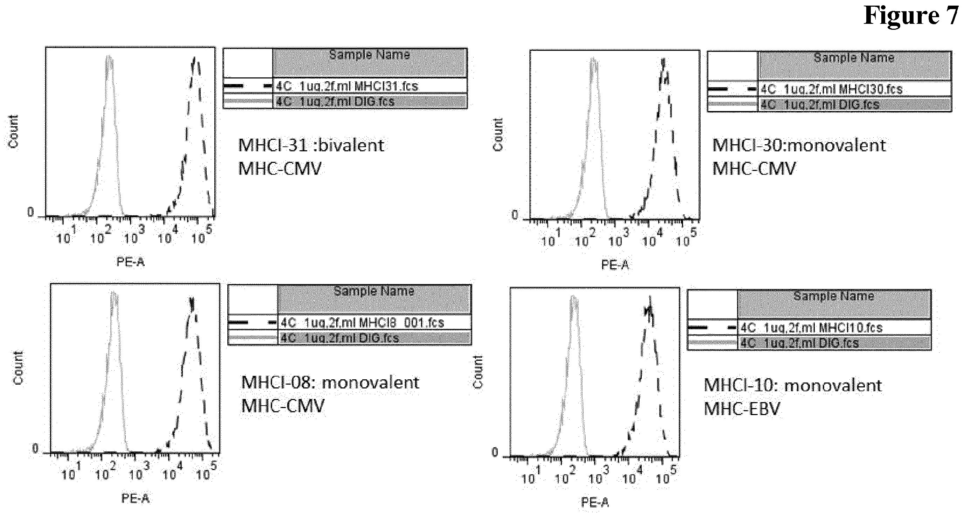

- **Figure 7**: Binding of different MHC-I-multi function proteins on MCSP+ target cells (Colo38): Colo38 cells were incubated for 5 min. with Accutase (PAA, Cat.# L11-007) to obtain a single cell suspension. 2x10⁵ cells per vial were incubated with 1µg/ml MHC-I-multi function protein in 100µl PBS/2%FCS for 45 min. at 4°C. After incubation cells were washed with 1 ml cold PBS/2%FCS and centrifuged for 7 min. with 910 rpm. Cells were resuspended in 100 µl PBS/2%FCS with secondary antibody (goat anti-human IgG1 antibody PE conjugate, Jackson, Cat.# 109-116-088) (2 µg/ml) and incubated for another 45 min. at 4°C. Cells were washed twice with 1 ml PBS%2%FCS and measured with BD Canto II Flow Cytometer.

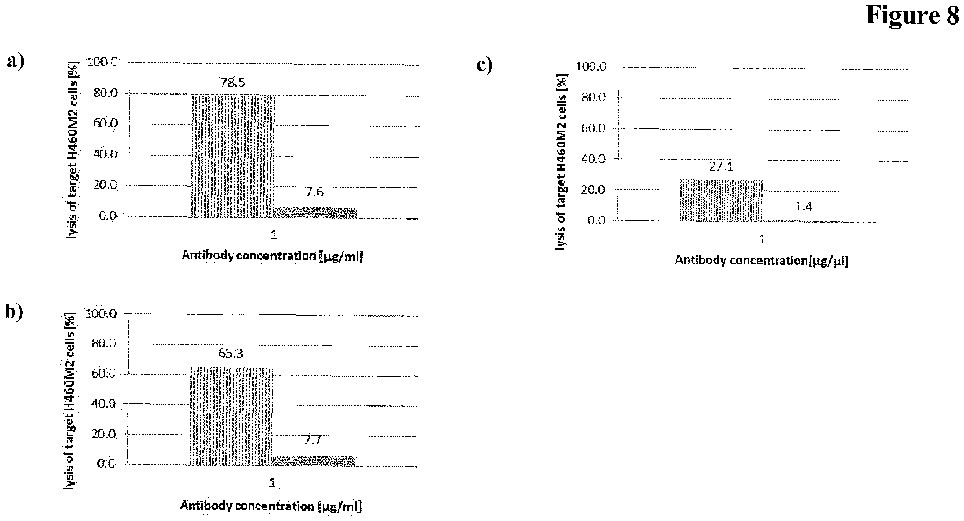

- **Figure 8**: Cytotoxicity assay: antigen binding multi-function protein as reported herein triggers lysis of H460M2 tumor cells through human CMV-specific T-cells. a) (6h) target Cells: CMV-specific effector T-cells 1:1.5; b) (6h) target cells:CMV-specific effector T-cells 1:0.75; c) (6h) target cells:CMV-specific effector T-cells 1:0.5; left bar: multi-function protein as reported herein; right bar MAB IGF-1R-afucosylated.

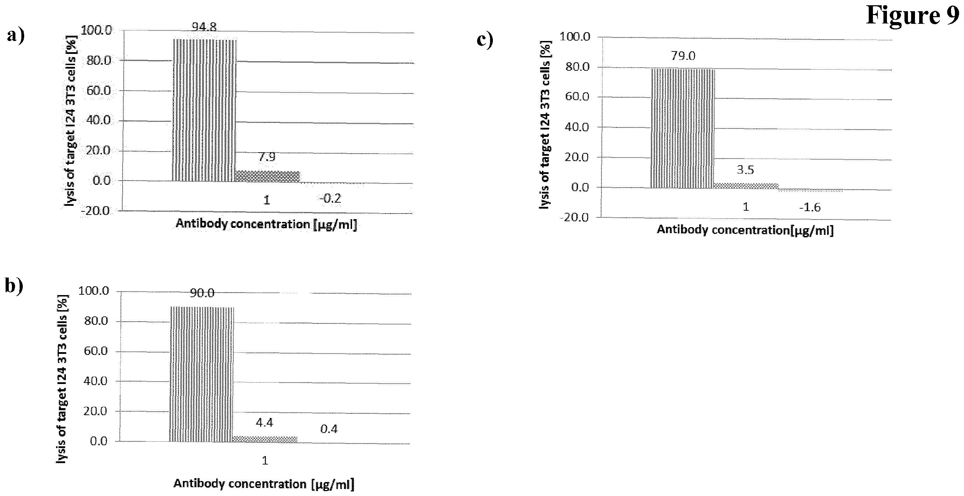

- **Figure 9**: Cytotoxicity assay: antigen binding multi-function protein as reported herein triggers lysis of I24 3T3 tumor cells through human CMV-specific T-cells; a) (9h) Target Cells : CMV-specific Effector T-cells 1:1.5; b) (9h) Target Cells : CMV-specific Effector T-cells 1:0.75; c) 9h) Target Cells : CMV-specific Effector T-cells 1:0.5; left bar: multi-function protein as reported herein; middle bar: MAB IGF-1R-afu; right bar: MAB ed; right bar: anti-digoxygenin antibody.

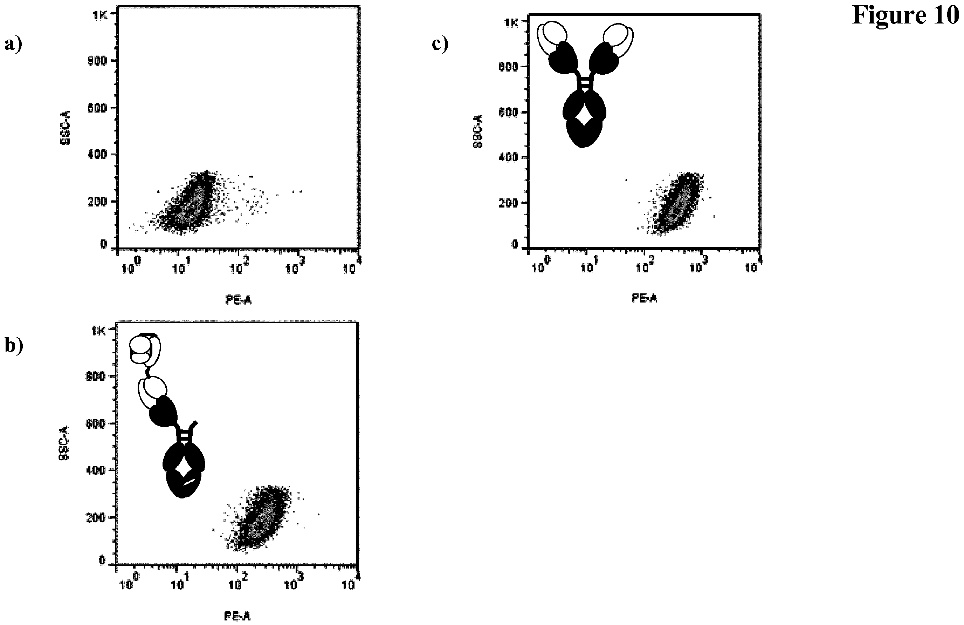

- **Figure 10**: FACS analysis of binding of anti-IGF-1R antibody and multi-function proteins as reported herein to lung adenocarcinoma cell line H460M2; a) secondary antibody only (goat anti-human IgG(H+L) (Jackson Laboratories, Cat# 109-116-088)); b) multi-function protein as reported herein wherein the fusion polypeptide is fused to the N-terminus of the heavy chain of an anti-IGF-1R antibody comprising only one pair of variable domains; c) anti-IGF-1R antibody.

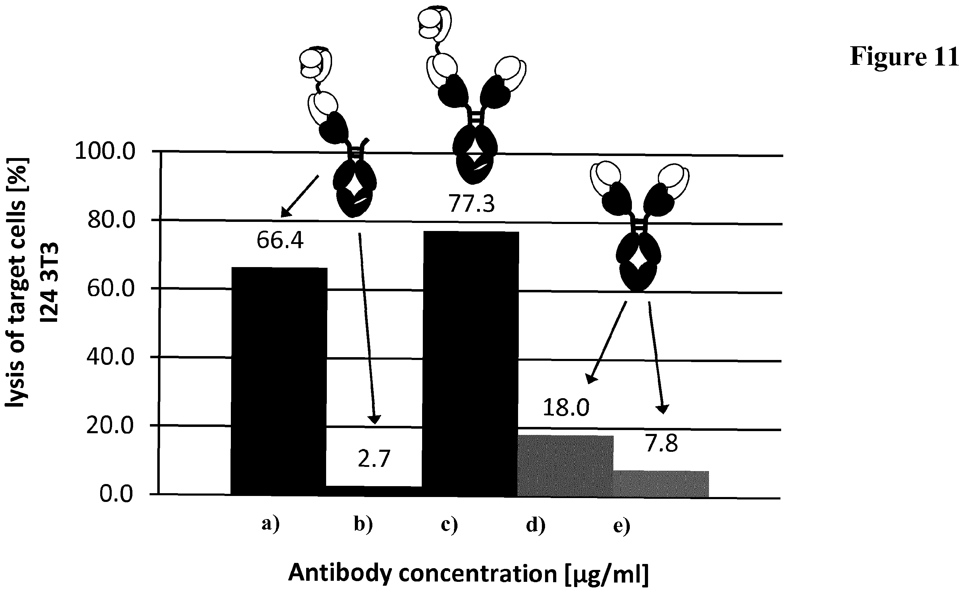

- **Figure 11**: In vitro efficacy and specificity (cytotoxicity assay) of different multi-function proteins as reported herein; a) multi-function protein comprising a monovalent anti-IGF1R antibody and a CMV-derived peptide ; b) multi-function protein comprising a monovalent anti-IGF1R antibody and an EBV-derived peptide (control); c) multi-function protein comprising a bivalent anti-IGF1R antibody and a CMV-derived peptide; d) anti-IGF-1R antibody (control); e)anti-digoxigenin antibody (control).

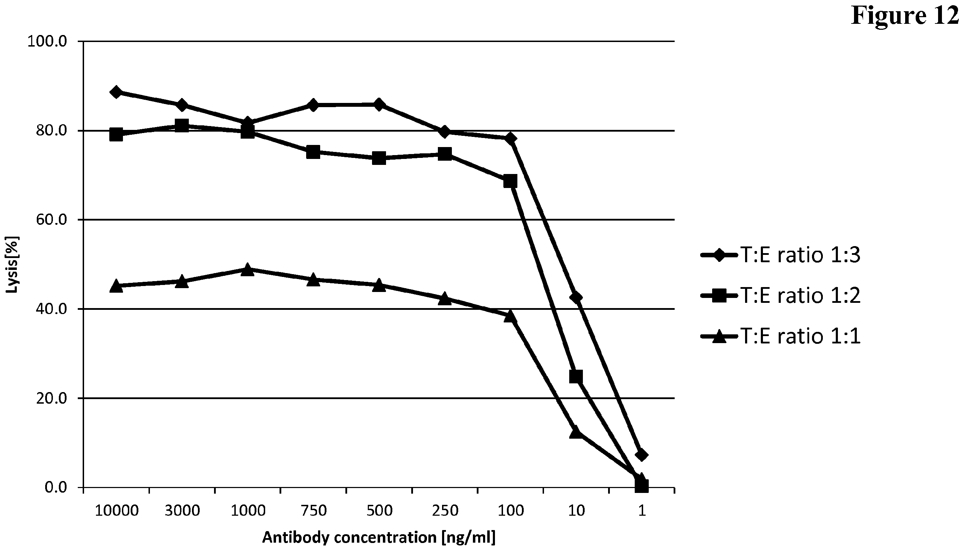

- **Figure 12**: In vitro efficacy and specificity (EC50 value) of a multi-function protein as reported herein wherein the fusion polypeptide is fused to the N-terminus of the heavy chain of a complete anti-IGF-1R antibody determined at different target (T) to effector (E) cell ratios.

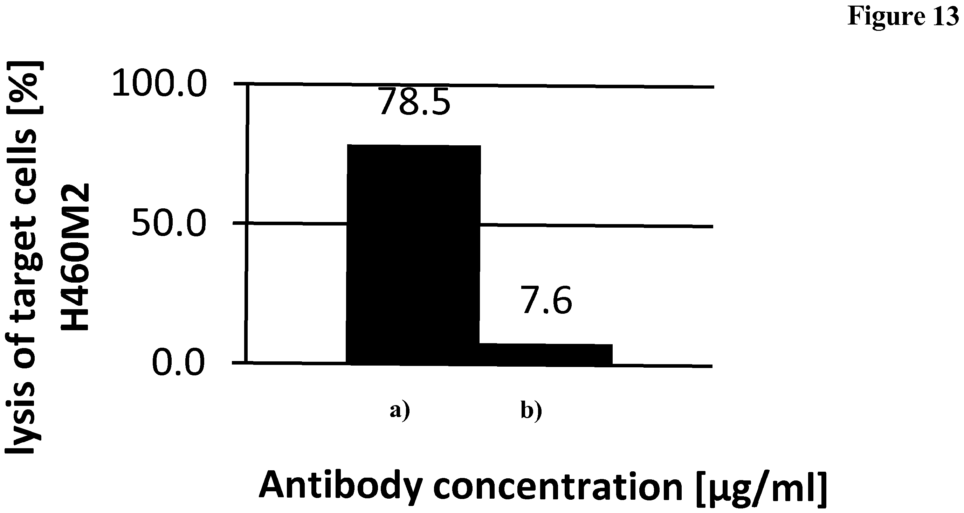

- **Figure 13**: Lysis of target cells after 6 hours incubation with a) a multi-function protein comprising a monovalent anti-IGF1R antibody and a fusion polypeptide comprising a CMV-derived peptide and b) an anti-IGF-1R antibody at a ratio of target to effector cells of 1:1.5.

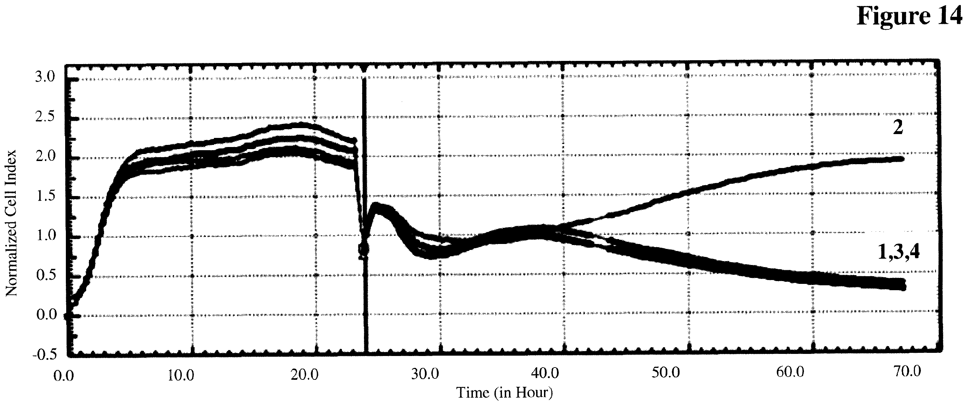

- **Figure 14**: Course of normalized cell index for Colo38 cells incubated with MHC-I-anti-MCSP multi-function proteins; 1 µg/ml multi-function protein concentration (MHCI-0008 (1), MHCI-0010 (2), MHCI-0030 (3), MHCI-0031(4)), effector to target cell ratio of 10:1; PBMCs from Donor 3 (200.000 cells, Donor 3 is CMV-positive but EBV negative) and melanoma tumor cell line Colo38 (20.000 cells) and per 96 well, data are triplicates.

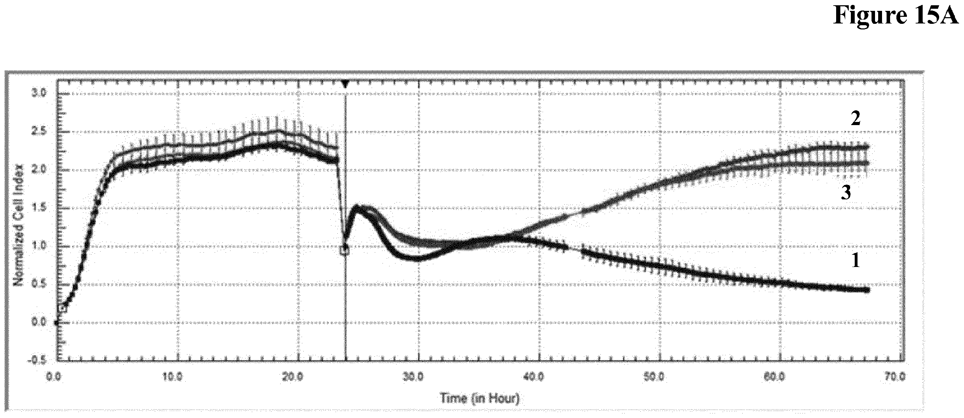

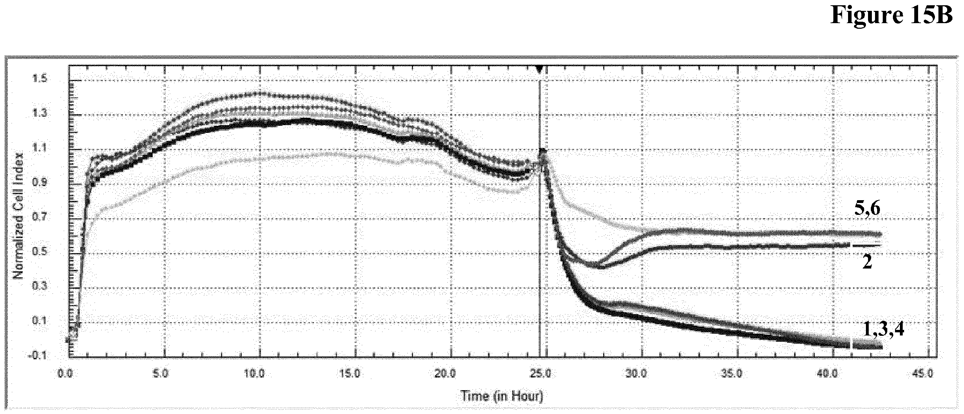

- **Figure 15**: A: Course of normalized cell index for Colo38 cells incubated with MHC-I-anti-MCSP multi-function proteins; 1 µg/ml multi-function protein concentration (MHCI-0008 (1), MHCI-0010 (2), PBMCs only (3)), effector to target cell ratio of 10:1; PBMCs from Donor 3 (200.000 cells, Donor 3 is CMV-positive but EBV negative) and melanoma tumor cell line Colo38 (20.000 cells) and per 96 well, data are triplicates.
B: Course of normalized cell index for WM266 cells incubated with MHC-I-anti-MCSP multi-function proteins; 1 µg/ml multi-function protein concentration (MHCI-0008 (1), MHCI-0010 (2), MHCI-0030 (3), MHCI-0031 (4), target cells alone (5), target cells + T-cells (6)), effector to target cell ratio of 10:1; PBMCs from Donor 3 (200.000 cells, Donor 3 is CMV-positive but EBV negative) and melanoma tumor cell line MW266 (20.000 cells) and per 96 well, data are triplicates.

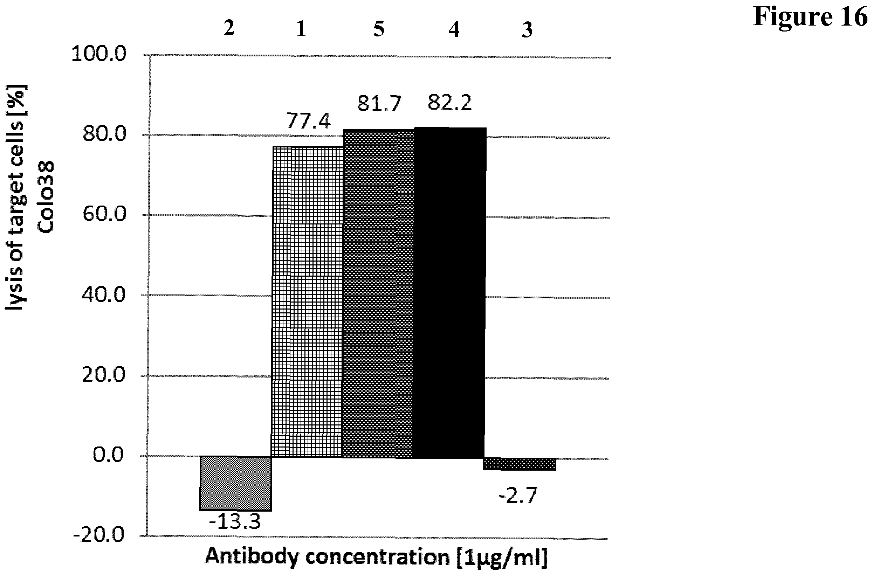

- **Figure 16**: Lysis of target cells after 42 hours of incubation with multi-function protein in the presence of non-stimulated PBMCs by the multi-function proteins MHCI-0008 (monovalent, CMV peptide loaded, 1), MHCI-0010 (monovalent, EBV peptide loaded control, 2), MHC-0026 (bivalent, CMV peptide loaded, non-binding control, 3), MHCI-0030 (monovalent, CMV peptide loaded, active, 4) and MHCI-0031 (bivalent, CMV peptide loaded, active, 5).

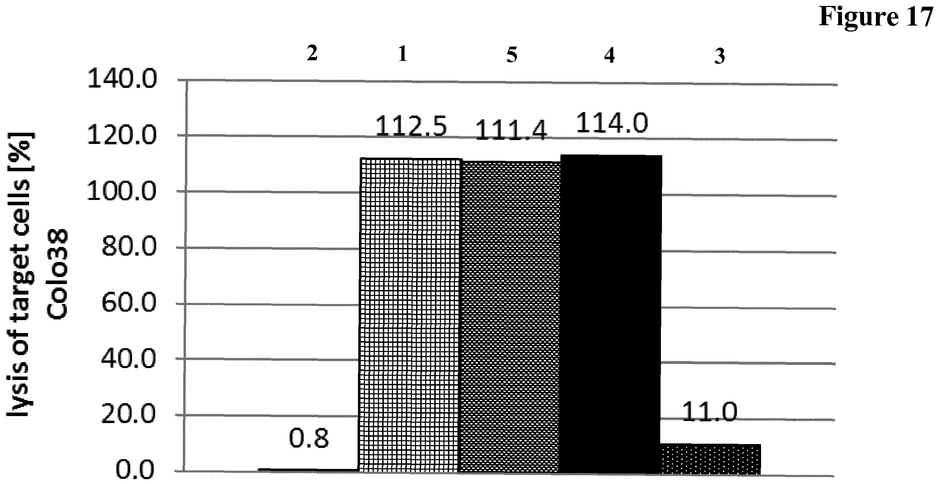

- **Figure 17**: LDH release after 48 hours of incubation with multi-function protein effected in the presence of non-stimulated PBMCs by the multi-function proteins MHCI-0008 (monovalent, CMV peptide loaded, 1), MHCI-0010 (monovalent, EBV peptide loaded control, 2), MHC-0026 (bivalent, CMV peptide loaded, non-binding control, 3), MHCI-0030 (monovalent, CMV peptide loaded, active, 4) and MHCI-0031 (bivalent, CMV peptide loaded, active, 5).

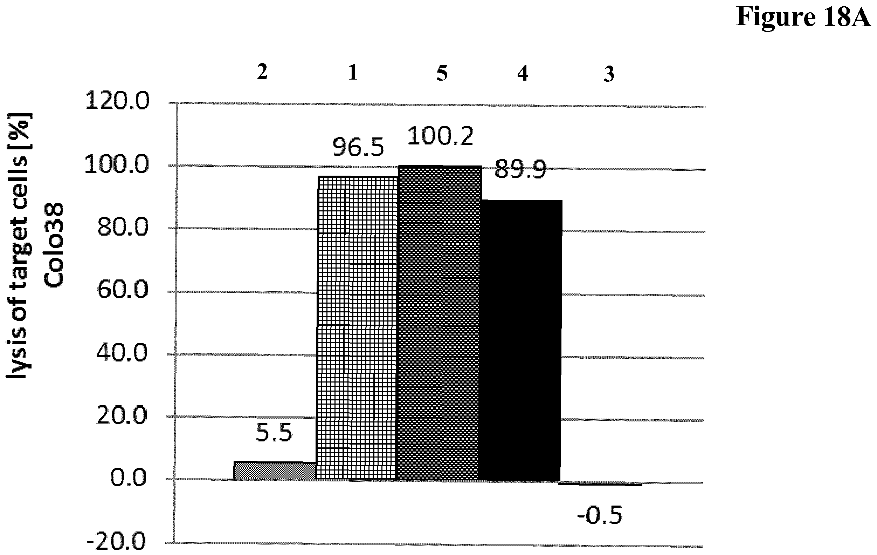

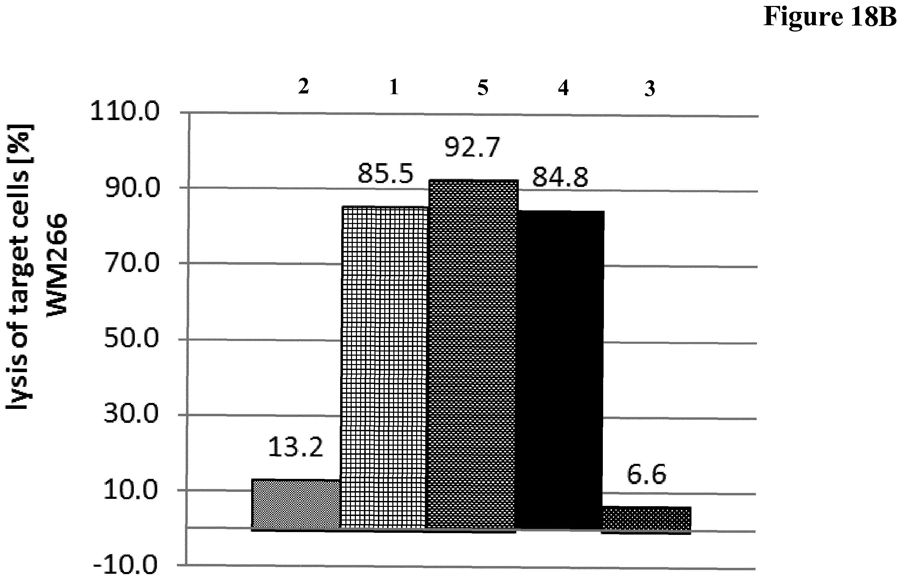

- **Figure 18**: A: Lysis of Colo38 cells after 10 hours of incubation with multi-function protein in the presence of stimulated PBMCs by the multi-function proteins MHCI-0008 (monovalent, CMV peptide loaded, 1), MHCI-0010 (monovalent, EBV peptide loaded control, 2), MHC-0026 (bivalent, CMV peptide loaded, non-binding control, 3), MHCI-0030 (monovalent, CMV peptide loaded, active, 4) and MHCI-0031 (bivalent, CMV peptide loaded, active, 5) at a concentration of 1µg/ml.
B: Lysis of WM266 cells after 10 hours of incubation with multi-function protein in the presence of stimulated PBMCs by the multi-function proteins MHCI-0008 (monovalent, CMV peptide loaded, 1), MHCI-0010 (monovalent, EBV peptide loaded control, 2), MHC-0026 (bivalent, CMV peptide loaded, non-binding control, 3), MHCI-0030 (monovalent, CMV peptide loaded, active, 4) and MHCI-0031 (bivalent, CMV peptide loaded, active, 5) at a concentration of 1µg/ml.

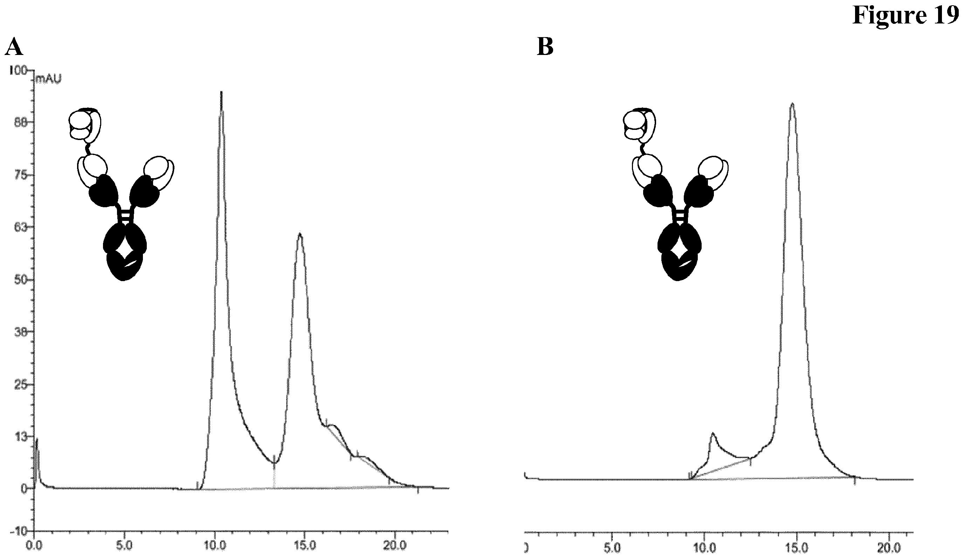

- **Figure 19**: Analytical size exclusion chromatogram after protein A affinity chromatography but prior to preparative size exclusion chromatography of a non-disulfide stabilized multi-function protein (A) and a disulfide stabilized multi-function protein (B).

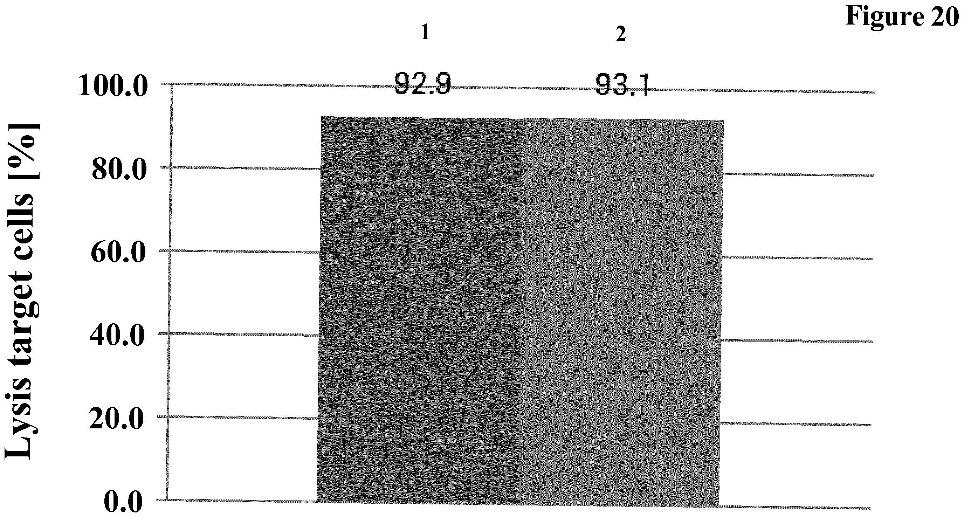

- **Figure 20**: Lysis of Colo38 cells with in the presence of stimulated PBMCs by the multi-function proteins MHCI-0031 (bivalent, CMV peptide loaded, active, non-disulfide-linked, 1) and MHCI-0054 (bivalent, CMV peptide loaded, active, disulfide-linked version of MHCI-0031, 2) at a concentration of 1µg/ml.

### Short description of the sequences

| | |
|---|---|
| **SEQ ID NO: 01 to 47** | Human cytomegalovirus-derived peptide. |
| **SEQ ID NO: 48** | Human immunodeficiency virus-derived peptide. |
| **SEQ ID NO: 49** | Human herpesvirus 4 derived peptide. |
| **SEQ ID NO: 50** | Influenza A virus-derived peptide. |
| **SEQ ID NO: 51** | Hepatitis-B-virus-derived peptide. |
| **SEQ ID NO: 52** | Human T-cell lymphotropic virus type 1 derived peptide. |
| **SEQ ID NO: 53** | V-jun Sarcoma Virus 17 Oncogene Homolog (JUN) derived peptide. |
| **SEQ ID NO: 54** | Human adenovirus type 3-derived peptide. |
| **SEQ ID NO: 55** | Hepatitis-C-virus-derived peptide. |
| **SEQ ID NO: 56 to 70** | Dengue virus-derived peptides. |
| **SEQ ID NO: 71** | Human β2-microglobulin amino acid sequence. |
| **SEQ ID NO: 72** | Human HLA-A*0201 α1 - α3 chain amino acid sequence. |
| **SEQ ID NO: 73-84** | Linker peptide amino acid sequences. |
| **SEQ ID NO: 85** | Human IgG1 CH2 domain amino acid sequence. |
| **SEQ ID NO: 86** | Human IgG1 CH2 domain amino acid sequence. |
| **SEQ ID NO: 87** | Human IgG1 Fc-region amino acid sequence. |
| **SEQ ID NO: 88** | Human IgG1 Fc-region L234A, L235A mutant amino acid sequence. |
| **SEQ ID NO: 89** | Human IgG1 Fc-region T366S, L368A, Y407V mutant amino acid sequence. |
| **SEQ ID NO: 90** | Human IgG1 Fc-region T366W mutant amino acid sequence. |
| **SEQ ID NO: 91** | Human IgG1 Fc-region L234A, L235A, T366S, L368A, Y407V mutant amino acid sequence. |
| **SEQ ID NO: 92** | Human IgG1 Fc-region L234A, L235A, T366W mutant amino acid sequence. |
| **SEQ ID NO: 93** | Human IgG1 Fc-region P329G mutant amino acid sequence. |
| **SEQ ID NO: 94** | Human IgG1 Fc-region L234A, L235A, P329G mutant amino acid sequence. |
| **SEQ ID NO: 95** | Human IgG1 Fc-region P329G, T366S, L368A, Y407V mutant amino acid sequence. |
| **SEQ ID NO: 96** | Human IgG1 Fc-region P329G, T366W mutant amino acid sequence. |
| **SEQ ID NO: 97** | Human IgG1 Fc-region L234A, L235A, P329G, T366S, L368A, Y407V mutant amino acid sequence. |
| **SEQ ID NO: 98** | Human IgG1 Fc-region L234A, L235A, P329G, T366W mutant amino acid sequence. |
| **SEQ ID NO: 99** | Human IgG4 Fc-region amino acid sequence. |
| **SEQ ID NO: 100** | Human IgG4 Fc-region S228P, L235E mutant amino acid sequence. |
| **SEQ ID NO: 101** | Human IgG4 Fc-region S228P, L235E, P329G mutant amino acid sequence. |
| **SEQ ID NO: 102** | Human IgG4 Fc-region S228P, L235E, P329G, T366S, L368A, Y407V mutant amino acid sequence. |
| **SEQ ID NO: 103** | Human IgG4 Fc-region S228P, L235E, P329G, T366W mutant amino acid sequence. |
| **SEQ ID NO: 104** | HVR-L1 |
| **SEQ ID NO: 105** | HVR-L2 |
| **SEQ ID NO: 106** | HVR-L3 |
| **SEQ ID NO: 107** | HVR-L1 |
| **SEQ ID NO: 108** | HVR-H1 |
| **SEQ ID NO: 109** | HVR-H2 |
| **SEQ ID NO: 110** | HVR-H3 |
| **SEQ ID NO: 111** | HVR-H1 |
| **SEQ ID NO: 112** | HVR-H2 |
| **SEQ ID NO: 113** | VL |
| **SEQ ID NO: 114** | VH |
| **SEQ ID NO: 115** | VL |
| **SEQ ID NO: 116** | VH |
| **SEQ ID NO: 117** | MHC-I-VH (MCSP)-IgG1 Fc-region L234A, L235A, P329G, T366S, L368A, Y407V mutant amino acid sequence. |
| **SEQ ID NO: 118** | VH(MCSP)-IgG1 Fc-region L234A, L235A, P329G, T366W mutant amino acid sequence. |
| **SEQ ID NO: 119** | VL(MCSP)-CL amino acid sequence. |
| **SEQ ID NO: 120** | Humanized anti-IGF-1R monoclonal light chain antibody amino acid sequence (kappa). |
| **SEQ ID NO: 121** | Humanized anti-IGF-1R monoclonal heavy chain antibody amino acid sequence (IgG1 L234A, L235A mutant). |
| **SEQ ID NO: 122** | Humanized anti-IGF-1R monoclonal heavy chain antibody amino acid sequence (IgG1 L234A, L235A mutant and knob variant). |
| **SEQ ID NO: 123** | Humanized anti-IGF-1R monoclonal heavy chain antibody amino acid sequence (IgG1 L234A, L235A mutant and hole variant). |
| **SEQ ID NO: 124** | Human IgG1 Fc-region mutant hinge region and L234A, L235A mutant and knob variant. |
| **SEQ ID NO: 125** | Disulfide-stabilized single chain Fv of humanized anti-IGF-1R monoclonal antibody. |
| **SEQ ID NO: 126** | Murine anti-MCSP monoclonal light chain antibody amino acid sequence (kappa). |
| **SEQ ID NO: 127** | Humanized anti-MCSP monoclonal light chain antibody amino acid sequence (kappa). |
| **SEQ ID NO: 128** | Murine anti-MCSP monoclonal heavy chain antibody amino acid sequence (IgG1 L234A, L235A mutant). |
| **SEQ ID NO: 129** | Humanized anti-MCSP monoclonal heavy chain antibody amino acid sequence (IgG1 L234A, L235A mutant). |
| **SEQ ID NO: 130** | Murine anti-MCSP monoclonal heavy chain antibody amino acid sequence (IgG1 L234A, L235A mutant and knob variant). |
| **SEQ ID NO: 131** | Humanized anti-MCSP monoclonal heavy chain antibody amino acid sequence (IgG1 L234A, L235A mutant and knob variant). |
| **SEQ ID NO: 132** | Murine anti-MCSP monoclonal heavy chain antibody amino acid sequence (IgG1 L234A, L235A mutant and hole variant). |
| **SEQ ID NO: 133** | Humanized anti-MCSP monoclonal heavy chain antibody amino acid sequence (IgG1 L234A, L235A mutant and hole variant). |
| **SEQ ID NO: 134** | Disulfide-stabilized single chain Fv of murine anti-MCSP monoclonal antibody. |
| **SEQ ID NO: 135** | Disulfide-stabilized single chain Fv of humanized anti-MCSP monoclonal antibody. |
| **SEQ ID NO: 136** | Linker peptide 13. |
| **SEQ ID NO: 137** | Disulfide-stabilized MHC-I-VH (MCSP)-IgG1 Fc-region L234A, L235A, P329G, T366S, L368A, Y407V mutant amino acid sequence. |
| **SEQ ID NO: 138** | Amino acid sequence of human MCSP. |

### I. DEFINITIONS

"Affinity" refers to the strength of the sum total of non-covalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

The term "amino acid" as used within this application denotes the group of carboxy α-amino acids, which directly or in form of a precursor can be encoded by a nucleic acid. The individual amino acids are encoded by nucleic acids consisting of three nucleotides, so called codons or base-triplets. Each amino acid is encoded by at least one codon. This is known as "degeneration of the genetic code". The term "amino acid" as used within this application denotes the naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The terms "anti-target antibody" and "an antibody that binds to a target" refer to an antibody that is capable of binding a target with sufficient affinity such that the antibody is useful as a diagnostic and/or therapeutic agent in targeting the target. In certain embodiments, an antibody that binds to the target has a dissociation constant (Kd) of ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); single domain antibodies; and multispecific antibodies formed from antibody fragments.

The term "antigen binding site" denotes a proteinaceous moiety that can specifically bind to a target. Exemplary antigen binding sites are peptides, antibody fragments, domain antibodies, or variable domains of single chain antibodies (e.g. camel or shark antibodies). The antigen binding site can be a naturally occurring antigen binding site or an engineered antigen binding site. Exemplary engineered antigen binding sites are DARPINs, domain exchanged antibodies or domain exchanged antibody fragments, and dual variable domain antibodies.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively.

The term "class I MHC molecule with a relative frequency of" denotes that the respective class I MHC molecule has a frequency of occurrence in a specific population of humans or within all humans of the given relative frequency. That is a class I MHC molecule with a relative frequency of 10 % or more can be found in 10 % or more of all humans of a specific population, such as e.g. in 27.2 % of all humans of European origin.

The term "comprising" as used herein encompasses the tem "consisting", i.e. a change from comprising to consisting is considered to be a limitation.

The "conjugation" of a multi-function protein to its conjugation partner can be performed by different methods, such as chemical binding, or binding via a specific binding pair. The term "conjugation partner" denotes e.g. polypeptides, detectable labels, members of specific binding pairs. In one embodiment the conjugation of multi-function protein to its conjugation partner is performed by chemically binding via N-terminal and/or ε-amino groups (lysine), ε-amino groups of different lysins, carboxy-, sulfhydryl-, hydroxyl-, and/or phenolic functional groups of the amino acid sequence of the parts of the multi-function protein, and/or sugar alcohol groups of the carbohydrate structure of the multi-function protein. In one embodiment the multi-function protein is conjugated to its conjugation partner via a specific binding pair.

The term "cytotoxic agent" as used herein refers to a substance that inhibits or prevents a cellular function and/or causes cell death or destruction. Cytotoxic agents include but are not limited to radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents.

Chromogens (fluorescent or luminescent groups and dyes), enzymes, NMR-active groups or metal particles, haptens, e.g. digoxigenin, are examples of "detectable labels". The detectable label can also be a photoactivatable crosslinking group, e.g. an azido or an azirine group. Metal chelates which can be detected by electrochemiluminescense are also suitable signal-emitting groups, with particular interest being given to ruthenium chelates, e.g. a ruthenium (bispyridyl)₃²⁺ chelate. Suitable ruthenium labeling groups are described, for example, in EP 0 580 979, WO 90/05301, WO 90/11511, and WO 92/14138. For direct detection the labeling group can be selected from any known detectable marker groups, such as dyes, luminescent labeling groups such as chemiluminescent groups, e.g. acridinium esters or dioxetanes, or fluorescent dyes, e.g. fluorescein, coumarin, rhodamine, oxazine, resorufin, cyanine and derivatives thereof. Other examples of labeling groups are luminescent metal complexes, such as ruthenium or europium complexes, enzymes, e.g. as used for ELISA or for CEDIA (Cloned Enzyme Donor Immunoassay, e.g. EP-A-0 061 888), and radioisotopes.

"Effector functions" refer to those biological activities attributable to the Fc-region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "expression" as used herein refers to transcription and/or translation and secretion processes occurring within a cell. The level of transcription of a nucleic acid sequence of interest in a cell can be determined on the basis of the amount of corresponding mRNA that is present in the cell. For example, mRNA transcribed from a sequence of interest can be quantitated by RT-PCR or by Northern hybridization (see Sambrook, J., et al., Molecular Cloning: A Laboratory Manual, Second Edition, Cold Spring Harbor Laboratory Press, Cold Spring Harbor, N.Y. (1989)). Polypeptides encoded by a nucleic acid can be quantitated by various methods, e.g. by ELISA, by assaying for the biological activity of the polypeptide, or by employing assays that are independent of such activity, such as Western blotting or radioimmunoassay, using immunoglobulins that recognize and bind to the polypeptide (see Sambrook, et al., (1989), supra).

An "expression cassette" denotes a construct that contains the necessary regulatory elements, such as promoter and polyadenylation site, for expression of at least the contained nucleic acid in a cell.

The term "expression machinery" denotes the sum of the enzymes, cofactors, etc. of a cell that is involved in the steps beginning with the transcription step of a nucleic acid or gene (i.e. also called "gene expression machinery") to the post-translational modification of the polypeptide encoded by the nucleic acid. The expression machinery e.g. comprises the steps of transcription of DNA into pre-mRNA, pre-mRNA splicing to mature mRNA, translation into a polypeptide of the mRNA, and post translational modification of the polypeptide.

An "expression plasmid" is a nucleic acid providing all required elements for the expression of the comprised structural gene(s) in a host cell. Typically, an expression plasmid comprises a prokaryotic plasmid propagation unit, e.g. for E. coli, comprising an origin of replication, and a selectable marker, an eukaryotic selection marker, and one or more expression cassettes for the expression of the structural gene(s) of interest each comprising a promoter, a structural gene, and a transcription terminator including a polyadenylation signal. Gene expression is usually placed under the control of a promoter, and such a structural gene is said to be "operably linked to" the promoter. Similarly, a regulatory element and a core promoter are operably linked if the regulatory element modulates the activity of the core promoter.

The term "Fc-region" denotes the C-terminal region of an immunoglobulin heavy chain. The Fc-region is a dimeric molecule comprising two disulfide-linked antibody heavy chain polypeptides. An Fc-region can be generated by papain digestion, or IdeS digestion, or trypsin digestion of an intact (full length) antibody or can be produced recombinantly.

The Fc-region obtainable from a full length antibody or immunoglobulin comprises at least residues 226 (Cys) to the C-terminus of the full length heavy chain and, thus, comprises a part of the hinge region and two or three constant domains, i.e. a CH2 domain, a CH3 domain, and an additional/extra CH4 domain in case of IgE and IgM class antibodies. However, the C-terminal lysine (Lys447) of the Fc-region may or may not be present. Unless otherwise specified herein, numbering of amino acid residues in the Fc-region or constant region is according to the EU numbering system, also called the EU index, as described in Kabat, E.A., et al., Sequences of Proteins of Immunological Interest, 5th ed., Public Health Service, National Institutes of Health, Bethesda, MD (1991), NIH Publication 91-3242.

The formation of the dimeric Fc-region comprising two identical or non-identical antibody heavy chain Fc-region polypeptides is mediated by the non-covalent dimerization of the comprised CH3 domains (for involved amino acid residues see e.g. Dall'Acqua, W., Biochem. 37 (1998) 9266-9273). The Fc-region is covalently stabilized by the formation of disulfide bonds in the hinge region (see e.g. Huber, R., et al., Nature 264 (1976) 415-420; Thies, M.J., et al., J. Mol. Biol. 293 (1999) 67-79).

It is known from US 5,648,260 and US 5,624,821 that the modification of defined amino acid residues in the Fc-region results in phenotypic effects.

The multi-function protein as reported herein may comprise in one embodiment as antibody heavy chain hinge region polypeptide a human Fc-region or an Fc-region derived from human origin. In a further embodiment the Fc-region is either an Fc-region of a human antibody of the subclass IgG4 or an Fc-region of a human antibody of the subclass IgG1, IgG2, or IgG3, which is modified in such a way that no Fcγ receptor (e.g. FcγRIIIa) binding and/or no C1q binding can be detected. In one embodiment the Fc-region is a human Fc-region and especially either from human IgG4 subclass or a mutated Fc-region from human IgG1 subclass. In one embodiment the Fc-region is from human IgG1 subclass with mutations L234A and L235A and P329G. While IgG4 shows reduced Fcγ receptor (FcγRIIIa) binding, antibodies of other IgG subclasses show strong binding. However Pro238, Asp265, Asp270, Asn297 (loss of Fc carbohydrate), Pro329, Leu234, Leu235, Gly236, Gly237, Ile253, Ser254, Lys288, Thr307, Gln311, Asn434, or/and His435 are residues which, if altered, provide also reduced Fcγ receptor binding (Shields, R.L., et al., J. Biol. Chem. 276 (2001) 6591-6604; Lund, J., et al., FASEB J. 9 (1995) 115-119; Morgan, A., et al., Immunology 86 (1995) 319-324; EP 0 307 434). In one embodiment a multi-function protein as reported herein is in regard to Fcγ receptor binding of IgG4 subclass or of IgG1 or IgG2 subclass, with a mutation in L234, L235, P329 and/or D265, and/or contains the PVA236 mutation. In one embodiment the mutations are S228P, L234A, L235A, L235E, PVA236 (PVA236 denotes that the amino acid sequence ELLG (given in one letter amino acid code) from amino acid position 233 to 236 of IgG1 or EFLG of IgG4 is replaced by PVA) and/or P329G. In one embodiment the mutations are S228P and P329G of IgG4, and L234A, L235A and P329G of IgG1. The Fc-region of an antibody is directly involved in ADCC (antibody-dependent cell-mediated cytotoxicity) and CDC (complement-dependent cytotoxicity). A multi-function protein which does not bind Fcγ receptor and/or complement factor C1q does not elicit antibody-dependent cellular cytotoxicity (ADCC) and/or complement dependent cytotoxicity (CDC).

A polypeptide chain of a wild-type human Fc-region of the IgG1 isotype has the following amino acid sequence:

A polypeptide chain of a variant human Fc-region of the IgG1 isotype with the mutations L234A, L235A has the following amino acid sequence:

A polypeptide chain of a variant human Fc-region of the IgG1 isotype with T366S, L368A and Y407V mutations has the following amino acid sequence:

A polypeptide chain of a variant human Fc-region of the IgG1 isotype with a T366W mutation has the following amino acid sequence:

A polypeptide chain of a variant human Fc-region of the IgG1 isotype with a L234A, L235A and T366S, L368A and Y407V mutations has the following amino acid sequence:

A polypeptide chain of a variant human Fc-region of the IgG1 isotype with a L234A, L235A and T366W mutation has the following amino acid sequence:

A polypeptide chain of a variant human Fc-region of the IgG1 isotype with a P329G mutation has the following amino acid sequence:

A polypeptide chain of a variant human Fc-region of the IgG1 isotype with a L234A, L235A and P329G mutation has the following amino acid sequence:

A polypeptide chain of a variant human Fc-region of the IgG1 isotype with a P239G and T366S, L368A and Y407V mutation has the following amino acid sequence:

A polypeptide chain of a variant human Fc-region of the IgG1 isotype with a P329G and T366W mutation has the following amino acid sequence:

A polypeptide chain of a variant human Fc-region of the IgG1 isotype with a L234A, L235A, P329G and T366S, L368A and Y407V mutation has the following amino acid sequence:

A polypeptide chain of a variant human Fc-region of the IgG1 isotype with a L234A, L235A, P329G and T366W mutation has the following amino acid sequence:

A polypeptide chain of a wild-type human Fc-region of the IgG4 isotype has the following amino acid sequence:

A polypeptide chain of a variant human Fc-region of the IgG4 isotype with a S228P and L235E mutation has the following amino acid sequence:

A polypeptide chain of a variant human Fc-region of the IgG4 isotype with a S228P, L235E and P329G mutation has the following amino acid sequence:

A polypeptide chain of a variant human Fc-region of the IgG4 isotype with a S228P, L235E, P329G and T366S, L368A and Y407V mutation has the following amino acid sequence:

A polypeptide chain of a variant human Fc-region of the IgG4 isotype with a S228P, L235E, P329G and T366W mutation has the following amino acid sequence:

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "cell" includes both prokaryotic cells, which are used for propagation of plasmids, and eukaryotic cells, which are used for the expression of a nucleic acid. In one embodiment the eukaryotic cell is a mammalian cell. In one embodiment the mammalian cell is selected from the group of mammalian cells comprising CHO cells (e.g. CHO K1, CHO DG44), BHK cells, NS0 cells, Sp2/0 cells, HEK 293 cells, HEK 293 EBNA cells, PER.C6® cells, and COS cells.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

An "immunoconjugate" denotes a multi-function protein as reported herein conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" multi-function protein is one which has been separated from a component of its natural environment. In some embodiments, a multi-function protein is purified to greater than 95 % or 99 % purity as determined by, for example, electrophoretic (e.g., SDS-PAGE, isoelectric focusing (IEF), capillary electrophoresis) or chromatographic (e.g., ion exchange or reverse phase HPLC).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "MCSP", as used herein, refers to any native MCSP (Melanoma Chondroitin Sulfate Proteoglycan) from any vertebrate source, including mammals such as primates (e.g. humans) and rodents (e.g., mice and rats), unless otherwise indicated. The term encompasses "full-length", unprocessed MCSP as well as any form of MCSP that results from processing in the cell. The term also encompasses naturally occurring variants of MCSP, e.g., splice variants or allelic variants. MCSP is also known as chondroitin sulfate proteoglycan 4 (CSPG4), chondroitin sulfate proteoglycan NG2, high molecular weight-melanoma associated antigen (HMW-MAA), and melanoma chondroitin sulfate proteoglycan. The amino acid sequence of an exemplary human MCSP is shown in SEQ ID NO: 1. See also Pluschke, G., et al., Proc. Natl. Acad. Sci. USA 93 (1996) 9710-9715, Staub, E., et al., FEBS Lett. 527 (2002) 114-118, and GenBank Accession No: NP_001888.

The term "one antigen presenting domain" denotes exactly one, i.e. a single, antigen presenting domain as defined and excludes the presence of a further, i.e. second, antigen presenting domains defined. The term "one" denotes "exactly one" or "a single".

"Operably linked" refers to a juxtaposition of two or more components, wherein the components so described are in a relationship permitting them to function in their intended manner. For example, a promoter and/or enhancer are operably linked to a coding sequence, if it acts in cis to control or modulate the transcription of the linked sequence. Generally, but not necessarily, the DNA sequences that are "operably linked" are contiguous and, where necessary to join two protein encoding regions such as a secretory leader and a polypeptide, contiguous and in (reading) frame. However, although an operably linked promoter is generally located upstream of the coding sequence, it is not necessarily contiguous with it. Enhancers do not have to be contiguous. An enhancer is operably linked to a coding sequence if the enhancer increases transcription of the coding sequence. Operably linked enhancers can be located upstream, within or downstream of coding sequences and at considerable distance from the promoter. A polyadenylation site is operably linked to a coding sequence if it is located at the downstream end of the coding sequence such that transcription proceeds through the coding sequence into the polyadenylation sequence. A translation stop codon is operably linked to an exonic nucleic acid sequence if it is located at the downstream end (3' end) of the coding sequence such that translation proceeds through the coding sequence to the stop codon and is terminated there. Linking is accomplished by recombinant methods known in the art, e.g., using PCR methodology and/or by ligation at convenient restriction sites. If convenient restriction sites do not exist, then synthetic oligonucleotide adaptors or linkers are used in accord with conventional practice.

The term "package insert" is used to refer to instructions customarily included in commercial packages of therapeutic products, that contain information about the indications, usage, dosage, administration, combination therapy, contraindications and/or warnings concerning the use of such therapeutic products.

The term "peptide linker" denotes amino acid sequences of natural and/or synthetic origin. They consist of a linear amino acid chain wherein the 20 naturally occurring amino acids are the monomeric building blocks. The peptide linker has a length of from 1 to 50 amino acids, in one embodiment between 1 and 28 amino acids, in a further embodiment between 2 and 25 amino acids. The peptide linker may contain repetitive amino acid sequences or sequences of naturally occurring polypeptides. The linker has the function to ensure that polypeptides conjugated to each other can perform their biological activity by allowing the polypeptides to fold correctly and to be presented properly. In one embodiment the peptide linker is rich in glycine, glutamine, and/or serine residues. These residues are arranged e.g. in small repetitive units of up to five amino acids, such as GS (SEQ ID NO: 73), GGS (SEQ ID NO: 74), GGGS (SEQ ID NO: 75), and GGGGS (SEQ ID NO: 80). This small repetitive unit may be repeated for one to five times. At the amino- and/or carboxy-terminal ends of the multimeric unit up to six additional arbitrary, naturally occurring amino acids may be added. Other synthetic peptidic linkers are composed of a single amino acid, which is repeated between 10 to 20 times and may comprise at the amino- and/or carboxy-terminal end up to six additional arbitrary, naturally occurring amino acids. All peptidic linkers can be encoded by a nucleic acid molecule and therefore can be recombinantly expressed. As the linkers are themselves peptides, the polypeptide connected by the linker are connected to the linker via a peptide bond that is formed between two amino acids.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

A "polypeptide" is a polymer consisting of amino acids joined by peptide bonds, whether produced naturally or synthetically. Polypeptides of less than about 25 amino acid residues may be referred to as "peptides", whereas molecules consisting of two or more polypeptides or comprising one polypeptide of more than 100 amino acid residues may be referred to as "proteins". A polypeptide may also comprise non-amino acid components, such as carbohydrate groups, metal ions, or carboxylic acid esters. The non-amino acid components may be added by the cell, in which the polypeptide is expressed, and may vary with the type of cell. Polypeptides are defined herein in terms of their amino acid backbone structure or the nucleic acid encoding the same. Additions such as carbohydrate groups are generally not specified, but may be present nonetheless.

A "structural gene" denotes the region of a gene without a signal sequence, i.e. the coding region.

The term "T-cell response eliciting peptide" denotes a peptide that is presented in the peptide-binding grove of a class I MHC multi-function protein and which is recognized by circulating memory or effector T-cells. Recognition of the peptide results in an immune response effecting the removal of the cell presenting such a peptide-class I MHC multi-function protein.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt, T.J., et al., Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano, S., et al., J. Immunol. 150 (1993) 880-887; Clackson, T., et al., Nature 352 (1991) 624-628).

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

### II. COMPOSITIONS AND METHODS

During recombinant production covalent peptide-MHC-immunoglobulin conjugates cannot be expressed at levels comparable to normal full length antibodies. This is only possible for certain specific formats. Full length antibody (IgG)-MHC fusions cannot be expressed in bacteria. Further, full length antibody (IgG) peptide-MHC-fusions cannot be expressed at significant levels. So far only fusions of antibody fragments (scFv and Fab, lacking a hinge and an Fc-region) could be expressed as MHC class I fusions in bacteria (preferably in E. coli). The expression was only successful via inclusion bodies followed by a complex refolding procedure which is a technical difficult process especially at larger scale.

An alternative is the recombinant expression of soluble MHC complexes in bacteria as such, i.e. without being fused to a full length antibody or antibody fragment, followed by a chemical conjugation or Biotin-Streptavidin mediated non-covalent coupling to an antibody fragment. Chemical conjugations are not site specific and lead to a large product heterogeneity compared to recombinantly produced fusion polypeptides.

Expression in eukaryotic was limited so far due to no or low expression levels. So far the only expression in mammalian cells is described in Greten, T.F. et al. (J. Immunol. Meth. 271 (2002) 125-135). However, the obtained yields are very low, and actually too low for a technical process and much lower than for normal antibodies.

It has now been found that full length antibodies (IgG) fused to peptide-MHC complexes can be recombinantly expressed at high levels comparable to normal antibodies when the fusion polypeptide carries only a single MHC complex.

### A. Exemplary multi-function protein

Herein is reported an antigen binding multi-function protein comprising as first part an antibody derived part that specifically binds to a target antigen, and as second part a virus-derived peptide linked to a MHC class I protein complex. If the multi-function protein as reported herein comprises one or more further antigen presenting domain(s) these further antigen presenting domains do not comprise an MHC molecule. I.e. the multi-function protein as reported herein comprises exactly one antigen presenting domain comprising an MHC molecule.

The term "MHC molecule" denotes a fusion polypeptide comprising the extracellular domains α1, α2, and α3 of a class I MHC molecule. In one embodiment the extracellular domains α1, α2, and α3 are of a human class I MHC molecule.

With the multi-function protein as reported herein existing virus-specific circulating cytotoxic T-cells (T-memory-cells and/or T-effector-cells) of an individual can be directed to cells expressing the target antigen, to which the antibody derived part of the multi-function protein specifically binds to, by dressing these cells with MHC class I multi-function complexes mimicking an acute viral infection.

In one aspect, the invention is based, in part, on the finding that a multi-function protein as reported herein, which comprises as first part a virus-derived peptide linked to a MHC class I protein and as second part an antibody derived disulfide-linked molecule, can be used to direct existing virus-specific cytotoxic T-cells of an individual to cells expressing a target antigen mimicking an acute viral infection and thereby removal of the cells expressing the target antigen can be initiated.

In certain embodiments a multi-function protein comprising an antigen presenting domain comprising (i) a virus-derived peptide, (ii) the soluble HLA-A allele A*0201, and (iii) beta-2-microglobulin, is provided.

Multi-function proteins as reported herein are useful, e.g., for the diagnosis or treatment of various diseases like cancer or viral infections.

In one aspect, the invention provides a multi-functions protein that binds (i) to a cell surface antigen and (ii) to cytotoxic T-cells.

The multi-function proteins as reported herein exploit a naturally occurring, highly effective anti-viral immune response to remove/disintegrate target cells, e.g. tumor cells or virus infected cells. The cell removal is achieved by using an individual's own very powerful circulating T-cells that do not need any co-stimulation for their activation. Additionally a small number of therapeutic molecules are needed on the cell surface for mechanism of action (see e.g. Mottez, E., et al., J. Exp. Med. 181 (1995) 493-502).

During the treatment the multi-function protein can trigger the anti-viral immune response of the individual similar to an immunization. Thereby multiple treatments/applications can enhance the efficacy of the treatment. Thus, an immunization as pretreatment can be used in order to enhance efficacy.

The targeting antigen binding site needs to be highly cell or antigen specific to limit toxicity and side effects.

Thus by using a multi-function protein as reported herein
(i) only a highly specific T-cell population is activated (CD8 positive effector/memory cells specific for a single virus-derived peptide displayed in the MHC-I protein complex of the multi-function protein), all other CD3⁺-cells are not affected (CD4⁺-T-cells: TH1, TH2, TH17, regulatory T-cells);
(ii) the natural response of the individual's immune system is mimicked (normal removal of virus-infected cells); and
(iii) the response to the application/treatment of/with the multi-function protein will be low at the beginning but can boost during treatment (specific T-cells will be activated and expand in number), therewith initially infusion reactions and initial cytokine release can be reduced.

Thus the method reported herein may comprise the step of stimulating CD8-positive cytotoxic T-cell by application of a selected virus-derived peptide, e.g. a human cytomegalovirus (huCMV) derived peptide. In one embodiment the peptide has the amino acid sequence of SEQ ID NO: 01.

It has been shown that the activated CMV-peptide specific T-cells mediate effective tumor cell removal in vitro (tumor cells loaded with the CMV-derived peptide in vitro).

Virus-infected cells present a complex of virus-derived peptides with MHC class I proteins on their cell surface. These are recognized by specific CD8⁺ T-cells which remove/deplete the virus-derived peptide presenting cells. Cytolytic (cytotoxic) CD8⁺-T-cells (CTL) recognize peptides in MHC class I proteins by their specific T-cell-receptor. The CTLs trigger removal of virus infected cells without the requirement of a co-stimulating signal.

Effector cells, e.g. peripheral blood mononuclear cells (PBMC) or FACS-sorted CD8⁺-T-cells, which can be pre-stimulated with the CMV-derived peptide as comprised in the fusion polypeptide as reported herein can be used.

The HLA-allotype of an individual to be treated has to be recognized.

According to NCBI the HLA-allotypes with a frequency of 10 % or more are distributed as it is shown in the following table.

**Table.**

| **HLA-allotype** | **Australian frequency** | **European frequency** | **North American frequency** | **South-East Asian frequency** |
|---|---|---|---|---|
| **HLA-** | **[%]** | **[%]** | **[%]** | **[%]** |
| A*01:01 | | 16.4 | | |
| A*02:01 | 12.7 | 27.2 | 19.7 | |
| A*02:04 | | | | |
| A*03:01 | | 14.1 | | |
| A*11:01 | 13.5 | | | 20.4 |
| A*24:02 | 25.9 | | 37.7 | 29.9 |
| A*31:01:02 | | | | |
| A*34:01:01 | 40.1 | | | |
| B*07:02 | | 13.9 | | |
| B*08:01 | | 11.8 | | |
| B*13:01 | 23.8 | | | |
| B*15:04 | | | | 11.7 |
| B*15:21 | 10.6 | | | |
| B*44:02 | | 10.6 | | |
| B*56:01 | 16.1 | | | |
| B*56:02 | 10.3 | | | |
| C*01:02 | 24.6 | | | 13.3 |
| C*02:02 | | | 12.7 | |
| C*03:03 | | | | |
| C*03:04 | | | 20.4 | 17.3 |
| C*04:01 | 26.0 | 10.1 | 15.0 | |
| C*04:03 | 13.9 | | | |
| C*05:01 | | 10.6 | | |
| C*06:02 | | | | |
| C*07:01 | | 17.0 | | |
| C*07:02 | | 15.9 | 10.2 | 18.9 |
| C*08:01 | | | | 12.8 |
| C*15:02 | 16.5 | | | |

The term "frequency" denotes the frequency with which a specific HLA-allotype occurs within the entire human population. Thus, the term "with a relative frequency of 1 % or more" denotes that the respective HLA-allotype has an occurrence within the entire human population of 1 % or more. In one embodiment of all aspects the relative frequency is the relative frequency in the human population. In one embodiment the relative frequency is the relative frequency in the European population. In one embodiment the relative frequency is the relative frequency in the North-American population.

Thus one aspect of the invention is a multi-function protein, characterized in that it comprises
- exactly one antigen presenting domain,
- exactly one antibody Fc-region, and
- at least one antigen binding site,
wherein the antigen presenting domain comprises in N- to C-terminal direction
either
   (i) a T-cell response eliciting peptide,
   (ii) a β2-microglobulin, and
   (iii) the extracellular domains α1, α2, and α3 of a class I MHC molecule selected from the group comprising HLA-A*0201, HLA-A*1101, HLA-A*2402, HLA-A*340101, HLA-C*0304, HLA-C*0401, and HLA-C*0702,
   or
   (i) a T-cell response eliciting peptide,
   (ii) the extracellular domains α1, α2, and α3 of a class I MHC molecule selected from the group comprising HLA-A*0201, HLA-A*1101, HLA-A*2402, HLA-A*340101, HLA-C*0304, HLA-C*0401, and HLA-C*0702, and
   (iii) a β2-microglobulin,
wherein the antigen binding site binds to melanoma-associated chondroitin sulfate proteoglycan (MCSP)

In one embodiment the peptide is a T-cell-response eliciting peptide.

In one embodiment the T-cell-response eliciting peptide is a virus-derived peptide. In one embodiment the virus is selected from adenovirus, human herpesvirus 1, human herpesvirus 2, human herpesvirus 4 (Epstein-Barr virus), hepatitis-B-virus, hepatitis-C-virus, human cytomegalovirus, human immunodeficiency virus, human papillomavirus type 16, human papillomavirus type 18, human papillomavirus type 31, human papillomavirus type 33, human papillomavirus type 35, human papillomavirus type 39, human papillomavirus type 45, human papillomavirus type 51, human papillomavirus type 52, human papillomavirus type 56, human papillomavirus type 58, human papillomavirus type 59, human papillomavirus type 68, human papillomavirus type 73, human papillomavirus type 82, human T-cell lymphotropic virus type I, human influenza A virus, human influenza B virus, or vaccinia virus.

In one embodiment the virus-derived peptide is selected from NLVPMVATV (SEQ ID NO: 01), SLYNTVATL (SEQ ID NO: 48), GLCTLVAML (SEQ ID NO: 49), GILGFVFTL (SEQ ID NO: 50), STNRQSGRQ (SEQ ID NO: 51), LLFGYPVYV (SEQ ID NO: 52), FAEGFVRAL (SEQ ID NO: 53), LIVIGILIL (SEQ ID NO: 54), or ILHTPGCV (SEQ ID NO: 55).

In one embodiment the β2-microglobulin is human β2-microglobulin. In one embodiment the β2-microglobulin is consisting of the amino acid sequence of SEQ ID NO: 71.

In one embodiment the class I MHC molecule is human HLA-A*0201. In one embodiment the extracellular domains α1, α2, and α3 of a class I MHC molecule is consisting of the amino acid sequence of SEQ ID NO: 72.

In one embodiment the virus-derived peptide is fused to the β2-microglobulin via a first linker peptide.

In one embodiment the β2-microglobulin is fused to the extracellular domain α1 of a class I MHC molecule via a second linker peptide.

In one embodiment the extracellular domain α3 of a class I MHC molecule is fused to the polypeptide (either disulfide-linked or not disulfide-linked) via a third linker peptide.

In one embodiment the first, second, and third linker peptide is the same or different.

In one embodiment the first linker peptide, the second linker peptide, and the third linker peptide are selected independently from each other from the amino acid sequences GS (SEQ ID NO: 73), GGS (SEQ ID NO: 74), GGGS (SEQ ID NO: 75), GGGSGGGS (SEQ ID NO: 76), GGGSGGGSGGGS (SEQ ID NO: 77), GGGSGGGSGGGSGGGS (SEQ ID NO: 78), GGGSGGGSGGGSGGGSGGGS (SEQ ID NO: 79), GGGGS (SEQ ID NO: 80), GGGGSGGGGS (SEQ ID NO: 81), GGGGSGGGGSGGGGS (SEQ ID NO: 82), GGGGSGGGGSGGGGSGGGGS (SEQ ID NO: 83), and GGGGSGGGGSGGGGSGGGGSGGGGS (SEQ ID NO: 84).

In one embodiment the first linker peptide comprises the amino acid sequence of SEQ ID NO: 82.

In one embodiment the second linker peptide comprises the amino acid sequence of SEQ ID NO: 83.

In one embodiment the third linker peptide comprises the amino acid sequence of SEQ ID NO: 73.

In one embodiment the antibody Fc-region is selected from an antibody Fc-region of a human antibody of the class IgG or the class IgE.

In one embodiment the antibody Fc-region is selected from an antibody Fc-region of a human antibody of the subclass IgG1, or IgG2, or IgG3, or IgG4.

In one embodiment the first disulfide-linked polypeptide and the second disulfide-linked polypeptide comprises a CH2 domain and a CH3 domain of human origin. In one embodiment the CH2 domain and the CH3 of human origin is of a human antibody of the class IgG or IgE. In one embodiment the CH2 domain and the CH3 domain is of a human antibody of the subclass IgG1, or IgG2, or IgG3, or IgG4. In one embodiment the CH2 domain comprises the amino acid sequence of SEQ ID NO: 85. In one embodiment the CH2 domain is of a human antibody of the subclass IgG1 or IgG2 and comprises at least one mutation of E233, L234, L235, G236, D265, D270, N297, E318, K320, K322, A327, P329, A330, and/or P331 (numbering according to the EU index of Kabat). In one embodiment the CH2 domain is of a human antibody of the subclass IgG1 or the human subclass IgG2 with the mutations L234A and L235A, and/or the mutations D265A and N297A, and/or contains the PVA236 mutation, and/or contains the mutation P329G. In one embodiment the CH2 domain is of a human antibody of the subclass IgG1 with the mutations L234A and L235A, and/or P329G. In one embodiment the CH2 domain is of a human antibody of the subclass IgG4 with the mutations S228P and/or L235E.

In one embodiment the first disulfide-linked polypeptide comprises the amino acid sequence of SEQ ID NO: 89 and the second disulfide-linked polypeptide comprises the amino acid sequence of SEQ ID NO: 90.

In one embodiment the first and the second disulfide-linked polypeptide comprise the amino acid sequence of SEQ ID NO: 94 or SEQ ID NO: 101.

In one embodiment the first disulfide-linked polypeptide or the second disulfide-linked polypeptide is consisting of the amino acid sequence of SEQ ID NO: 97 or SEQ ID NO: 98.

In one embodiment the first disulfide-linked polypeptide comprises the amino acid sequence of SEQ ID NO: 102 and the second disulfide-linked polypeptide comprises the amino acid sequence of SEQ ID NO: 103.

In one embodiment the disulfide-linked polypeptides are linked by two, or three, or four disulfide bonds.

In one embodiment the antigen presenting domain is characterized in that it comprises in N- to C-terminal direction
(i) a virus-derived peptide that has an amino acid sequence of SEQ ID NO: 01,
(ii) a first linker peptide that has an amino acid sequence of SEQ ID NO: 82.
(iii) a β2-microglobulin that has an amino acid sequence of SEQ ID NO: 71,
(iv) a second linker peptide that has an amino acid sequence of SEQ ID NO: 83,
(v) the extracellular domains α1, α2, and α3 of a class I MHC molecule that has an amino acid sequence of SEQ ID NO: 72, and
(vi) a third linker peptide that has an amino acid sequence of SEQ ID NO: 73.

From Figure 10 it can be seen that the multi-function protein as reported herein maintains the binding properties of the antibody to which it is fused (Figure 10 b) and c)).

In Figures 11 and 13 the in vitro efficacy and specificity of a multi-function protein as reported herein is shown.

The cytotoxicity assay was performed in the presence of CMV-specific CD8⁺ T-cells. It can be seen that a multi-function protein comprising a CMV-derived virus peptide induce the lysis/removal/disintegration of the target cells (see Figure 11a) for monovalent antibody, Figure 11b) for bivalent antibody). It can further be seen that the lysis of the target cells is highly specific as the incubation with the multi-function protein comprising an EBV-derived viral peptide (Figure 11b)) and control antibodies (Figure 11d) and e)) do not result in extensive cell lysis (the spontaneous lysis is about 3.5 %).

In Figure 13 the lysis of IGF-1R positive lung adenocarcinoma cell line H460M2 is shown.

The EC₅₀ value for a multi-function protein comprising a CMV-derived peptide and a bivalent antibody is about 10 ng/ml corresponding to about 50 pM. The determined EC₅₀ value is independent from the target cell to effector cell ratio (see Figure 12; target cell to effector cell ratio from 1:3 to 1:1 corresponding to an effective ratio of 1:0.44 to 1:0.14 (76 % of effector cells are CD8 positive and 19 % are CMV specific)).

### 1. Affinity

In certain embodiments, a multi-function protein as provided herein comprises an antigen binding site derived from an antibody, e.g. a pair of antibody variable domains or a single domain antibody. In certain embodiments the antigen binding site has a dissociation constant (Kd) of ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M) with respect to its antigen.

In one embodiment, Kd is measured using surface plasmon resonance assays.

For example this can be done by using a BIACORE®-2000 or a BIACORE®-3000 instrument (BIAcore, Inc., Piscataway, NJ) at 25 °C with immobilized antigen CM5 chips art ∼10 response units (RU). Briefly, carboxymethylated dextran biosensor chips (CM5, BIAcore, Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (-0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05 % polysorbate 20 (TWEEN-20™) surfactant (PBST) at 25 °C at a flow rate of approximately 25 µl/min. Association rates (kon) and dissociation rates (koff) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio koff/kon. See, e.g., Chen, Y., et al., J. Mol. Biol. 293 (1999) 865-881.

### 2. Expression

Expression of specific formats of the multi-function protein as reported herein (different linker, different combinations of HLA and β*2-microglobulin) in HEK 293 and CHO cells led to an accumulation of the multi-function protein, if detectable at all, within the endoplasmatic reticulum, i.e. isolation and secretion of the multi-function protein was strongly impaired.

No secretion of a multi-function protein to the cultivation medium could be detected when the multi-function protein was intended to comprise one of the polypeptides as outlined in the following tables.

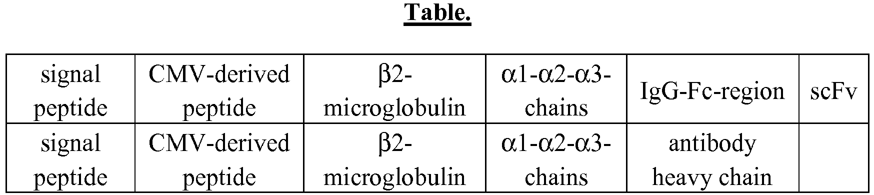

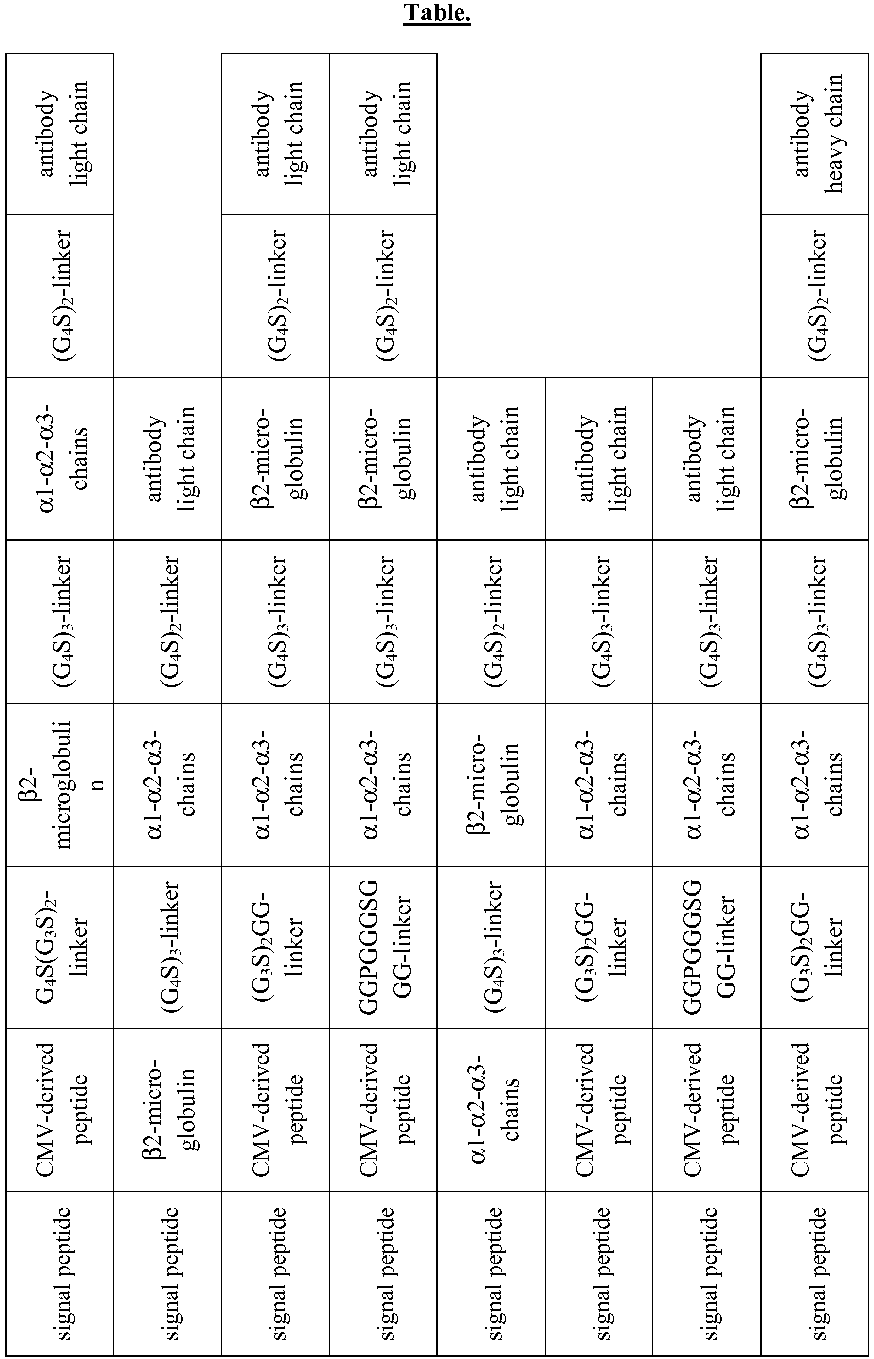

It has been found that the expression, and especially the secretion, of multi-function proteins comprising two antigen presenting domains formed of a virus-derived peptide linked to a MHC class I protein complex, and at least one variable domain and one constant domain of an antibody is not possible in eukaryotic cells.

Further it has been found that the expression, and especially the secretion, of multi-function proteins comprising two antigen presenting domains formed of a virus-derived peptide linked to a MHC class I protein multi-function protein, at least one variable domain, and an antibody hinge region is not possible in eukaryotic cells.

Thus, in a multi-function protein as reported herein an antigen presenting domain comprising a virus-derived peptide linked to a MHC class I protein cannot be present more than once and at least one antibody variable domain and one antibody constant domain has to be present in order to allow for the production and the secretion of the multi-function protein using eukaryotic cells.

Thus, a multi-function protein comprising exactly one antigen presenting domain of a virus-derived peptide linked to a MHC class I protein, an antibody heavy chain hinge region, and at least one antibody variable domain and one antibody constant domain can be recombinantly produced in and secreted from eukaryotic cells.

Thus, a multi-function protein comprising an antibody heavy chain hinge region, at least one pair of antibody variable domains, optionally an antibody constant domain, and exactly one antigen presenting domain of a virus-derived peptide linked to a MHC class I protein can be recombinantly produced in and secreted from eukaryotic cells.

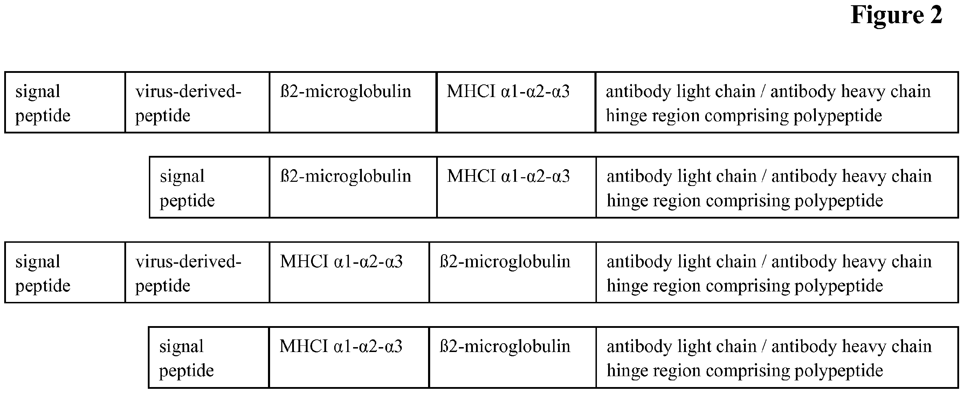

Various combinations were tested. Secreted expression of multi-function proteins can be accomplished by e.g. N-terminal fusion of an immunoglobulin-derived signal peptide wherein the virus-derived peptide is fused N-terminally to the class I MHC molecule. Class I MHC molecule heavy chain (α1-α2-α3 lacking the transmembrane and the cytoplasmatic domain) and light chain (β2-microglobulin) can be changed in order. The different antigen presenting domains were N-terminally fused to either an antibody light chain or an antibody heavy chain hinge region comprising polypeptide. Exemplary combinations are shown in Figure 2.

As can be seen from the following table multi-function proteins comprising antigen presenting domains containing an MHC-I protein complex can only be expressed in the presence of variable antibody domain and antibody hinge region derived polypeptides when a single viral-derived-peptide-microglobulin-HLA-fusion polypeptide is present.

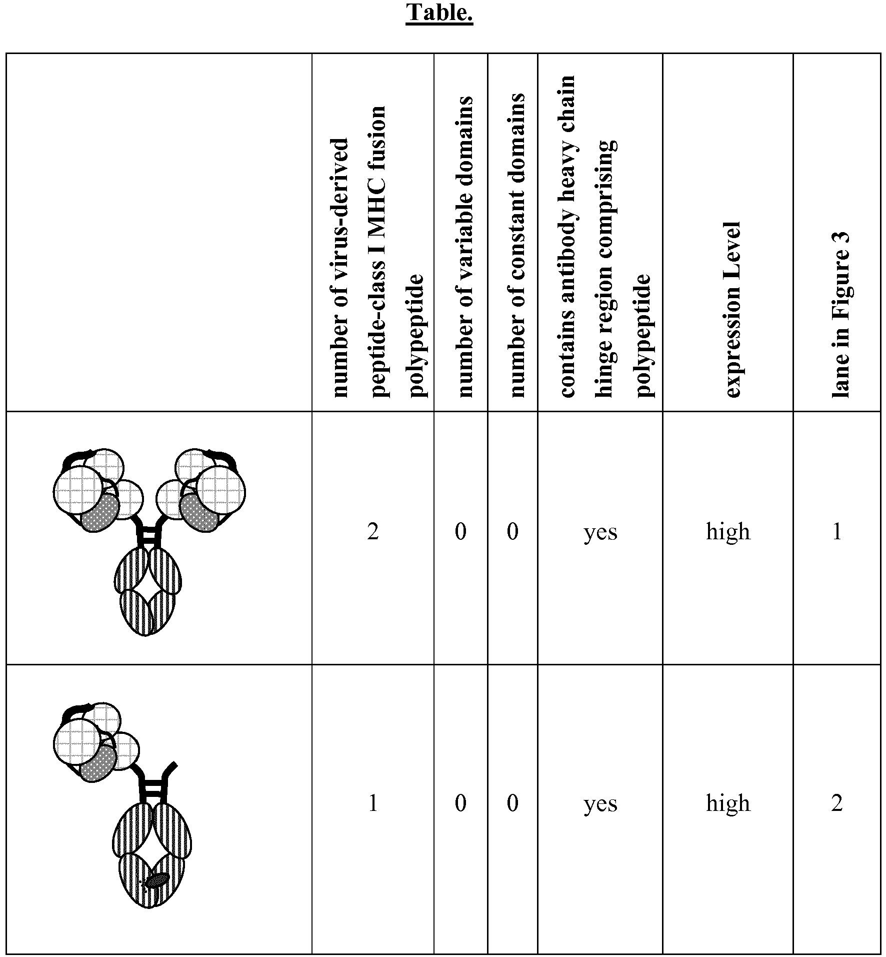

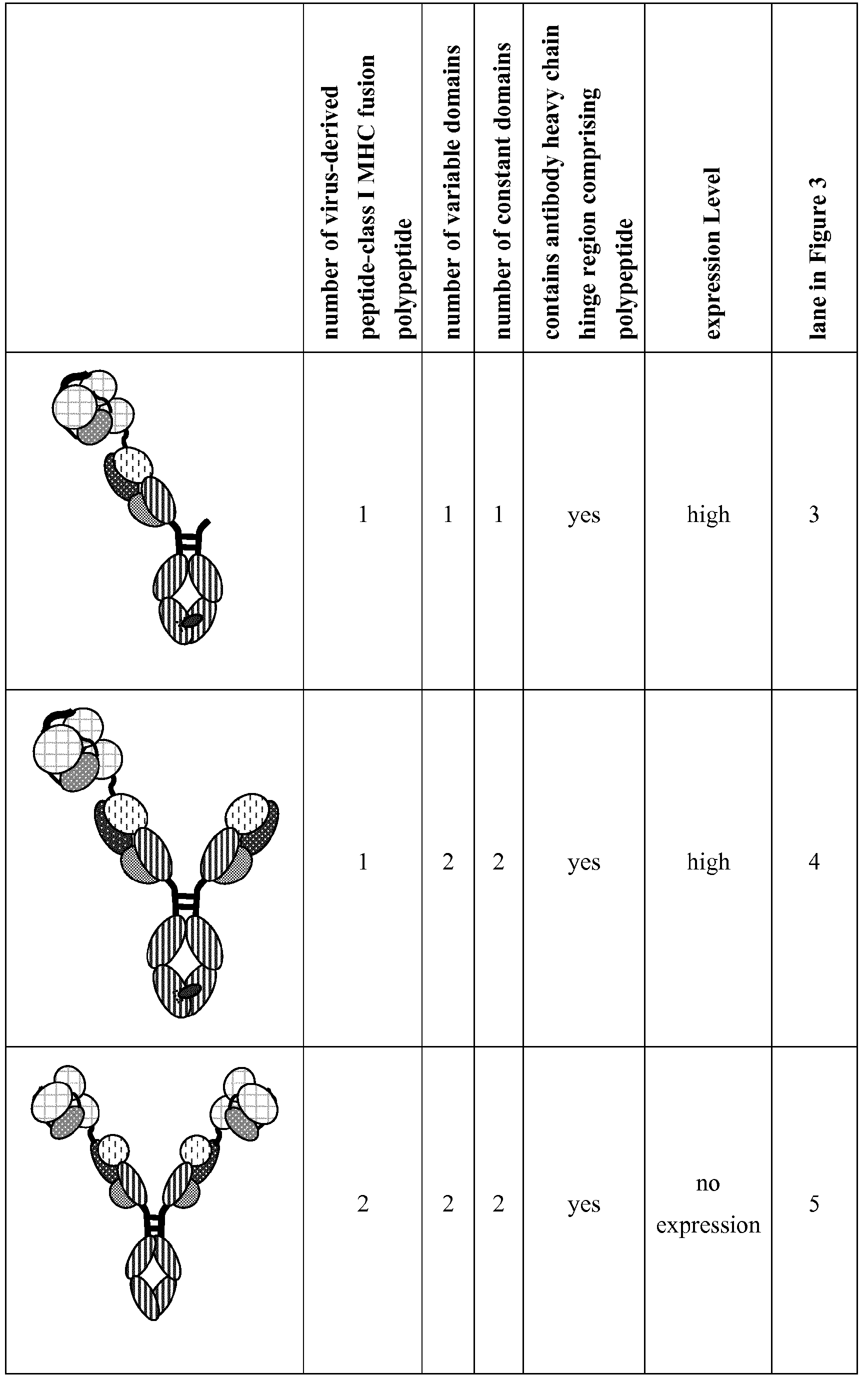

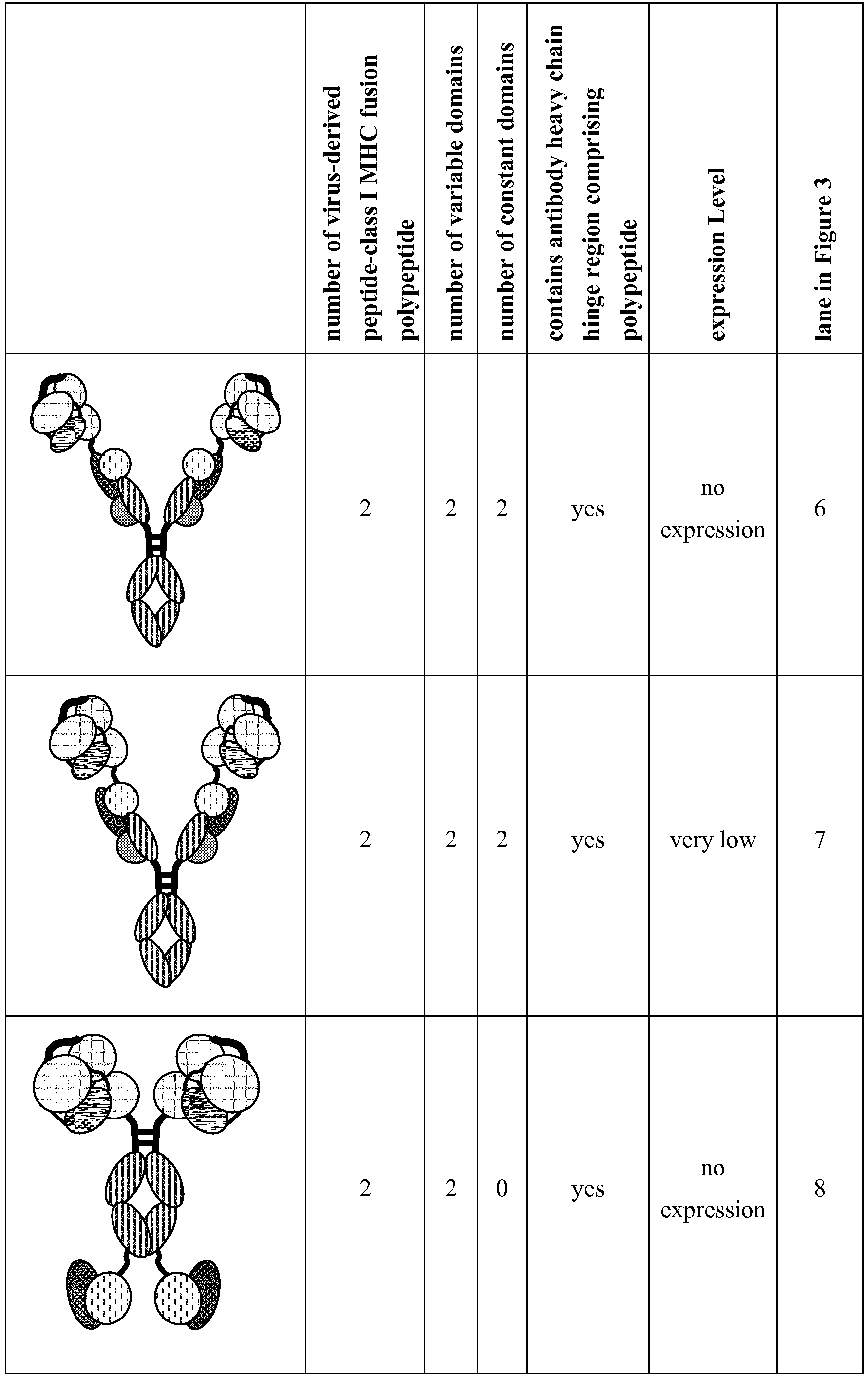

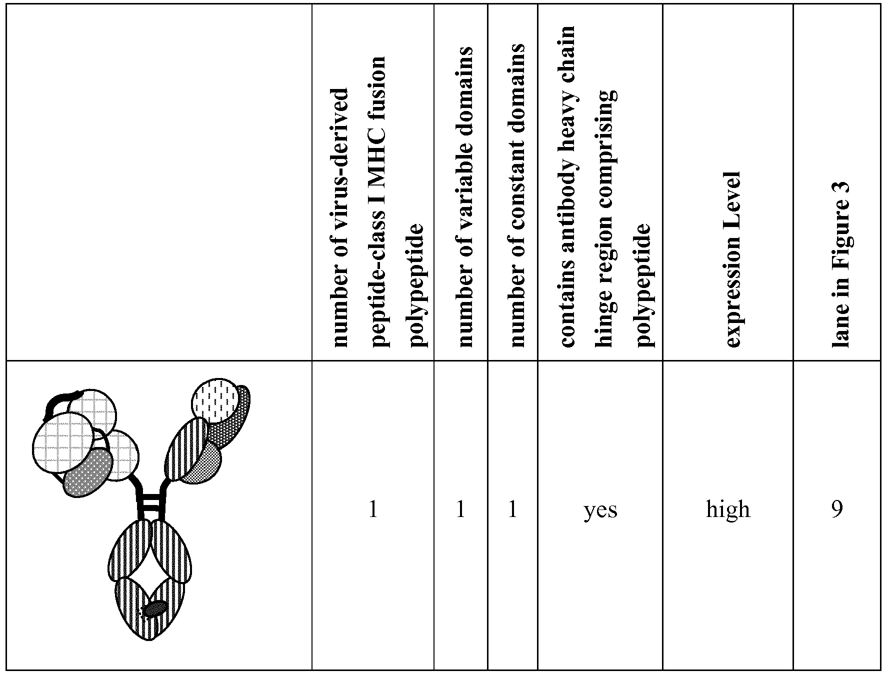

In some embodiments the multi-function protein as reported herein comprises different pairs of polypeptides. In order to allow proper pairing of the polypeptides the knobs-into-holes technology or the cross-mAb technology can be used in order to reduce the amount of not correctly associated multi-function protein.

The knob modification denotes the mutation T366W in the CH3 domain of an antibody (numbering according to EU index of Kabat).

The hole-modification denotes the mutations T366S, L368A and Y407V in the CH3 domain of an antibody (numbering according to EU index of Kabat).

In addition to the knob and hole modification the mutation S354C in the one CH3 domain and the mutation Y349C in the other CH3 domain can be present.

The cross-mAb technology is reported e.g. in WO 2009/080251, WO 2009/080252, WO 2009/080254, WO 2009/080253, WO 2010/112193, WO 2010/115589, WO 2010/136172, WO 2010/145792, and WO 2010/145793.

### 3. Variants

In certain embodiments, amino acid sequence variants of the multi-function protein provided herein are contemplated. For example, it may be desirable to improve the binding affinity and/or other biological properties of the multi-function protein. Amino acid sequence variants of the multi-function protein may be prepared by introducing appropriate modifications into the nucleotide sequence encoding the polypeptide chains of the multi-function protein, or by peptide synthesis. Such modifications include, for example, deletions from, and/or insertions into and/or substitutions of residues within the amino acid sequences of the polypeptides of the multi-function protein. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, multi-function protein variants having one or more amino acid substitutions in one or more of the polypeptide chains are provided. Exemplary changes are provided in the following table under the heading of "exemplary substitutions", and as further described below in reference to amino acid side chain classes. Conservative substitutions are shown in the following Table under the heading of "preferred substitutions". Amino acid substitutions may be introduced into a multi-function protein of interest and the products screened for a desired activity, e.g., retained/improved antigen binding, decreased immunogenicity, or improved ADCC or CDC.

**Table.**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a multi-function protein comprising a polypeptide with an N-terminal methionyl residue. Other insertional variants include the fusion to the N- or C-terminus of the polypeptide chains of the multi-function protein to an enzyme.

### b) Glycosylation variants

In certain embodiments, one or more polypeptides of the multi-function protein provided herein can be altered to increase or decrease the extent to which the polypeptide(s) is(are) glycosylated. Addition or deletion of glycosylation sites to a polypeptide may be conveniently accomplished by altering the amino acid sequence such that one or more glycosylation sites is created or removed.

The multi-function protein comprises an antibody Fc-region and the carbohydrate attached thereto may be altered. Native Fc-regions produced by mammalian cells typically comprise a branched, biantennary oligosaccharide that is generally attached by an N-linkage to Asn297 of the CH2 domain of the Fc-region. See, e.g., Wright, A., and Morrison, S.L., TIBTECH 15 (1997) 26-32. The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in a multi-function protein as reported herein may be made in order to create variants with certain improved properties.

In one embodiment, multi-function protein comprising polypeptide variants are provided having a carbohydrate structure that lacks fucose attached (directly or indirectly) to the Fc-region. For example, the amount of fucose in such Fc-region may be from 1 % to 80 %, from 1 % to 65 %, from 5 % to 65 % or from 20 % to 40 %. The amount of fucose is determined by calculating the average amount of fucose within the sugar chain at Asn297, relative to the sum of all glycostructures attached to Asn 297 (e.g. multi-function protein, hybrid and high mannose structures) as measured by MALDI-TOF mass spectrometry, as described in WO 2008/077546, for example. Asn297 refers to the asparagine residue located at about position 297 in the Fc-region (EU numbering of Fc-region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US 2003/0157108; US 2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki, A., et al., J. Mol. Biol. 336 (2004) 1239-1249; Yamane-Ohnuki, N., et al., Biotech. Bioeng. 87 (2004) 614-622. Examples of cell lines capable of producing defucosylated antibodies include Lec13 CHO cells deficient in protein fucosylation (Ripka, J., et al., Arch. Biochem. Biophys. 249 (1986) 533-545; US 2003/0157108; and WO 2004/056312, especially at Example 11), and knockout cell lines, such as alpha-1,6-fucosyltransferase gene, FUT8, knockout CHO cells (see, e.g., Yamane-Ohnuki, N., et al., Biotech. Bioeng. 87 (2004) 614-622; Kanda, Y., et al., Biotechnol. Bioeng. 94 (2006) 680-688; and WO 2003/085107).

Multi-function proteins comprising Fc-region variants are further provided with bisected oligosaccharides, e.g., in which a biantennary oligosaccharide attached to the Fc-region is bisected by GlcNAc. Such variants may have reduced fucosylation and/or improved ADCC function. Examples of such antibody variants are described, e.g., in WO 2003/011878; US 6,602,684; and US 2005/0123546. Fc-region variants with at least one galactose residue in the oligosaccharide attached to the Fc-region are also provided. Such Fc-region variants may have improved CDC function. Corresponding antibody variants are described, e.g., in WO 97/30087; WO 98/58964; and WO 99/22764.

### c) Fc-region variants

In certain embodiments, one or more amino acid modifications may be introduced into the Fc-region of the multi-function protein provided herein, thereby generating an Fc-region variant. The Fc-region variant may comprise a human Fc-region sequence (e.g., a human IgG1, IgG2, IgG3 or IgG4 Fc-region) comprising an amino acid modification (e.g. a substitution) at one or more amino acid positions.

In certain embodiments, the invention contemplates an Fc-region variant that possesses some but not all effector functions, which make it a desirable candidate for applications in which the half-life of the multi-function protein in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the multi-function protein lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch, J.V., and Kinet, J.P., Annu. Rev. Immunol. 9 (1991) 457-492. Nonlimiting examples of in vitro assays to assess ADCC activity of a molecule of interest is described in US 5,500,362 (see, e.g. Hellstrom, I., et al., Proc. Natl. Acad. Sci. USA 83 (1986) 7059-7063; and Hellstrom, I., et al., Proc. Natl. Acad. Sci. USA 82 (1985) 1499-1502); US 5,821,337 (see Bruggemann, M., et al., J. Exp. Med. 166 (1987) 1351-1361). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes, R., et al., Proc. Natl. Acad. Sci. USA 95 (1998) 652-656. C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro, H., et al., J. Immunol. Methods 202 (1996) 163-171; Cragg, M.S., et al., Blood 101 (2003) 1045-1052; and Cragg, M.S. and Glennie, M.J., Blood 103 (2004) 2738-2743). FcRn binding and in vivo clearance/half-life determinations can also be performed using methods known in the art (see, e.g., Petkova, S.B., et al., Int. Immunol. 18 (2006) 1759-1769).

Fc-regions with reduced effector function include those with substitution of one or more of Fc-region residues 234, 235, 238, 265, 269, 270, 297, 327 and 329 (see e.g. US 6,737,056). Such Fc-region mutants include Fc-region mutants with substitutions at two or more of amino acid positions 265, 269, 270, 297 and 327, including the so-called "DANA" Fc-region mutant with substitution of residues 265 and 297 to alanine (US 7,332,581).

Certain Fc-region variants with improved or diminished binding to FcRs are described. (See, e.g., US 6,737,056; WO 2004/056312, and Shields, R.L., et al., J. Biol. Chem. 276 (2001) 6591-6604).

In certain embodiments, a multi-function protein variant comprises an Fc-region with one or more amino acid substitutions which improve ADCC, e.g., substitutions at positions 298, 333, and/or 334 of the Fc-region (EU numbering of residues).

In some embodiments, alterations are made in the Fc-region that result in altered (i.e., either improved or diminished) C1q binding and/or Complement Dependent Cytotoxicity (CDC), e.g., as described in US 6,194,551, WO 99/51642, and Idusogie, E.E., et al., J. Immunol. 164 (2000) 4178-4184.

Antibodies with increased half-lives and improved binding to the neonatal Fc receptor (FcRn), which is responsible for the transfer of maternal IgGs to the fetus (Guyer, R.L., et al., J. Immunol. 117 (1976) 587-593, and Kim, J.K., et al., J. Immunol. 24 (1994) 2429-2434), are described in US 2005/0014934. Those antibodies comprise an Fc-region with one or more substitutions therein which improve binding of the Fc-region to FcRn. Such Fc-region variants include those with substitutions at one or more of Fc-region residues: 238, 256, 265, 272, 286, 303, 305, 307, 311, 312, 317, 340, 356, 360, 362, 376, 378, 380, 382, 413, 424 or 434, e.g., substitution of Fc-region residue 434 (US 7,371,826).

See also Duncan, A.R. and Winter, G., Nature 322 (1988) 738-740; US 5,648,260; US 5,624,821; and WO 94/29351 concerning other examples of Fc-region variants.

### B. Recombinant Methods and Compositions

Multi-function proteins as reported herein may be produced using recombinant methods and compositions, e.g., as described in US 4,816,567. In one embodiment, isolated nucleic acids encoding the polypeptides of the multi-function protein described herein are provided. In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0 cell). In one embodiment, a method of making a multi-function protein as reported herein is provided, wherein the method comprises culturing a host cell comprising a nucleic acid encoding the polypeptides of the multi-function protein, as provided above, under conditions suitable for expression of the polypeptides and formation of the multi-function protein, and optionally recovering the multi-function protein from the host cell (or host cell culture medium).

For recombinant production of a multi-function protein, nucleic acid encoding the polypeptides of the multi-function protein, e.g., as described above, are isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression vectors include prokaryotic or eukaryotic cells described herein. For example, multi-function proteins may be produced in bacteria, in particular when glycosylation and Fc effector function are not needed. For expression of antibody fragments and polypeptides in bacteria, see, e.g., US 5,648,237, US 5,789,199, and US 5,840,523. (See also Charlton, K.A., In: Methods in Molecular Biology, Vol. 248, Lo, B.K.C. (ed.), Humana Press, Totowa, NJ (2003), pp. 245-254, describing expression of antibody fragments in *E. coli*.) After expression, the multi-function protein may be isolated from the bacterial cell paste in a soluble fraction and can be further purified.

In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of an antibody with a partially or fully human glycosylation pattern. See Gerngross, T.U., Nat. Biotech. 22 (2004) 1409-1414; and Li, H., et al., Nat. Biotech. 24 (2006) 210-215.

Suitable host cells for the expression of glycosylated multi-function proteins are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US 5,959,177, US 6,040,498, US 6,420,548, US 7,125,978, and US 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (HEK 293 or 293 cells as described, e.g., in Graham, F.L., et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P., et al., Annals N.Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G., et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### C. Pharmaceutical Formulations

Pharmaceutical formulations of a multi-function protein as described herein are prepared by mixing such multi-function protein having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX^{®}, Baxter International, Inc.). Certain exemplary sHASEGPs and methods of use, including rhuPH20, are described in US 2005/0260186 and US 2006/0104968. In one aspect, a sHASEGP is combined with one or more additional glycosaminoglycanases such as chondroitinases.

Exemplary lyophilized antibody formulations are described in US 6,267,958. Aqueous antibody formulations include those described in US 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, e.g. films, or microcapsules.

The formulations to be used for in vivo administration are generally sterile. Sterility may be readily accomplished, e.g., by filtration through sterile filtration membranes.

### D. Therapeutic Methods and Compositions

Any of the multi-function proteins provided herein may be used in therapeutic methods.

In one aspect, a multi-function protein as reported herein for use as a medicament is provided.

In further aspects, a multi-function protein as reported herein for use in treating cancer is provided.

In certain embodiments, a multi-function protein as reported herein for use in a method of treatment is provided.

In certain embodiments, the invention provides a multi-function protein as reported herein for use in a method of treating an individual having cancer comprising administering to the individual an effective amount of the multi-function protein as reported herein. In one such embodiment, the method further comprises administering to the individual an effective amount of at least one additional therapeutic agent, e.g., as described below.

In further embodiments, the invention provides a multi-function protein as reported herein for use in removal of cancer cells. In certain embodiments, the invention provides a multi-function protein as reported herein for use in a method of removal of cancer cells in an individual comprising administering to the individual an effective of the multi-function protein as reported herein to remove cancer cells. An "individual" according to any of the above embodiments may be a human.

Also reported herein is the use of a multi-function protein as reported herein in the manufacture or preparation of a medicament. In one embodiment, the medicament is for treatment of cancer. In a further embodiment, the medicament is for use in a method of treating cancer comprising administering to an individual having cancer an effective amount of the medicament. In one such embodiment, the method further comprises administering to the individual an effective amount of at least one additional therapeutic agent. In a further embodiment, the medicament is for the removal of cancer cells. In a further embodiment, the medicament is for use in a method of removal of cancer cells in an individual comprising administering to the individual an amount effective of the medicament to remove cancer cells. An "individual" according to any of the above embodiments may be a human.

Also reported herein is a method for treating cancer. In one embodiment, the method comprises administering to an individual having such cancer an effective amount of a multi-function protein as reported herein. In one such embodiment, the method further comprises administering to the individual an effective amount of at least one additional therapeutic agent. An "individual" according to any of the above embodiments may be a human.

Also reported herein isa method for removal of cancer cells in an individual. In one embodiment, the method comprises administering to the individual an effective amount of a multi-function protein as reported herein to remove cancer cells. In one embodiment, an "individual" is a human.

In a further aspect, the invention provides pharmaceutical formulations comprising any of the multi-function proteins as reported herein, e.g., for use in any of the above therapeutic methods. In one embodiment, a pharmaceutical formulation comprises any of the multi-function proteins as reported herein and a pharmaceutically acceptable carrier. In another embodiment, a pharmaceutical formulation comprises any of the multi-function proteins as reported herein and at least one additional therapeutic agent.

Multi-function proteins of the invention can be used either alone or in combination with other agents in a therapy. For instance, a multi-function protein of the invention may be co-administered with at least one additional therapeutic agent.

Such combination therapies noted above encompass combined administration (where two or more therapeutic agents are included in the same or separate formulations), and separate administration, in which case, administration of the multi-function protein of the invention can occur prior to, simultaneously, and/or following, administration of the additional therapeutic agent and/or adjuvant. Multi-function proteins of the invention can also be used in combination with radiation therapy.

A multi-function protein of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Multi-function proteins of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The multi-function protein need not be, but is optionally formulated with one or more agents currently used to prevent or treat the disorder in question. The effective amount of such other agents depends on the amount of multi-function protein present in the formulation, the type of disorder or treatment, and other factors discussed above. These are generally used in the same dosages and with administration routes as described herein, or about from 1 to 99 % of the dosages described herein, or in any dosage and by any route that is empirically/clinically determined to be appropriate.

For the prevention or treatment of disease, the appropriate dosage of a multi-function protein of the invention (when used alone or in combination with one or more other additional therapeutic agents) will depend on the type of disease to be treated, the type of multi-function protein, the severity and course of the disease, whether the multi-function protein is administered for preventive or therapeutic purposes, previous therapy, the patient's clinical history and response to the multi-function protein, and the discretion of the attending physician. The multi-function protein is suitably administered to the patient at one time or over a series of treatments. Depending on the type and severity of the disease, about 1 µg/kg to 15 mg/kg (e.g. 0.5 mg/kg - 10 mg/kg) of multi-function protein can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to a multi-function protein as reported herein.

One aspect as reported herein is the multi-function protein as reported herein for use in a method of treating a cancer in a patient, wherein the multi-function protein is to be administered before, simultaneously or after the immunization of the patient with the virus-derived peptide comprised in the multi-function protein.

One aspect as reported herein is the use of a multi-function protein as reported herein for the manufacture of a medicament for the treatment of cancer in combination with immunization against the virus-derived peptide comprised in the multi-function protein.

In the first step the virus-derived peptide as contained in the multi-function protein is administered first to the individual to be treated. At a certain time span later, i.e. between 4 days and 28 days, the multi-function protein as reported herein is administered to the individual.

By this successive and separated application of the virus-derived polypeptide, in the first step alone and in the second step in the multi-function protein as reported herein, it is possible to increase the number of virus-derived peptide specific T-cell and, thus, to increase the efficacy of the treatment.

### III. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a multi-function protein of the invention. The label or package insert indicates that the composition is used for treating the condition of choice. Moreover, the article of manufacture may comprise (a) a first container with a composition contained therein, wherein the composition comprises a multi-function protein of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to a multi-function protein as reported herein.

### IV. SPECIFIC EMBODIMENTS

1. A multi-function protein, characterized in that it comprises
   - exactly one antigen presenting domain,
   - exactly one antibody Fc-region, and
   - at least one antigen binding site,
   wherein the antigen presenting domain comprises in N- to C-terminal direction
   either
      (i) a T-cell response eliciting peptide,
      (ii) a β2-microglobulin, and
      (iii) the extracellular domains α1, α2, and α3 of a class I MHC molecule selected from the group comprising HLA-A*0201, HLA-A*1101, HLA-A*2402, HLA-A*340101, HLA-C*0304, HLA-C*0401, and HLA-C*0702,
      or
      (i) a T-cell response eliciting peptide,
      (ii) the extracellular domains α1, α2, and α3 of a class I MHC molecule selected from the group comprising HLA-A*0201, HLA-A*1101, HLA-A*2402, HLA-A*340101, HLA-C*0304, HLA-C*0401, and HLA-C*0702, and
      (iii) a β2-microglobulin,
   wherein the antigen binding site binds to melanoma-associated chondroitin sulfate proteoglycan (MCSP).
2. The multi-function protein according to item 1, characterized in that the antibody Fc-region comprises a first and second disulfide-linked Fc-region polypeptide, whereby the antigen binding site comprises the first Fc-region polypeptide.
3. The multi-function protein according to any one of items 1 to 2, characterized in that the antigen binding site comprises i) a pair of an antibody heavy chain and an antibody light chain, or ii) a scFv fusion polypeptide comprising in N-to C-terminal direction a scFv antibody fragment and an antibody Fc-region polypeptide, or iii) a scFab fusion polypeptide comprising in N- to C-terminal direction a scFab and an antibody Fc-region polypeptide.
4. The multi-function protein according to any one of items 1 to 3, characterized in that i) the antigen presenting domain is linked to the N-terminus of the heavy chain or to the N-terminus of the light chain of the antigen binding site, or ii) the antigen presenting domain is linked to the C-terminus of the heavy chain or to the C-terminus of the light chain of the antigen binding site, or iii) the antigen presenting domain is linked to the N- or C-terminus of the scFv fusion polypeptide, or iv) the antigen presenting domain is linked to the N- or C-terminus of the scFab fusion polypeptide, or iv) the antigen presenting domain is linked to the N- or C-terminus of the second Fc-region polypeptide.
5. The multi-function protein according to any one of items 1 to 4, characterized in that the cancer cell surface antigen is melanoma-associated chondroitin sulfate proteoglycan (MCSP).
6. The multi-function protein according to any one of items 1 to 5, characterized in that the T-cell response eliciting peptide is a virus-derived peptide.
7. The multi-function protein according to any one of items 1 to 6, characterized in that the T-cell response eliciting peptide is a CD8⁺-T-cell response eliciting peptide.
8. The multi-function protein according to any one of items 6 to 7, characterized in that the virus-derived peptide is a human cytomegalovirus-derived peptide.
9. The multi-function protein according to any one of items 1 to 8, characterized in that the virus-derived peptide has an amino acid sequence selected from the group of SEQ ID NO: 01 to SEQ ID NO: 70.
10. The multi-function protein according to any one of items 1 to 9, characterized in that the virus-derived peptide has the amino acid sequence of SEQ ID NO: 01.
11. The multi-function protein according to any one of items 1 to 10, characterized in that the antigen presenting domain comprises
   (i) a virus-derived peptide,
   (ii) β2-microglobulin, and
   (iii) the soluble HLA-A allele A*0201
   or
   (i) a virus-derived peptide,
   (ii) the soluble HLA-A allele A*0201, and
   (iii) β2-microglobulin.
12. The multi-function protein according to any one of items 1 to 11, characterized in that the β2-microglobulin is consisting of the amino acid sequence of SEQ ID NO: 71 or is a variant thereof comprising of from 1 to 10 amino acid exchanges, additions, and/or deletions.
13. The multi-function protein according to any one of items 1 to 12, characterized in that the extracellular domains α1, α2 and α3 of a class I MHC molecule is consisting of the amino acid sequence of SEQ ID NO: 72 or is a variant thereof comprising of from 1 to 10 amino acid exchanges, additions, and/or deletions.
14. The multi-function protein according to any one of items 1 to 13, characterized in that the antigen presenting domain comprises in N- to C-terminal direction
   (i) a virus-derived peptide that has an amino acid sequence selected from the group comprising SEQ ID NO: 01 to SEQ ID NO: 70,
   (ii) a first linker peptide that has an amino acid sequence selected from the group comprising SEQ ID NO: 77, 78, 79, 82, 83, and 84,
   (iii) a β2-microglobulin that has an amino acid sequence of SEQ ID NO: 71,
   (iv) a second linker peptide that has an amino acid sequence selected from the group comprising SEQ ID NO: 77, 78, 79, 82, 83, and 84,
   (v) the extracellular domains α1, α2, and α3 of a class I MHC molecule that has an amino acid sequence of SEQ ID NO: 72, and
   (vi) a third linker peptide that has an amino acid sequence selected from the group comprising SEQ ID NO: 73, 77, 78, 79, 82, 83, 84, and 136.
15. The multi-function protein according to any one of items 1 to 14, characterized in that the multi-function protein is characterized in that the antigen presenting domain comprises in N- to C-terminal direction
   (i) a virus-derived peptide that has an amino acid sequence selected from the group comprising SEQ ID NO: 01 to SEQ ID NO: 70,
   (ii) a first linker peptide that has an amino acid sequence selected from the group comprising SEQ ID NO: 77, 78, 79, 82, 83, and 84,
   (iii) a β2-microglobulin that has an amino acid sequence of SEQ ID NO: 71 or is a variant thereof comprising of from 1 to 10 amino acid exchanges, additions, and/or deletions,
   (iv) a second linker peptide that has an amino acid sequence selected from the group comprising SEQ ID NO: 77, 78, 79, 82, 83, and 84,
   (v) the extracellular domains α1, α2 and α3 of a class I MHC molecule that has an amino acid sequence of SEQ ID NO: 72 or is a variant thereof comprising of from 1 to 10 amino acid exchanges, additions, and/or deletions, and
   (vi) a third linker peptide that has an amino acid sequence selected from the group comprising SEQ ID NO: 73, 77, 78, 79, 82, 83, 84, 136.
16. The multi-function protein according to item 15, characterized in that
   - the first linker peptide has the amino acid sequence of SEQ ID NO: 82, and/or
   - the second linker peptide has the amino acid sequence of SEQ ID NO: 83, and/or
   - the third linker peptide has the amino acid sequence of SEQ ID NO: 136.
17. The multi-function protein according to any one of items 1 to 16, characterized in that the antibody Fc-region is selected from an antibody Fc-region of a human antibody of the class IgG or the class IgE.
18. The multi-function protein according to any one of items 1 to 17, characterized in that the antibody Fc-region is selected from an antibody Fc-region of a human antibody of the subclass IgG1, or IgG2, or IgG3, or IgG4.
19. The multi-function protein according to any one of items 1 to 18, characterized in that the antibody Fc-region is of a human antibody of the subclass IgG1 or IgG2 and comprises at least one mutation in E233, L234, L235, G236, D265, D270, N297, E318, K320, K322, A327, P329, A330, and/or P331 (numbering according to the EU index of Kabat).
20. The multi-function protein according to any one of items 1 to 19, characterized in that the antibody Fc-region is of a human antibody of the subclass IgG1 or the human subclass IgG2 with the mutations L234A and L235A, and/or the mutations D265A and N297A, and/or contains the PVA236 mutation, and/or contains the mutation P329G.
21. The multi-function protein according to any one of items 1 to 20, characterized in that the antibody Fc-region is of a human antibody of the subclass IgG1 with the mutations L234A and L235A and/or P329G.
22. The multi-function protein according to any one of items 1 to 20, characterized in that the antibody Fc-region is of a human antibody of the subclass IgG4 with the mutation S228P and/or L235E.
23. The multi-function protein according to any one of items 1 to 20, characterized in that the first and second antibody Fc-region polypeptide is selected independently of each other from the group comprising SEQ ID NO: 87 to 103.
24. The multi-function protein according to any one of items 1 to 20, characterized in that the antibody Fc-region comprises two Fc-region polypeptides with the amino acid sequence of SEQ ID NO: 94.
25. The multi-function protein according to any one of items 1 to 20, characterized in that the antibody Fc-region comprises two Fc-region polypeptides with the amino acid sequence of SEQ ID NO: 100.
26. The multi-function protein according to any one of items 1 to 20, characterized in that the antibody Fc-region comprises two Fc-region polypeptides with the amino acid sequence of SEQ ID NO: 101.
27. The multi-function protein according to any one of items 1 to 20, characterized in that the antibody Fc-region comprises a first Fc-region polypeptide with the amino acid sequence of SEQ ID NO: 89 and a second Fc-region polypeptide with the amino acid sequence of SEQ ID NO: 90.
28. The multi-function protein according to any one of items 1 to 20, characterized in that the antibody Fc-region comprises a first Fc-region polypeptide with the amino acid sequence of SEQ ID NO: 97 and a second Fc-region polypeptide with the amino acid sequence of SEQ ID NO: 98.
29. The multi-function protein according to any one of items 1 to 20, characterized in that the antibody Fc-region comprises a first Fc-region polypeptide with the amino acid sequence of SEQ ID NO: 102 and a second Fc-region polypeptide with the amino acid sequence of SEQ ID NO: 103.
30. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, and
   - at least one antigen binding site, which specifically binds to melanoma-associated chondroitin sulfate proteoglycan (MCSP).
31. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
   - at least one antigen binding site, which specifically binds to melanoma-associated chondroitin sulfate proteoglycan (MCSP).
32. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
   - at least one antigen binding site, which specifically binds to melanoma-associated chondroitin sulfate proteoglycan (MCSP).
33. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
   - at least one antigen binding site, which comprises an antibody light chain variable domain comprising an amino acid sequence from the group of SEQ ID NO: 104 to 106 and an antibody heavy chain variable domain comprising an amino acid sequence from the group of SEQ ID NO: 108 to 110, which specifically binds to melanoma-associated chondroitin sulfate proteoglycan (MCSP).
34. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
   - at least one antigen binding site, which specifically binds to melanoma-associated chondroitin sulfate proteoglycan (MCSP), and which comprises an antibody light chain with a variable domain comprising an amino acid sequence from the group of SEQ ID NO: 104 to 106 and an antibody heavy chain with a variable domain comprising an amino acid sequence from the group of SEQ ID NO: 108 to 110.
35. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
   - two antigen binding sites, which specifically bind to melanoma-associated chondroitin sulfate proteoglycan (MCSP), and which each comprise an antibody light chain with a variable domain comprising an amino acid sequence from the group of SEQ ID NO: 105 to 107 and an antibody heavy chain with a variable domain comprising an amino acid sequence from the group of SEQ ID NO: 110 to 112.
36. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
   - at least one antigen binding site, which specifically binds to melanoma-associated chondroitin sulfate proteoglycan (MCSP), and which comprises an antibody light chain with a variable domain comprising an amino acid sequence from the group of SEQ ID NO: 105 to 107 and an antibody heavy chain with a variable domain comprising an amino acid sequence from the group of SEQ ID NO: 110 to 112, whereby the Fc-region of the antibody heavy chain is one of the disulfide-linked Fc-region polypeptides.
37. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
   - at least one antigen binding site, which specifically binds to melanoma-associated chondroitin sulfate proteoglycan (MCSP), and which comprises an antibody light chain with a variable domain comprising an amino acid sequence from the group of SEQ ID NO: 104 to 106 and an antibody heavy chain with a variable domain comprising an amino acid sequence from the group of SEQ ID NO: 108 to 110, whereby the Fc-region of the antibody heavy chain is one of the disulfide-linked Fc-region polypeptides,
   wherein the antigen presenting domain is linked to the N-terminus of one of the variable domains.
38. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
   - two antigen binding site, which specifically bind to melanoma-associated chondroitin sulfate proteoglycan (MCSP), and which each comprise an antibody light chain with a variable domain comprising an amino acid sequence from the group of SEQ ID NO: 104 to 106 and an antibody heavy chain with a variable domain comprising an amino acid sequence from the group of SEQ ID NO: 108 to 110, whereby the Fc-regions of the antibody heavy chains are the disulfide-linked Fc-region polypeptides,
   wherein the antigen presenting domain is linked to the N-terminus of one of the variable domains.
39. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 97 and the second disulfide-linked Fc-region polypeptide has the amino acid sequence of SEQ ID NO: 98, and
   - two antigen binding site, which specifically bind to melanoma-associated chondroitin sulfate proteoglycan (MCSP), and which each comprise an antibody light chain with a variable domain comprising an amino acid sequence from the group of SEQ ID NO: 105 to 107 and an antibody heavy chain with a variable domain comprising an amino acid sequence from the group of SEQ ID NO: 110 to 112, whereby the Fc-regions of the antibody heavy chains are the disulfide-linked Fc-region polypeptides,
   wherein the antigen presenting domain is linked to the N-terminus of one of the heavy chain variable domains.
40. A multi-function protein, characterized in that it comprises
   - exactly one antigen presenting domain, which comprises in N- to C-terminal direction
      (i) a T-cell response eliciting peptide of SEQ ID NO: 01,
      (ii) a β2-microglobulin of SEQ ID NO: 71, and
      (iii) the extracellular domains α1, α2, and α3 of a class I MHC molecule of SEQ ID NO: 72,
   - exactly one antibody Fc-region, which comprises two disulfide-linked Fc-region polypeptides, whereof the first disulfide-linked Fc-region polypeptide has the amino acid sequence of SEQ ID NO: 102 and the second disulfide-linked Fc-region polypeptide has the amino acid sequence of SEQ ID NO: 103, and
   - two antigen binding site, which specifically bind to melanoma-associated chondroitin sulfate proteoglycan (MCSP), and which each comprise an antibody light chain with a variable domain comprising an amino acid sequence from the group of SEQ ID NO: 105 to 107 and an antibody heavy chain with a variable domain comprising an amino acid sequence from the group of SEQ ID NO: 110 to 112, whereby the Fc-regions of the antibody heavy chains are the disulfide-linked Fc-region polypeptides,
   wherein the antigen presenting domain is linked to the N-terminus of one of the heavy chain variable domains.
41. A multi-function protein, characterized in that it comprises
   - one polypeptide chain of SEQ ID NO: 117 or 137,
   - one polypeptide chain of SEQ ID NO: 118,
   - two polypeptide chains each of SEQ ID NO: 119.
42. The multi-function protein according to any one of items 1 to 41, characterized in that the MCSP binding site comprises an antibody heavy chain variable domain comprising an HVR-H1 comprising the amino acid sequence of SEQ ID NO: 108, an HVR-H2 comprising the amino acid sequence of SEQ ID NO: 109, and an HVR-H3 comprising the amino acid sequence of SEQ ID NO: 110.
43. The multi-function protein according to any one of items 1 to 41, characterized in that the MCSP binding site comprises an antibody light chain variable domain comprising an HVR-L1 comprising the amino acid sequence of SEQ ID NO: 104; an HVR-L2 comprising the amino acid sequence of SEQ ID NO: 105; and an HVR-L3 comprising the amino acid sequence of SEQ ID NO: 106.
44. The multi-function protein according to any one of items 1 to 41, characterized in that the MCSP binding site comprises an antibody heavy chain variable domain comprising an HVR-H1 comprising the amino acid sequence of SEQ ID NO: 108; an HVR-H2 comprising the amino acid sequence of SEQ ID NO: 109; an HVR-H3 comprising the amino acid sequence of SEQ ID NO: 110; and an antibody light chain variable domain comprising an HVR-L1 comprising the amino acid sequence of SEQ ID NO: 104; an HVR-L2 comprising the amino acid sequence of SEQ ID NO: 105; and an HVR-L3 comprising the amino acid sequence of SEQ ID NO: 106.
45. The multi-function protein according to any one of items 1 to 41, characterized in that the MCSP binding site comprises an antibody heavy chain variable domain comprising an HVR-H1 comprising the amino acid sequence of SEQ ID NO: 111, an HVR-H2 comprising the amino acid sequence of SEQ ID NO: 112, and an HVR-H3 comprising the amino acid sequence of SEQ ID NO: 110.
46. The multi-function protein according to any one of items 1 to 41, characterized in that the MCSP binding site comprises an antibody light chain variable domain comprising an HVR-L1 comprising the amino acid sequence of SEQ ID NO: 107; an HVR-L2 comprising the amino acid sequence of SEQ ID NO: 105; and an HVR-L3 comprising the amino acid sequence of SEQ ID NO: 106.
47. The multi-function protein according to any one of items 1 to 41, characterized in that the MCSP binding site comprises an antibody heavy chain variable domain comprising an HVR-H1 comprising the amino acid sequence of SEQ ID NO: 111; an HVR-H2 comprising the amino acid sequence of SEQ ID NO: 112; an HVR-H3 comprising the amino acid sequence of SEQ ID NO: 110; and an antibody light chain variable domain comprising an HVR-L1 comprising the amino acid sequence of SEQ ID NO: 107; an HVR-L2 comprising the amino acid sequence of SEQ ID NO: 105; and an HVR-L3 comprising the amino acid sequence of SEQ ID NO: 106.
48. The multi-function protein according to any one of items 1 to 41, characterized in that the MCSP binding site comprises an antibody heavy chain variable domain having at least 95% sequence identity to the amino acid sequence of SEQ ID NO: 114; and an antibody light chain variable domain having at least 95% sequence identity to the amino acid sequence of SEQ ID NO: 113.
49. The multi-function protein according to any one of items 1 to 41, characterized in that the MCSP binding site comprises an antibody heavy chain variable domain of SEQ ID NO: 114; and an antibody light chain variable domain of SEQ ID NO: 113.
50. The multi-function protein according to any one of items 1 to 41, characterized in that the MCSP binding site comprises SEQ ID NO: 114 and SEQ ID NO: 113.
51. The multi-function protein according to any one of items 1 to 41, characterized in that the MCSP binding site comprises an antibody heavy chain variable domain having at least 95% sequence identity to the amino acid sequence of SEQ ID NO: 116; and an antibody light chain variable domain having at least 95% sequence identity to the amino acid sequence of SEQ ID NO: 115.
52. The multi-function protein according to any one of items 1 to 41, characterized in that the MCSP binding site comprises an antibody heavy chain variable domain of SEQ ID NO: 116; and an antibody light chain variable domain of SEQ ID NO: 115.
53. The multi-function protein according to any one of items 1 to 41, characterized in that the MCSP binding site comprises SEQ ID NO: 116 and SEQ ID NO: 115.
54. A nucleic acid encoding the multi-function protein of any one of items 1 to 53.
55. A host cell comprising the nucleic acid of item 53.
56. A pharmaceutical formulation comprising the multi-function protein according to any one of items 1 to 53 and optionally a pharmaceutically acceptable carrier.
57. The multi-function protein according to any one of items 1 to 53 for use as a medicament.
58. The multi-function protein according to any one of items 1 to 53 for use in treating cancer.
59. The multi-function protein according to any one of items 1 to 53 for use in attracting virus-specific cytotoxic T-cells of an individual to a target.
60. The multi-function protein according to any one of items 1 to 53 for use in removal of cancer cells or virus infected cells.
61. Use of the multi-function protein according to any one of items 1 to 53 in the manufacture of a medicament.
62. The use according to item 61, wherein the medicament is for treatment of cancer.
63. The use according to item 61, wherein the medicament is for attracting virus-specific cytotoxic T-cells of an individual to a target.
64. The use according to item 61, wherein the medicament is for removal cancer cells.

### V. EXAMPLES

The following are examples of methods and compositions of the invention.

### Example 1

### Procedure for isolation and stimulation of CMV-specific CD8 positive T-cells from human donors

### Isolation of PBLs

PBL were isolated by Ficoll gradient centrifugation from human donor blood (Greiner bio-one, Cat. No. 227290). PBLs were cultured in RPMI supplemented with 5 % human serum (Sigma Cat. No. H2520), 2 mM L-glutamine (PAN Biotech, Cat. No. P04-80100), 100 µg/ml Penicillin/Streptomycin (Roche, Cat. No.14001100).

### Stimulation of PBLs

Cells (2 x 10⁷ cells/ml) were cultured in cell culture medium supplemented with 50 µg/ml CMV pp65-derived peptide (SEQ ID NO: 01) for two hours under cell culture conditions (37 °C, 5 % CO₂, 80 % humidity). Thereafter the cell suspension was 20-fold diluted with culture medium and further cultured in flat-bottom 96-well plates at a seeding density of 2 x 10⁵ cells per 96 well. After 4 to 5 days, 20 U/ml IL-2 (Roche, Cat. No. 11011456001), 25 ng/ml IL-7 (Peprotech, Cat. No. 200-01) and 25 ng/ml IL-15 (Peprotech, Cat. No. 200-15) were added and the cells were cultured for another 7 to 8 days. Stimulation of T-cells is visible under the microscope as cell clusters.

### Re-Stimulation of PBLs

T-cells were co-cultured with stimulator cells, which are peptide-pulsed autologous primary PBLs of the same donor (either freshly prepared or derived from frozen stocks). The stimulator cells were pulsed with the peptide as described above. After the two hours of peptide incubation the PBLs were irradiated (4000 rad; STS GmbH OB29 Nr.9510-5) and washed twice in culture medium without peptide. The re-stimulation was carried out in 96 well plates round bottom plates. 8 x 10⁴ to 1 x 10⁵ stimulator cells were used per 96 well. Cells from the primary culture were washed twice with culture medium, resuspended in 200 µl culture medium and 80 µl were transferred to each well of the stimulator cells. After 3 days 20 U/ml IL-2, 25 ng/ml IL-7 and 25 ng/ml IL-15 were added. Cells did proliferate and were expanded every 2 to 3 days in new wells with fresh medium.

### Analysis of T-cells

Cells were stained for CD8 expression (BD, Cat. No. 345772) and CMV-specific T-cell receptors (ProImmune, Cat. No. F008-4A-E) and analyzed in FACS.

### Cell culture medium

RPMI1640 (PAN Biotech, Cat. No. P04-17500), 5 % Human Serum (HS; Sigma Cat. No. H2520), 2 mM L-glutamine (PAN Biotech, Cat. No. P04-80100), 100 µg/ml Penicillin/Streptomycin (Roche, Cat. No. 14001100).

### Results

FACS analysis of four human donor derived peripheral blood lymphocytes (PBLs) was performed. The cells were labeled with a FITC-conjugated anti-CD8 antibody (BD, Cat. No. 345772) combined with APC-conjugated Pro5 pentamer (ProImmune, Cat. No. F008-4A-E) to stain T-cells which carry a T-cell receptor (TCR) recognizing MHC-class I (HLA-A*0201) loaded with CMV-derived peptide (NLVPMVATV (SEQ ID NO: 01)). For results see Figure 4. At day 0 donor 1 and 4 carry low numbers of CMV-specific CD8 T-cells (0.08% and 0.1 %, respectively). Donor 2 and 3 carry a higher number of CMV-specific CD8 T cells in their peripheral blood (0.25 % and 3.12 %, respectively). Fourteen days later after stimulation with CMV-derived peptide pulsed autologous cells only donors 2 and 3 show a significant increase in CMV-specific CD8 T cells (6.2 % and 71.2 %, respectively) whereas donors 1 and 4 do not show increased numbers of CMV-specific CD8 T cells (0.01 % and 0.05 %, respectively). Another 14 days later after a second stimulation with CMV-derived peptide pulsed autologous cells donors 2 and 3 show a further increase in CMV-specific CD8 T cells (15.1 % and 96.6 %, respectively).

### Example 2

### Cytotoxicity Assay

Acute lymphoblastic leukemia cells MN60 carry the A*0201 HLA-A allele. MN60 cells (1 x 10⁶ cells/ml) were incubated with 50 µg/ml CMV pp65 peptide (SEQ ID NO: 01) for 45 minutes under cell culture conditions (37 °C, 5 % CO₂, 80 % humidity). The incubation results in a peptide exchange in the HLA-A*0201 peptide binding groove. The peptide exchanged MN60 cells were centrifuged and diluted to a density of 1 x 10⁶ cells/ml with PBS (PanBiotech, Cat. No. P04-36500) and stained with 1 µM of the cell tracer carboxyfluorescein succinimidyl ester (CFSE, Invitrogen, Cat. No. 34554) 15 minutes at room temperature (RT). Cells were washed thereafter once with PBS and diluted to 1 x 10⁵ cells/ml with AIM-V media (Gibco, Cat. No. 0870112DK). For the assay MN60 cells (target cells) were co-cultured in 96-well round bottom plates with CMV-specific human donor 3 derived CD8⁺ T-cells (effector cells, see example 1) for four hours under cell culture conditions. The effector to target cell ratio of was 4:1. Dead cells are stained with 1 µl/100 µl propidium iodide (PI, Sigma, Cat. No. P-4864) and were FACS analyzed.

### Results

Flow Cytometric Analysis was performed to analyze the cytolytic capability of stimulated CTLs through lysis of MN60 tumor cells loaded with CMV peptide:
A co-culture of MN60 cells not loaded with the CMV-derived peptide was performed. MN60 cells are FITC-positive. Effector cells are FITC-negative. Dead cells are PI positive, alive cells are PI-negative. More than 85 % of the MN60 cells are alive when they are not loaded with the CMV-derived peptide (Q2 and Q4).

A co-culture of MN60 cells loaded with the CMV-derived peptide was performed. More than 80 % of the MN60 cells are dead (Q2 and Q4) whereas the ratio of alive and dead effector cells is not remarkably altered between the FACS analysis indicating a specific lysis of CMV-peptide-loaded target cells.

Flow Cytometric Analysis to analyze the cytolytic capability of stimulated CTLs through lysis of MN60 tumor cells loaded with CMV peptide depending on the effector to target cell ratio:
The cytotoxic assay was performed as described above. Different effector cell to target cell ratios were applied ranging from 0.5 effector cells per target cell to four effector cells per target cell. Incubation time was four hours. MN60 cells which were not loaded with the CMV-derived peptide do not show an increased number of dead cells with an increased effector to target ratio, i.e. ranging from 8 % to 13 % with ratio 0.5:1 to 4:1.

Almost 20 % of the MN60 cells loaded with CMV-derived peptide are already killed with a low effector to target ratio of 0.5:1 within four hours. The number of dead cells increases steeply with an increase in effector to target ratio reaching up to 83 % at a ratio of 4:1 effector cells per target cell.

### Example 3

### DNA preparation, transfection, expression, purification and analysis

### DNA preparation

250 ml of overnight bacterial LB culture were harvested and plasmid DNA was extracted according to the manufacturer's protocol (High speed Maxi kit, Qiagen, Cat. No. 12663). The resulting plasmid DNA was eluted in 1 ml TE buffer and DNA concentration was determined by spectrophotometric measurement (Epoch, BioTek).

The final expression vector comprised the following elements:
- the endonucleolytic restriction sites HindIII, NheI,
- a CMV-promoter,
- a 5'UTR 1 (derived from the human CMV),
- Intron A,
- a 5'UTR 2,
- an ampicillin-resistance gene,
- a BGH poly A site (bovine growth hormone polyadenylation signal),
- pUC Ori.

Amino acid sequences of the elements of the multi-function protein comprising a CMV-derived peptide and IGF1R binding specificity (anti-IGF1R antibody):

| | |
|---|---|
| CMV pp65 Peptide: | SEQ ID NO: 01 |
| | NLVPMVATV |
| Linker 1: | SEQ ID NO: 82 |
| | GGGGSGGGGSGGGGS |
| β2-microglobulin: | SEQ ID NO: 71 |
| | |
| Linker 2: | SEQ ID NO: 83 |
| | GGGGSGGGGSGGGGSGGGGS |
| HLA-A*0201 α1 - α3: | SEQ ID NO: 72 |
| | |
| Linker 3: | SEQ ID NO: 73 |
| | GS |
| Linker 4: | SEQ ID NO: 83 |
| | GGGGSGGGGSGGGGSGGGGS |
| Linker 13: | SEQ ID NO: 136 |
| | GSG |
| Ig light chain: | SEQ ID NO: 120 |
| | |
| Ig heavy chain (IgG1-L234A, L235A mutant): SEQ ID NO: 121 | |
| | |
| Ig heavy chain (IgG1-L234A, L235A mutant with knob variation): SEQ ID NO: 122 | |
| | |
| Ig heavy chain (IgG1-L234A, L235A mutant with hole variation): SEQ ID NO: 123 | |
| | |
| | |
| Ig heavy chain Fc-region (IgG1-L234A, L235A mutant Fc-region knob variant): SEQ ID NO: 124 | |
| | |
| scFv: | SEQ ID NO: 125 |
| | |

Amino acid sequences of the elements of the multi-function protein comprising a CMV-derived peptide and MCSP binding specificity (anti-MCSP antibody):

| | |
|---|---|
| CMV pp65 Peptide: | SEQ ID NO: 01 |
| | NLVPMVATV |
| Linker 1: | SEQ ID NO: 82 |
| | GGGGSGGGGSGGGGS |
| β2-microglobulin: | SEQ ID NO: 71 |
| | |
| | |
| Linker 2: | SEQ ID NO: 83 |
| | GGGGSGGGGSGGGGSGGGGS |
| HLA-A*0201 α1 - α3: | SEQ ID NO: 72 |
| | |
| Linker 3: | SEQ ID NO: 73 |
| | GS |
| Linker 4: | SEQ ID NO: 83 |
| | GGGGSGGGGSGGGGSGGGGS |
| Linker 13: | SEQ ID NO: 136 |
| | GSG |
| Ig light chain: | MHCI-0008 |
| | |
| | |
| Ig light chain: | MHCI-0030 and MHCI-0031 |
| | SEQ ID NO: 127 |
| | |
| | |
| Ig heavy chain (IgG1-L234A, L235A mutant): | |
| | MHCI-0008 |
| | SEQ ID NO: 128 |
| | |
| Ig heavy chain (IgG1-L234A, L235A mutant): | |
| | MHCI-0030 and MHCI-0031 |
| | SEQ ID NO: 129 |
| | |
| Ig heavy chain (IgG1-L234A, L235A mutant with knob variation): | |
| | MHCI-0008 |
| | SEQ ID NO: 130 |
| | |
| Ig heavy chain (IgG1-L234A, L235A mutant with knob variation): | |
| | MHCI-0030 and MHCI-0031 |
| | SEQ ID NO: 131 |
| | |
| Ig heavy chain (IgG1-L234A, L235A mutant with hole variation): | |
| | MHCI-0008 |
| | SEQ ID NO: 132 |
| | |
| | |
| Ig heavy chain (IgG1-L234A, L235A mutant with hole variation): | |
| | MHCI-0030 and MHCI-0031 |
| | SEQ ID NO: 133 |
| | |
| Ig heavy chain Fc-region (IgG1-L234A, L235A mutant Fc-region knob variant): | |
| | SEQ ID NO: 124 |
| | |
| scFv: | MHCI-0008 |
| | SEQ ID NO: 134 |
| | |
| | |
| scFv: | MHCI-0030 and MHCI-0031 |
| | SEQ ID NO: 135 |
| | |

### Transfection

HEK 293 cells were diluted to 8 x 10⁵ cells/ml the day before transfection. About 1 to 1.6 x 10⁶ cells/ml were transfected according to the manufacturer's protocol. For a final transfection volume of 30 ml, 30 µg DNA were diluted to a final volume of 1 ml with Opti-MEM® I Reduced Serum Medium (Gibco, Cat. No. 31985070). 2 µl of 293fectin™ Reagent (Invitrogen, Cat. No. 12347019) per 1 µg DNA were equally diluted to a final volume of 1 ml with Opti-MEM® medium and incubated for 5 minutes. After incubation the diluted DNA was added to the diluted 293fectin^{™}Reagent, gently mixed, incubated for another 20-30 minutes and afterwards drop wise pipetted to 28 ml of the HEK 293 cells to obtain a final volume of 30 ml. The cells were incubated under cell culture condition (37 °C, 8 % CO₂, 80 % humidity) on an orbital shaker rotating at 125 rpm and harvested after 72 hours. The harvest was centrifuged for 10 minutes at 1000 rpm, for 10 minutes at 3000 rpm and filtered through a 22 µm sterile filter (Millipore, Cat. No. SCGPU05RE).

### Western Blotting

500 µl of cell culture supernatant was concentrated with Pall Nanosep Omega-Membrane 30KD Centrifugal Devices (Pall, Cat. No. OD030C33) to a volume of 50 µl. 17.5 µl of each concentrate was diluted to a final volume of 25 µl with 4x XT Sample Buffer (Bio Rad, Cat. No. 161-0791) and 20x XT Reducing Agent (BioRad, Cat. No. 161-0792), heated for 8 minutes at 96 °C and applied on a 4-12 % Criterion XT Precast Gel (Cat. No. 345-0124). Blotting was performed with Trans-Blot SD semi-dry Transfer Cell (BioRad) at 20 V for 30 minutes on a Trans-blot Pure Nitrocellulose membrane (0.45 µm) (BioRad, Cat. No. 162-0117). Blocking of the membrane was performed with 1x Western Blocking Reagent (Roche, Cat. No. 11921681001) for one hour at room temperature. Staining was performed with peroxidase conjugated polyclonal rabbit anti-human κ-light chain (DAKO, Cat. No. P0129, diluted 1:3000) and polyclonal rabbit anti-human IgG antibody HRP conjugate (DAKO, Cat. No. P0214, diluted 1:5000) for one hour at room temperature. Luminescence detection was carried out with LUMI-Imager F1 (Roche).

### Purification

Cells were removed from culture medium by centrifugation. Multi-function proteins were purified from supernatants by protein A affinity chromatography (MabSelect-Sepharose on an ÄKTA-Avant). Eluted multi-function proteins were concentrated with Amicon centrifugation tubes to a protein concentration of 3 mg/ml. An aliquot was analyzed on a size exclusion chromatography (HPLC TSKgel GFC300 Sys89). Preparative SEC on a Superdex 200 was performed to remove aggregates and buffer the fusion proteins in 20 mM histidine, 140 mM NaCl, pH 6.0. Eluted multi-function proteins were concentrated with Amicon centrifugation tube to a protein concentration of 1 mg/ml and sterile filtered (0.2 µm pore size).

### Analytics

Multi-function protein samples were analyzed by OD280 using a UV spectrophotometer to determine the protein concentration in solution. The extinction coefficient required for this was calculated from the amino acid sequence according to Pace, C.N., et al., Protein Science 4 (1995) 2411-2423). Size-exclusion chromatography (SE-HPLC) was performed on TSK-Ge1300SWXL or Superdex 200 columns with a 0.2 M potassium phosphate buffer, comprising 0.25 M KCl, pH 7.0 as mobile phase in order to determine the content of monomeric, aggregated and degraded species in the samples. Sodium dodecyl sulfate (SDS) polyacrylamide gel electrophoresis (reducing and non-reducing) was performed to analyze the purity of the multi-function protein preparations with regard to product-related degradation products and unrelated impurities. Electrospray ionization mass spectrometry (ESI-MS) was performed with reduced (TCEP) and deglycosylated (N-glycosidase F) samples to confirm the correct mass/identity of each chain and detect chemical modifications. ESI-MS of the deglycosylated samples was carried out to analyze the nature and quality of the fully assembled protein and detect potential product-related side products.

### Method SDS-PAGE and Coomassie Staining

| | |
|---|---|
| Device: | Invitrogen XCell Sure Lock Mini-Cell |
| Gel: | 4-20% Tris-Glycine Gel, Invitrogen EC6025BOX |
| Buffer: | Tris-Glycine SDS Running Buffer (10x), Invitrogen LC2675-5 |
| Sample buffer: | Tris-Glycine SDS Sample Buffer (2x), Invitrogen LC2676 |
| Reducing buffer: | NuPAGE Sample Reducing Agent (10x), Invitrogen NP0004 |
| Molecular Weight Marker: | Mark 12, MW Standard, Invitrogen LC5677 |

### Protein Sample preparation

The sample was adjusted to a protein concentration of 1 mg/ml with buffer. For sample reduction the following procedure was carried out:
- reduction buffer: 4 ml Sample buffer (2x) and 1 ml reducing buffer (10x),
- dilute sample 1:1 with reduction buffer,
- incubate for 5 minutes at 70 °C.

The gel electrophoresis was carried out at 125 V for 90 minutes. The gels were stained with Simply Blue Safe Stain (Invitrogen, Cat. No. LC6065).

### Results

**Table.**

| **No.** | **polypeptides comprised in the multi-function protein with IGF-1R binding specificity** | **scheme** | **yield** |
|---|---|---|---|
| 1 | 1. [CMV-pp65-peptide]-[linker 1]-[β2-microglobulin]-[linker 2]-[HLA-A-α1-α2-α3]-[linker 3]-[IgG1-L234A, L235A mutant with hole variation] | 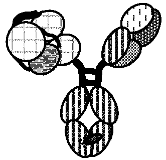 | 5 mg/l |
| | 2. Ig heavy chain (IgG1-L234A, L235A mutant with knob variation) | | |
| | 3. Ig light chain | | |
| 2 | A: | 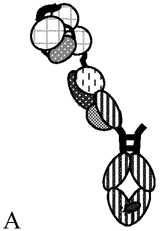 | A: 5-18 mg/l |
| | 1. [CMV-pp65-peptide]-[linker 1]-[β2-microglobulin]-[linker 2]-[HLA-A-α1-α2-α3]-[linker 3]-[IgG1-L234A, L235A mutant with hole variation] | | |
| | 2. IgG1-L234A, L235A mutant Fc-region knob variant | 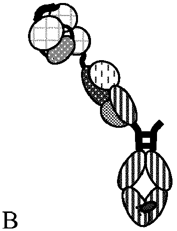 | |
| | 3. Ig light chain | | |
| | B: Fusion to the Ig light chain | | |
| 3 | A: | 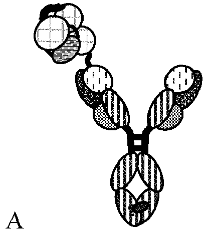 | A: 4 - 23 mg/l |
| | 1. [CMV-pp65-peptide]-[linker 1]-[β2-microglobulin]-[linker 2]-[HLA-A-α1-α2-α3]-[linker 3]- [IgG1-L234A, L235A mutant with hole variation] | | |
| | 2. IgG1-L234A, L235A mutant with knob variation | 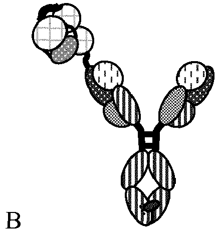 | |
| | 3. Ig light chain | | |
| | B: Fusion to the Ig light chain | | |
| 4 | 1. [CMV-pp65-Peptide]-[Linker 1]-[β2-microglobulin]-[Linker 2]-[HLA-A-α1-α2-α3]-[Linker 3]-[IgG1-L234A, L235A mutant with hole variation] | 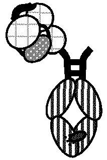 | 4 mg/l |
| | 2. IgG1-L234A, L235A mutant with knob variation | | |
| 5 | 1. [CMV-pp65-peptide]-[linker 1]-[β2-microglobulin]-[linker 2]-[HLA-A-α1-α2-α3]-[linker 3]-[IgG1-L234A, L235 A-Fc-region] | 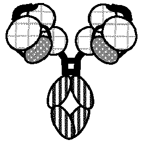 | 4 mg/l |
| 6 | 1. [CMV-pp65-peptide]-[linker 1]-[β2-microglobulin]-[linker 2]-[HLA-A-α1-α2-α3]-[linker 3]-[IgG1-L234A, L235A mutant Fc-region]-[linker 4]-[scFv] | 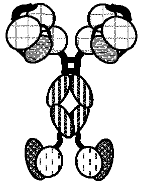 | < 1 µg/l |
| 7 | A: 1. [CMV-pp65-peptide]-[linker 1]-[β2-microglobulin]-[linker 2]-[HLA-A-α1-α2-α3]-[linker 3]-[IgG1-L234A, L235A mutant] | 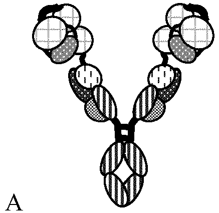 | < 1 µg/l |
| | 2. Ig light chain | 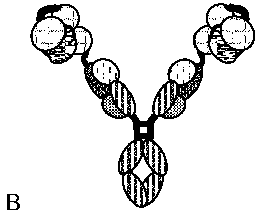 | |
| | B: Fusion to the Ig light chain | | |

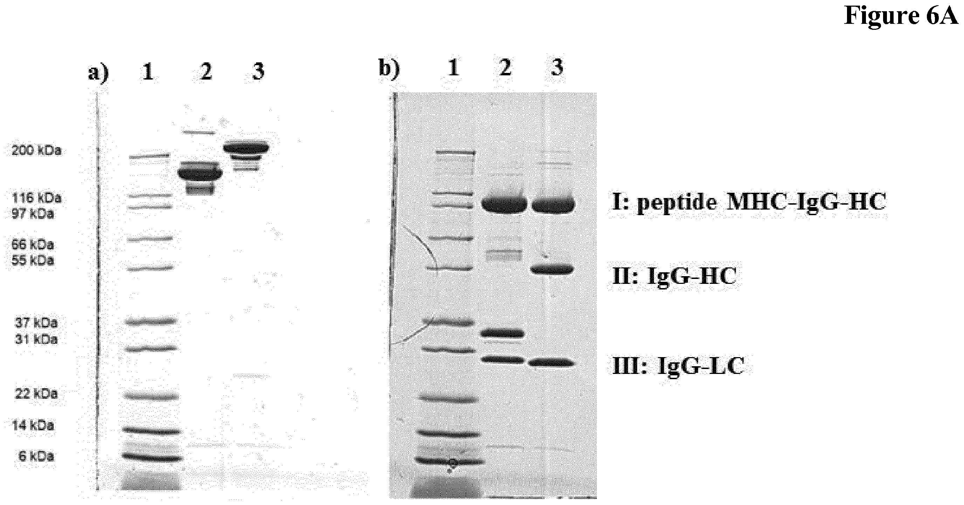

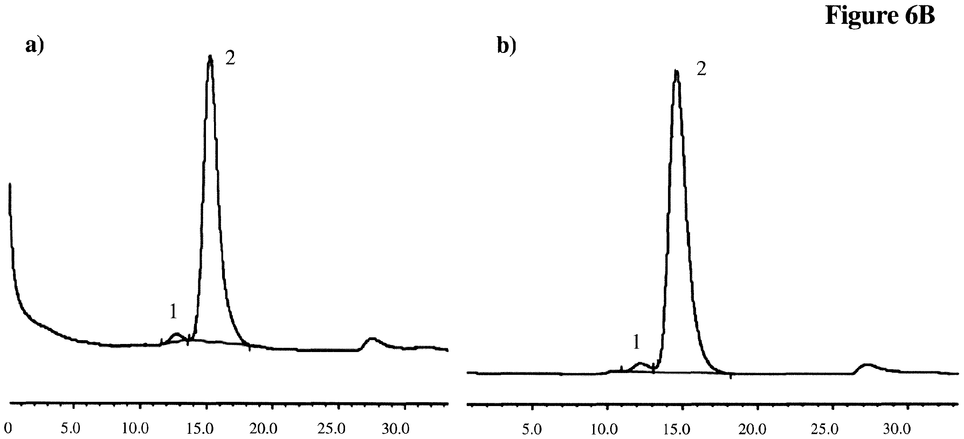

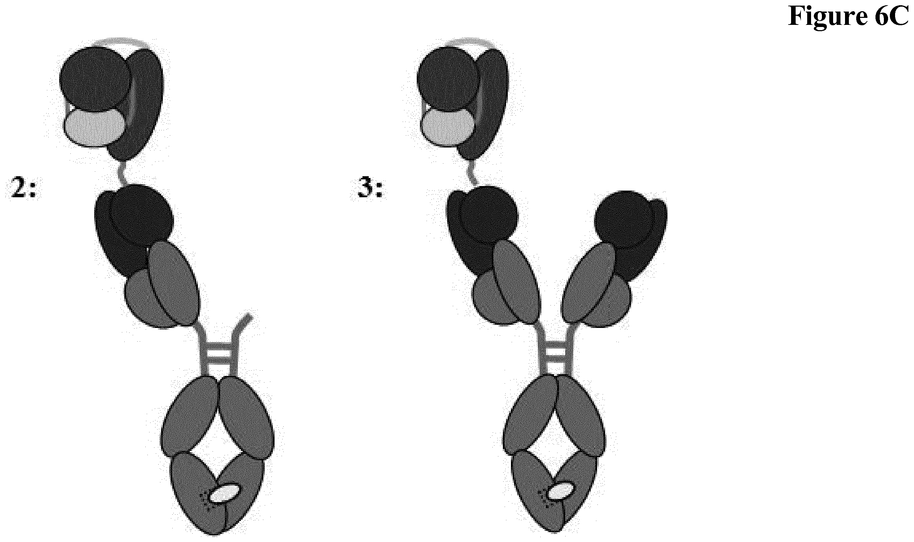

The SDS gel with Coomassie staining and the corresponding SEC chromatograms of selected multi-function proteins with a structure corresponding to number 1, 2A and 3A according to the previous table are shown in Figures 5 and 6. It can be seen that defined multi-function proteins can be obtained.

### Example 4

### Binding of MHC-I-anti-IGF-1R multi-function protein to human IGF-1R positive Cell Line

H460M2 cells were diluted to 8 x 10⁵ cells/ml in AIM-V medium (Gibco, Cat. No. 0870112DK). 500 µl of the cell suspension was stained with 10 µg of a MHC-I-anti-IGF-1R multi-function protein as reported herein either at 4 °C or 37 °C for one hour. Thereafter cells were washed once with ice-cold AIM-V medium and stained with a second antibody, which was a goat F(ab')₂ anti-human IgG (H+L) antibody conjugated to R-PE (Dianova, Cat. No. 109-116-088, dilution 1:50) for 30 minutes at 4 °C. Thereafter cells were washed once with ice-cold AIM-V medium and fluorescence was measured via FACS Canto II (BD Bioscience) with gating on living cells. A bispecific antibody served as Isotype control, an anti IGF-1R antibody (see e.g. WO 2004/087756 and WO 2007/115814) served as positive control.

### Results

Considering the shift in the PE-fluorescence measurement (X-axis), the MHC-I-anti-IGF-1R multi-function protein shows no visible difference in binding to H460M2 target cells in comparison to the control antibody. There is also no difference whether the incubation with the MHC-I-anti-IGF-1R multi-function protein is accomplished at 4 °C or 37 °C. Neither the incubation with the isotype control nor with the fluorescence labeled secondary antibody alone shows any shift in the PE fluorescence measurement. Despite the fusion of the class I MHC molecule the antibody variable domain of the MHC-I-anti-IGF-1R multi-function protein herein still binds to the H460M2 target cells.

### Example 5

### In vitro removal of antigen expressing cells

I24 target cells (1x10⁵ cells/ml) were seeded in cell culture media (RPMI 1640 supplemented with 2 mM L-glutamine, 1 mM sodium pyruvate, 0.1 mM NEAA, and 10 % (v/w) FCS) on WillCo Glass Bottom Dishes (FA. WillCo Wells BV, REF GWST-3522) for 24 to 48 hours. WillCo Glass Bottom Dishes were precoated with 50 µg/ml poly-L-lysine hydrochloride (Sigma Aldrich, Cat # P2658) per dish for 30 min. After coating the dishes were thoroughly rinsed with sterile tissue culture grade water and dried for two hours.

After the cultivation cell culture media was removed and the IGF-1R binding multi-function protein comprising one [CMV-pp65-peptide]-[linker 1]-[β2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the multi-function protein specifically binds to human IGF-1R as reported herein (see e.g. Example 3) was added in a final concentration of 5 µg/ml in 3 mM K⁺ Krebs Ringer HEPES Buffer pH 7.3 (supplemented with 0.5 mM DL-dithiothreitol, 1 mM ascorbic acid, and 4 mM glutathione).

T-cells were added in a target cell to effector cell ration of 1:10. Imaging was performed for 4 hours with a Zeiss Axiovert 135 microscope.

### Results

The IGF-1R binding multi-function protein mediated lysis of human IGF-1R expressing I24 3T3 cells (large adherently growing cells). Lysis is mediated by human CMV-specific T-cells (small cells either round shaped or amoeboid migrating cells). I24 cells are incubated with the multi-function protein at a concentration of 5 µg/ml and human CMV-specific T-cells (pre-activated with HLA-A0201⁺/CMV peptide pulsed APCs). Note the interaction of the I24 cells with the T-cells at 56 min and 76 min and subsequently the collapse of the I24 cell after 125 min.

A control showing the absence of lysis of I24 3T3 cells (large adherently growing cells, white arrowhead) through human CMV-specific T-cells (small cells either round shaped or amoeboid migrating cells) in the absence of an antigen binding multi-function protein as reported herein was performed. I24 cells are incubated with specific cytotoxic T-cells (pre-activated with HLA-A0201⁺ /CMV peptide pulsed APCs). Time lapse is indicated below the respective picture.

### Example 6

### Cytotoxicity assay

Cell culture medium (50 µl) was pipetted into each well of an Xcelligence 96well E-plate (Roche, Cat # 05232368001) to perform background measurement.

I24 cells were diluted to 1x10⁶ cells/ml in cell culture media (RPMI 1640, 2 mM L-glutamine, 1 mM Sodium pyruvate, 0.1 mM NEAA, 10 % (v/w) FCS) and 50 µl (2x10⁴ cells/well) were pipetted in each well of an Xcelligence 96well plate to a final volume of 100 µl and cultivated for 24 hours (37 °C, 8 % CO₂, 80 % humidity). After 24 hours the medium was removed and the cells were washed with 200 µl AIM-V (Serum Free Medium (Invitrogen) T-cell medium (Cat-No): 12055-083) medium. The IGF-1R binding multi-function protein comprising one [CMV-pp65-peptide]-[linker 1]-[β2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the multi-function protein specifically binds to human IGF-1R, was added to the washed target cells in a final concentration of 1 µg/ml in AIM-V medium. Effector cells in the respectable ratio were added in AIM-V media to a final volume of 150 µl. Afucosylated IgG1 monoclonal antibody directed against human IGF-1R (anti-IGF-1R antibody-afucosylated) and non-binding human anti-digoxigenin antibody (anti-digoxygenin antibody) served as Isotype control and specific antibody control, respectively. Measurement was performed for 6 to 9 hours respectively with the Xcelligence System (Roche).

### Results

The IGF-1R binding multi-function protein triggers lysis of H460M2 tumor cells through human CMV-specific T-cells.

Tumor cells were incubated for 4 hours with 1 µg/ml of the multi-function protein comprising one [CMV-pp65-peptide]-[linker 1]-[p2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the multi-function protein specifically binds to human IGF-1R, and specific T-cells in the respective ratio (1:1.5 to 1:0.5) (see Figure 8). Percentage of lysis is denoted above the respective bars. Afucosylated IgG1 monoclonal antibody directed against human IGF-1R (MAB IGF-1R-afu) did not trigger a significant tumor cell lysis.

The multi-function protein as reported herein triggers lysis of I24 3T3 target cells through human CMV-specific T-cells.

Target cells were incubated for 4 hours with 1 µg/ml of an antigen binding multi-function protein comprising one [CMV-pp65-peptide]-[linker 1]-[β2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the multi-function protein specifically binds to human IGF-1R, and specific T-cells in the respective ratio (1:1.5 to 1:0.5) (see Figure 9). Percentage of lysis is denoted above the respective bars. Afucosylated IgG1 monoclonal antibody directed against human IGF-1R (anti-IGF-1R antibody-afucosylated) and non-binding human anti-Digoxigenin antibody (anti-digoxygenin antibody) did not trigger a significant target cell lysis.

### Example 7

### In vitro efficacy

IGF-1R positive lung adenocarcinoma cell line H460M2 was incubated with 1 µg/ml of a multi-function protein comprising an hCMV-derived peptide and an anti-IGF-1R antibody and human CMV-specific CD8-positive T-cells at a low effector cell to target cell ratio (1.5 to 0.5 specific T-cells per tumor cell). Control antibody was a glyco-engineered anti-IGF-1R antibody. The incubation time was 6 hours. The incubation with multi-function protein results in a potent removal of H460M2 tumor cells.

### Example 8

### Binding of different MHC-I-anti-MCSP multi-function protein to MCSP positive target cells

Colo38 cells were incubated for 5 min. with Accutase (PAA, Cat.# L11-007) to obtain a single cell suspension. 2x10⁵ cells per vial were incubated with 1 µg/ml MHC-I-anti-MCSP multi-function protein construct in 100 µl PBS/2%FCS for 45 min. at 4°C. After incubation cells were washed with 1 ml cold PBS/2%FCS and centrifuged for 7 min. with 910 rpm. Cells were resuspended in 100 µl PBS/2%FCS with secondary antibody (goat anti-human IgG1 antibody-PE conjugate, Jackson Lab., Cat.# 109-116-088) at 2 µg/ml and incubated for another 45 min. at 4°C. Cells were washed twice with 1 ml PBS%2%FCS and measured with BD Canto II Flow Cytometer. The results are shown in Figure 7.

### Example 9

### Incubation of MCSP positive cells with MHC-I-anti-MCSP multi-function proteins

Colo38 or WM266-4 cells were incubated for 5 min. with Accutase (PAA, Cat.# L11-007) to obtain a single cell suspension. 2x10⁴ cells of the Colo38 cell line or 1x10⁴ cells of the WM266-4 cell line per well were incubated for 24 h in Eplates96 (Roche, Cat.#05232368001) in 100 µl of the respective cell culture medium (Colo38 cell line: RPMI1640 supplemented with 2 mM glutamine, 10 % FCS; WM-266-4 cell line: RPMI1640 supplemented with 2 mM glutamine, 10 % FKS, 2 mM sodium pyruvate, 2 mM NEAA) and adherence (impedance) was measured every 15min with ACEA technology (Xcelligence RTCA). After 24h (undisturbed growth phase) the cells were washed with 200 µl of AIMV-medium (Gibco, Cat.# 0870112DK). MHC-I-anti-MCSP multi-function proteins were added in a final concentration of 1 µg/ml together with stimulated T-cells or PBMCs in different ratios to a final volume of 150 µl in AIMV-medium to the cells. Incubation was continued for another 4 to 48 hours with simultaneous ACEA measurement every 5 minutes. The read-out is based on impedance measurement, detecting lysed or collapsed cells as detached from the Eplate bottom. The cell index has been normalized to 1 at the first measurement point after addition of the multi-function protein. The results for 1 µg/ml multi-function protein concentration (MHCI-0008 (1), MHCI-0010 (2), MHCI-0030 (3), MHCI-0031 (4)), effector to target cell ratio of 10:1; PBMCs from Donor 3 (200.000 cells, Donor 3 is CMV-positive but EBV negative) and melanoma tumor cell line Colo38 (20.000 cells) and per 96 well, data are triplicates is shown in Figure 14. The results for 1 µg/ml multi-function protein concentration (MHCI-0008 (1), MHCI-0010 (2), PBMCs only (3)), effector to target cell ratio of 10:1; PBMCs from Donor 3 (200.000 cells, Donor 3 is CMV-positive but EBV negative) and melanoma tumor cell line Colo38 (20.000 cells) and per 96 well, data are triplicates is shown in Figure 15A (Colo38) and 15B (WM266).

### Example 10

### Cytotoxicity assay

Cell culture medium (50 µl) was pipetted into each well of an Xcelligence 96well E-plate (Roche, Cat # 05232368001) to perform background measurement.

Colo38 cells were diluted to 1x10⁶ cells/ml in cell culture media (RPMI1640 supplemented with 2 mM glutamine, 10 % FCS) and 50 µl (2x10⁴ cells/well) were pipetted in each well of an Xcelligence 96well plate to a final volume of 100 µl and cultivated for 24 hours (37 °C, 8 % CO₂, 80 % humidity). After 24 hours the medium was removed and the cells were washed with 200 µl AIM-V (Serum Free Medium (Invitrogen) T-cell medium (Cat-No): 12055-083) medium. The MCSP binding multi-function proteins MHCI-0008 (monovalent, CMV peptide loaded), MHCI-0010 (monovalent, EBV peptide loaded control), MHC-0026 (bivalent, CMV peptide loaded, non-binding control), MHCI-0030 (monovalent, CMV peptide loaded, active) and MHCI-0031 (bivalent, CMV peptide loaded, active) were individually added to the washed target cells in a final concentration of 1 µg/ml in AIM-V medium. Effector cells in the respectable ratio of 10:1 (E:T) were added in AIM-V media to a final volume of 150 µl. Measurement was performed 42 hours post addition with the Xcelligence System (Roche).

The results obtained for 200.000 PBMCs (effector cells) freshly isolated from Donor 3 co-cultured with 20.000 adherent Colo38 cells (96 well plates in triplicates) are shown in Figure 16 (lysis of cells after 42 hours of incubation with multi-function protein). 25% of the PBMCs are CD8-positive T cells of which in turn 3 % are CMV-pp65-peptide specific resulting in approx. 1.500 CMV-pp65-peptide-specific CD8+ T cells per 20.000 Colo38 target cells (real E:T (Effector to Target Cell Ratio) = 1:13).

### Results

The MCSP binding multi-function protein triggers lysis of Colo38 tumor cells through human CMV-specific T-cells.

### Example 11

### LDH Release Assay

ACEA plates were centrifuged for 7 min. at 910 rpm. 50 µl of ACEA supernatants were transferred in another 96well flat bottom plate (Costar). LDH reagent (Cytotoxicity Detection Kit, Roche, Cat.# 11644793001) 1 and 2 are diluted according to the manufacturer's instructions and 50 µl of the solution were added to the supernatant. Absorption was detected after an incubation period of 5 to 25 minutes in Tecan Reader Sunrise (Tecan). Total lysis is detected through addition of 1 % Triton X-100 (Sigma, Cat.# T-8787) to the target cells before centrifugation.

The results obtained for 200.000 PBMCs (effector cells) freshly isolated from Donor 3 co-cultured with 20.000 adherent Colo38 cells (96 well plates in triplicates) are shown in Figure 17 (LDH release after 48 hours of incubation with multi-function protein). 200.000 PBMCs freshly isolated from Donor 3 were co-cultured with 20.000 adherent Colo38 cells (96 well plates in triplicates). 25% of the PBMCs are CD8-positive T cells of which in turn 3 % are CMV-pp65-peptide specific resulting in approx. 1.500 CMV-pp65-peptide-specific CD8+ T cells per 20.000 Colo38 target cells (E:T (Effector to Target Cell Ratio) = 1:13)

### Results

The MCSP binding multi-function protein triggers lysis of Colo38 tumor cells through human CMV-specific T-cells.

### Example 12

### Cytotoxicity assay

PBMCs were obtained from whole blood via Ficoll centrifugation. 1x10⁷ PBMCs per ml were diluted in T-cell medium (RPMI 1640 supplemented with 10 % HS, 2 mM glutamine) and peptide exchange on HLA-A0201 molecules was accomplished by addition of 50 µg/ml CMV pp65 peptide to the suspension. After 2-3 h incubation the PBMCs were diluted 1:10 and plated á 200µl in 96well round bottom plates. On day 3 20 U/ml IL-2, 25 ng/ml IL-7 and IL-15 were added. After 14 d a re-stimulation was performed.

The stimulated T-cells were washed two times in the 96well plates and diluted in 200 µl T-cell medium from which 80 µl were transferred in new 96well round bottom plates.

PBMCs were stimulated according to the protocol above. Stimulated PBMCs were irradiated after peptide exchange with 4000 Gray, washed with T-cell medium twice and 1x10⁵ PBMCs were pipetted to the 80 µl of T-cells. On day 3 20 U/ml IL-2, 25 ng/ml IL-7 and IL-15 were added. An Xcelligence cytotoxicity assay was performed with re-stimulated T-cells on day 11.

63% of the effector cells are CMV-pp65-peptide specific CD8⁺ T-cells.

The target cell to effector cell ratio was 1:3.5. Cell lysis was determined 10 hours after addition of the respective multi-function protein. The multi-function fusion protein was added to a final concentration of 1µg/ml.

The results are shown in Figure 18A for Colo38 cells and in Figure 18B for WM266 cells.

### Example 13

### Disulfide-stabilized multi-function proteins

A disulfide-bridge between position 11 and 227 of the antigen presenting domain in the multi-function protein as reported herein has been introduced.

The amino acid sequence of the disulfide stabilized antigen presenting domain is:

Without disulfide stabilization 6.4 mg of multi-function protein can be obtained from 11 cultivation supernatant after protein A affinity purification. The final yield after size exclusion chromatography to separate aggregates was 2.2 mg.

With disulfide stabilization 17.8 mg of multi-function protein can be obtained from 11 cultivation supernatant after protein A affinity purification. The final yield after size exclusion chromatography was 11.4 mg due to a lower amount of aggregates due to increased thermal stability by the introduced disulfide bridge.

The analytical size exclusion chromatograms after protein A affinity chromatography but prior to aggregate removal by preparative size exclusion chromatography are shown in Figure 19.

The disulfide-linked multi-function proteins show the same functionality as the non-disulfide-linked multi-function proteins.

PBMCs were obtained from whole blood via Ficoll centrifugation. 1x10⁷ PBMCs per ml were diluted in T-cell medium (RPMI 1640, supplemented with 10 % HS, 2 mM glutamine) and peptide exchange on HLA-A0201 molecules was accomplished by addition of 50 µg/ml CMV pp65 peptide to the suspension. After 2-3 h incubation the PBMCs are diluted 1:10 and plated á 200µl in 96well round bottom plates. On day 3 20 U/ml IL-2, 25 ng/ml IL-7 and IL-15 were added. Cells were taken 11 d after primary stimulation; 45 % of the cells were CMV specific. The results for a target cell to effector cell ration of 1:3 are shown in Figure 20.

## Claims

1. A multi-function protein, **characterized in that** it comprises
- exactly one antigen presenting domain,
- exactly one antibody Fc-region, and
- at least one antigen binding site,
wherein the antigen presenting domain comprises in N- to C-terminal direction
either
(i) a T-cell response eliciting peptide,
(ii) a β2-microglobulin, and
(iii) the extracellular domains α1, α2, and α3 of a class I MHC molecule selected from the group comprising HLA-A*0201, HLA-A*1101, HLA-A*2402, HLA-A*340101, HLA-C*0304, HLA-C*0401, and HLA-C*0702,
or
(i) a T-cell response eliciting peptide,
(ii) the extracellular domains α1, α2, and α3 of a class I MHC molecule selected from the group comprising HLA-A*0201, HLA-A*1101, HLA-A*2402, HLA-A*340101, HLA-C*0304, HLA-C*0401, and HLA-C*0702, and
(iii) a β2-microglobulin,
wherein the antigen binding site binds to melanoma-associated chondroitin sulfate proteoglycan (MCSP).

2. The multi-function protein according to claim 1, **characterized in that** the antibody Fc-region comprises a first and second disulfide-linked Fc-region polypeptide, whereby the antigen binding site comprises the first Fc-region polypeptide.

3. The multi-function protein according to any one of claims 1 to 2, **characterized in that** the antigen binding site comprises i) a pair of an antibody heavy chain and an antibody light chain, or ii) a scFv fusion polypeptide comprising in N- to C-terminal direction a scFv antibody fragment and an antibody Fc-region polypeptide, or iii) a scFab fusion polypeptide comprising in N- to C-terminal direction a scFab and an antibody Fc-region polypeptide.

4. The multi-function protein according to any one of claims 1 to 3, **characterized in that** i) the antigen presenting domain is linked to the N-terminus of the heavy chain or to the N-terminus of the light chain of the antigen binding site, or ii) the antigen presenting domain is linked to the C-terminus of the heavy chain or to the C-terminus of the light chain of the antigen binding site, or iii) the antigen presenting domain is linked to the Nor C-terminus of the scFv fusion polypeptide, or iv) the antigen presenting domain is linked to the N- or C-terminus of the scFab fusion polypeptide, or iv) the antigen presenting domain is linked to the N- or C-terminus of the second Fc-region polypeptide.

5. The multi-function protein according to any one of claims 1 to 4, **characterized in that** the T-cell response eliciting peptide is a virus-derived peptide.

6. The multi-function protein according to claim 5, **characterized in that** the virus-derived peptide is a human cytomegalovirus-derived peptide.

7. The multi-function protein according to any one of claims 1 to 6, **characterized in that** the virus-derived peptide has the amino acid sequence of SEQ ID NO: 01.

8. The multi-function protein according to any one of claims 1 to 7, **characterized in that** the antigen presenting domain comprises
(i) a virus-derived peptide,
(ii) β2-microglobulin, and
(iii) the soluble HLA-A allele A*0201.

9. The multi-function protein according to any one of claims 1 to 8, **characterized in that** the antigen presenting domain comprises in N- to C-terminal direction
(i) a virus-derived peptide that has an amino acid sequence selected from the group comprising SEQ ID NO: 01 to SEQ ID NO: 70,
(ii) a first linker peptide that has an amino acid sequence selected from the group comprising SEQ ID NO: 77, 78, 79, 82, 83, and 84,
(iii) a β2-microglobulin that has an amino acid sequence of SEQ ID NO: 71,
(iv) a second linker peptide that has an amino acid sequence selected from the group comprising SEQ ID NO: 77, 78, 79, 82, 83, and 84,
(v) the extracellular domains α1, α2, and α3 of a class I MHC molecule that has an amino acid sequence of SEQ ID NO: 72, and
(vi) a third linker peptide that has an amino acid sequence selected from the group comprising SEQ ID NO: 73, 77, 78, 79, 82, 83, 84, and 136.

10. A pharmaceutical formulation comprising the multi-function protein according to any one of claims 1 to 9 and optionally a pharmaceutically acceptable carrier.

11. The multi-function protein according to any one of claims 1 to 9 for use as a medicament.

12. The multi-function protein according to any one of claims 1 to 9 for use in treating cancer.

## Patentansprüche

1. Multifunktionelles Protein, **dadurch gekennzeichnet, dass** es Folgendes umfasst:
- genau eine antigenpräsentierende Domäne,
- genau eine Antikörper-Fc-Region und
- mindestens eine Antigenbindungsstelle,
wobei die antigenpräsentierende Domäne in Richtung des N- bis C-Terminus
entweder
(i) ein eine T-Zellantwort hervorrufendes Peptid,
(ii) ein β2-Mikroglobulin und
(iii) die extrazellulären Domänen α1, α2 und α3 eines MHC-Klasse-I-Moleküls, ausgewählt aus der Gruppe umfassend HLA-A*0201, HLA-A*1101, HLA-A*2402, HLA-A*340101, HLA-C*0304, HLA-C*0401 und HLA-C*0702,
oder
(i) ein eine T-Zellantwort hervorrufendes Peptid,
(ii) die extrazellulären Domänen α1, α2 und α3 eines MHC-Klasse-I-Moleküls, ausgewählt aus der Gruppe umfassend HLA-A*0201, HLA-A*1101, HLA-A*2402, HLA-A*340101, HLA-C*0304, HLA-C*0401 und HLA-C*0702, und
(iii) ein β2-Mikroglobulin umfasst,
wobei die Antigenbindungsstelle an Melanom-assoziiertes Chondroitinsulfat-Proteoglykan (MCSP) bindet.

2. Multifunktionelles Protein nach Anspruch 1, **dadurch gekennzeichnet, dass** die Antikörper-Fc-Region ein erstes und zweites Disulfid-gebundenes Fc-Region-Polypeptid umfasst, wobei die Antigenbindungsstelle das erste Fc-Region-Polypeptid umfasst.

3. Multifunktionelles Protein nach einem der Ansprüche 1 bis 2, **dadurch gekennzeichnet, dass** die Antigenbindungsstelle i) ein Paar einer Antikörper-Schwerkette und einer Antikörper-Leichtkette oder ii) ein scFv-Fusionspolypeptid, das in Richtung des N- bis C-Terminus ein scFv-Antikörperfragment und ein Antikörper-Fc-Region-Polypeptid umfasst, oder iii) ein scFab-Fusionspolypeptid umfasst, das in Richtung des N- bis C-Terminus ein scFab- und ein Antikörper-Fc-Region-Polypeptid umfasst.

4. Multifunktionelles Protein nach einem der Ansprüche 1 bis 3, **dadurch gekennzeichnet, dass** i) die antigenpräsentierende Domäne an den N-Terminus der Schwerkette oder an den N-Terminus der Leichtkette der Antigenbindungsstelle gebunden ist oder ii) die antigenpräsentierende Domäne an den C-Terminus der Schwerkette oder an den C-Terminus der Leichtkette der Antigenbindungsstelle gebunden ist oder iii) die antigenpräsentierende Domäne an den N- oder C-Terminus des scFv-Fusionspolypeptids gebunden ist oder iv) die antigenpräsentierende Domäne an den N- oder C-Terminus des scFab-Fusionspolypeptids gebunden ist oder iv) die antigenpräsentierende Domäne an den N- oder C-Terminus des zweiten Fc-Region-Polypeptids gebunden ist.

5. Multifunktionelles Protein nach einem der Ansprüche 1 bis 4, **dadurch gekennzeichnet, dass** das eine T-Zellantwort hervorrufende Peptid ein Virus-abgeleitetes Peptid ist.

6. Multifunktionelles Protein nach Anspruch 5, **dadurch gekennzeichnet, dass** das Virus-abgeleitete Peptid ein humanes, vom Cytomegalievirus abgeleitetes Peptid ist.

7. Multifunktionelles Protein nach einem der Ansprüche 1 bis 6, **dadurch gekennzeichnet, dass** das Virus-abgeleitete Peptid die Aminosäuresequenz SEQ ID NO: 01 aufweist.

8. Multifunktionelles Protein nach einem der Ansprüche 1 bis 7, **dadurch gekennzeichnet, dass** die antigenpräsentierende Domäne Folgendes umfasst:
(i) ein Virus-abgeleitetes Peptid,
(ii) ein β2-Mikroglobulin und
(iii) das lösliche HLA-A-Allel A*0201.

9. Multifunktionelles Protein nach einem der Ansprüche 1 bis 8, **dadurch gekennzeichnet, dass** die antigenpräsentierende Domäne in Richtung des N- bis C-Terminus Folgendes umfasst:
(i) ein Virus-abgeleitetes Peptid, das eine aus der Gruppe umfassend SEQ ID NO: 01 bis SEQ ID NO: 70 ausgewählte Aminosäuresequenz aufweist,
(ii) ein erstes Linker-Peptid, das eine aus der Gruppe umfassend SEQ ID NO: 77, 78, 79, 82, 83 und 84 ausgewählte Aminosäuresequenz aufweist,
(iii) ein β2-Mikroglobulin, das eine Aminosäuresequenz SEQ ID NO: 71 aufweist,
(iv) ein zweites Linker-Peptid, das eine aus der Gruppe umfassend SEQ ID NO: 77, 78, 79, 82, 83 und 84 ausgewählte Aminosäuresequenz aufweist,
(v) die extrazellulären Domänen α1, α2 und α3 eines MHC-Klasse-I-Moleküls, das eine Aminosäuresequenz SEQ ID NO: 72 aufweist und
(vi) ein drittes Linker-Peptid, das eine aus der Gruppe umfassend SEQ ID NO: 73, 77, 78, 79, 82, 83, 84 und 136 ausgewählte Aminosäuresequenz aufweist.

10. Pharmazeutische Formulierung, umfassend das multifunktionelle Protein nach einem der Ansprüche 1 bis 9 und gegebenenfalls einen pharmazeutisch annehmbaren Träger.

11. Multifunktionelles Protein nach einem der Ansprüche 1 bis 9 zur Verwendung als Medikament.

12. Multifunktionelles Protein nach einem der Ansprüche 1 bis 9 zur Verwendung bei der Behandlung von Krebs.

## Revendications

1. Protéine multifonction, **caractérisée en ce qu'**elle comprend
- exactement un domaine de présentation de l'antigène,
- exactement une région Fc d'anticorps et
- au moins un site de liaison à l'antigène,
le domaine de présentation de l'antigène comprenant dans le sens N- à C-terminal
soit
(i) un peptide provoquant la réponse des lymphocytes T,
(ii) une β2-microglobuline et
(iii) les domaines extracellulaires α1, α2 et α3 d'une molécule du CMH de classe I choisie dans le groupe comprenant HLA-A*0201, HLA-A*1101, HLA-A*2402, HLA-A*340101, HLA-C*0304, HLA-C*0401 et HLA-C*0702,
soit
(i) un peptide provoquant la réponse des lymphocytes T,
(ii) les domaines extracellulaires α1, α2 et α3 d'une molécule du CMH de classe I choisie dans le groupe comprenant HLA-A*0201, HLA-A*1101, HLA-A*2402, HLA-A*340101, HLA-C*0304, HLA-C*0401 et HLA-C*0702, et
(iii) une β2-microglobuline,
le site de liaison à l'antigène se liant au protéoglycane à chondroïtine sulfate associé à un mélanome (MCSP).

2. Protéine multifonction selon la revendication 1, **caractérisée en ce que** la région Fc d'anticorps comprend un premier et un second polypeptide de région Fc liés par un disulfure, le site de liaison à l'antigène comprenant le premier polypeptide de région Fc.

3. Protéine multifonction selon l'une quelconque des revendications 1 à 2, **caractérisée en ce que** le site de liaison à l'antigène comprend i) une paire d'une chaîne lourde d'anticorps et d'une chaîne légère d'anticorps ou ii) un polypeptide de fusion scFv comprenant, dans le sens N- à C-terminal, un fragment d'anticorps scFv et un polypeptide de région Fc d'anticorps ou iii) un polypeptide de fusion scFab comprenant, dans le sens N- à C-terminal, un scFab et un polypeptide de région Fc d'anticorps.

4. Protéine multifonction selon l'une quelconque des revendications 1 à 3, **caractérisée en ce que** i) le domaine de présentation de l'antigène est lié à l'extrémité N-terminale de la chaîne lourde ou à l'extrémité N-terminale de la chaîne légère du site de liaison à l'antigène ou ii) le domaine de présentation de l'antigène est lié à l'extrémité C-terminale de la chaîne lourde ou à l'extrémité C-terminale de la chaîne légère du site de liaison à l'antigène ou iii) le domaine de présentation de l'antigène est lié à l'extrémité N- ou C-terminale du polypeptide de fusion scFv ou iv) le domaine de présentation de l'antigène est lié à l'extrémité N- ou C-terminale du polypeptide de fusion scFab ou iv) le domaine de présentation de l'antigène est lié à l'extrémité N- ou C-terminale du second polypeptide de région Fc.

5. Protéine multifonction selon l'une quelconque des revendications 1 à 4, **caractérisée en ce que** le peptide provoquant une réponse des lymphocytes T est un peptide dérivé d'un virus.

6. Protéine multifonction selon la revendication 5, **caractérisée en ce que** le peptide dérivé d'un virus est un peptide dérivé du cytomégalovirus humain.

7. Protéine multifonction selon l'une quelconque des revendications 1 à 6, **caractérisée en ce que** le peptide dérivé d'un virus a la séquence d'acides aminés SEQ ID NO : 01.

8. Protéine multifonction selon l'une quelconque des revendications 1 à 7, **caractérisée en ce que** le domaine de présentation de l'antigène comprend
(i) un peptide dérivé d'un virus,
(ii) une β2-microglobuline et
(iii) l'allèle d'HLA-A soluble A*0201.

9. Protéine multifonction selon l'une quelconque des revendications 1 à 8, **caractérisée en ce que** le domaine de présentation de l'antigène comprend, dans le sens N- à C-terminal,
(i) un peptide dérivé d'un virus qui a une séquence d'acides aminés choisie dans le groupe comprenant de la SEQ ID NO : 01 à la SEQ ID NO : 70,
(ii) un premier peptide lieur qui a une séquence d'acides aminés choisie dans le groupe comprenant les SEQ ID NO : 77, 78, 79, 82, 83 et 84,
(iii) une β2-microglobuline qui a une séquence d'acides aminés SEQ ID NO : 71,
(iv) un deuxième peptide lieur qui a une séquence d'acides aminés choisie dans le groupe comprenant les SEQ ID NO : 77, 78, 79, 82, 83 et 84,
(v) les domaines extracellulaires α1, α2 et α3 d'une molécule du CMH de classe I qui a une séquence d'acides aminés SEQ ID NO : 72 et
(vi) un troisième peptide lieur qui a une séquence d'acides aminés choisie dans le groupe comprenant les SEQ ID NO : 73, 77, 78, 79, 82, 83, 84 et 136.

10. Formulation pharmaceutique comprenant la protéine multifonction selon l'une quelconque des revendications 1 à 9 et éventuellement un véhicule pharmaceutiquement acceptable.

11. Protéine multifonction selon l'une quelconque des revendications 1 à 9, destinée à être utilisée comme médicament.

12. Protéine multifonction selon l'une quelconque des revendications 1 à 9, destinée à être utilisée dans le traitement d'un cancer.


### SEQUENCE LISTING

<110> Roche Glycart AG
<120> Removal of cancer cells by circulating virus-specific cytotoxic T-cells using cancer cell targeted MHC class I comprising multi-function proteins
<130> 31355 WO
<150> EP121950943
   <151> 2012-11-30
<160> 138
<170> PatentIn version 3.5
<210> 1
   <211> 9
   <212> PRT
   <213> Human cytomegalovirus
<400> 1
   Asn Leu Val Pro Met Val Ala Thr Val
   1               5
<210> 2
   <211> 9
   <212> PRT
   <213> Human cytomegalovirus
<400> 2
   Val Thr Glu His Asp Thr Leu Leu Tyr
   1               5
<210> 3
   <211> 9
   <212> PRT
   <213> Human cytomegalovirus
<400> 3
   Asn Thr Asp Phe Arg Val Leu Glu Leu
   1               5
<210> 4
   <211> 9
   <212> PRT
   <213> Human cytomegalovirus
<400> 4
   Cys Val Glu Thr Met Cys Asn Glu Tyr
   1 5
<210> 5
   <211> 9
   <212> PRT
   <213> Human cytomegalovirus
<400> 5
   Val Leu Glu Glu Thr Ser Val Met Leu
   1 5
<210> 6
   <211> 9
   <212> PRT
   <213> Human cytomegalovirus
<400> 6
   Asn Leu Val Pro Met Val Ala Thr Val
   1 5
<210> 7
   <211> 9
   <212> PRT
   <213> Human cytomegalovirus
<400> 7
   Arg Ile Phe Ala Glu Leu Glu Gly Val
   1 5
<210> 8
   <211> 9
   <212> PRT
   <213> Human cytomegalovirus
<400> 8
   Ile Ile Tyr Thr Arg Asn His Glu Val
   1 5
<210> 9
   <211> 9
   <212> PRT
   <213> Human cytomegalovirus
<400> 9
   Val Leu Ala Glu Leu Val Lys Gln Ile
   1 5
<210> 10
   <211> 9
   <212> PRT
   <213> Human cytomegalovirus
<400> 10
   Ala Val Gly Gly Ala Val Ala Ser Val
   1               5
<210> 11
   <211> 9
   <212> PRT
   <213> Human cytomegalovirus
<400> 11
   Thr Val Arg Ser His Cys Val Ser Lys
   1               5
<210> 12
   <211> 9
   <212> PRT
   <213> Human cytomegalovirus
<400> 12
   Ile Met Arg Glu Phe Asn Ser Tyr Lys
   1               5
<210> 13
   <211> 10
   <212> PRT
   <213> Human cytomegalovirus
<400> 13
   Gly Pro Ile Ser His Gly His Val Leu Lys
   1               5                   10
<210> 14
   <211> 9
   <212> PRT
   <213> Human cytomegalovirus
<400> 14
   Ala Thr Val Gln Gly Gln Asn Leu Lys
   1               5
<210> 15
   <211> 9
   <212> PRT
   <213> Human cytomegalovirus
<400> 15
   Val Tyr Ala Leu Pro Leu Lys Met Leu
   1               5
<210> 16
   <211> 10
   <212> PRT
   <213> Human cytomegalovirus
<400> 16
   Ala Tyr Ala Gln Lys Ile Phe Lys Ile Leu
   1 5 10
<210> 17
   <211> 9
   <212> PRT
   <213> Human cytomegalovirus
<400> 17
   Gln Tyr Asp Pro Val Ala Ala Leu Phe
   1 5
<210> 18
   <211> 9
   <212> PRT
   <213> Human cytomegalovirus
<400> 18
   Tyr Val Lys Val Tyr Leu Glu Ser Phe
   1 5
<210> 19
   <211> 9
   <212> PRT
   <213> Human cytomegalovirus
<400> 19
   Asp Ile Tyr Arg Ile Phe Ala Glu Leu
   1 5
<210> 20
   <211> 10
   <212> PRT
   <213> Human cytomegalovirus
<400> 20
   Val Phe Glu Thr Ser Gly Gly Leu Val Val
   1 5 10
<210> 21
   <211> 9
   <212> PRT
   <213> Human cytomegalovirus
<400> 21
   Lys Ala Arg Asp His Leu Ala Val Leu
   1 5
<210> 22
   <211> 9
   <212> PRT
   <213> Human cytomegalovirus
<400> 22
   Gln Ala Arg Leu Thr Val Ser Gly Leu
   1 5
<210> 23
   <211> 9
   <212> PRT
   <213> Human cytomegalovirus
<400> 23
   Lys Ala Arg Ala Lys Lys Asp Glu Leu
   1 5
<210> 24
   <211> 9
   <212> PRT
   <213> Human cytomegalovirus
<400> 24
   Gln Ile Lys Val Arg Val Asp Met Val
   1 5
<210> 25
   <211> 9
   <212> PRT
   <213> Human cytomegalovirus
<400> 25
   Arg Arg Arg His Arg Gln Asp Ala Leu
   1 5
<210> 26
   <211> 9
   <212> PRT
   <213> Human cytomegalovirus
<400> 26
   Ala Arg Val Tyr Glu Ile Lys Cys Arg
   1 5
<210> 27
   <211> 9
   <212> PRT
   <213> Human cytomegalovirus
<400> 27
   Lys Met Gln Val Ile Gly Asp Gln Tyr
   1 5
<210> 28
   <211> 9
   <212> PRT
   <213> Human cytomegalovirus
<400> 28
   Asn Val Arg Arg Ser Trp Glu Glu Leu
   1 5
<210> 29
   <211> 12
   <212> PRT
   <213> Human cytomegalovirus
<400> 29
   Cys Pro Ser Gln Glu Pro Met Ser Ile Tyr Val Tyr
   1 5 10
<210> 30
   <211> 13
   <212> PRT
   <213> Human cytomegalovirus
<400> 30
   Lys Pro Gly Lys Ile Ser His Ile Met Leu Asp Val Ala
   1 5 10
<210> 31
   <211> 9
   <212> PRT
   <213> Human cytomegalovirus
<400> 31
   Glu Leu Arg Arg Lys Met Met Tyr Met
   1 5
<210> 32
   <211> 9
   <212> PRT
   <213> Human cytomegalovirus
<400> 32
   Ile Pro Ser Ile Asn Val His His Tyr
   1 5
<210> 33
   <211> 9
   <212> PRT
   <213> Human cytomegalovirus
<400> 33
   Phe Glu Gln Pro Thr Glu Thr Pro Pro
   1 5
<210> 34
   <211> 9
   <212> PRT
   <213> Human cytomegalovirus
<400> 34
   Tyr Ala Tyr Ile Tyr Thr Thr Tyr Leu
   1 5
<210> 35
   <211> 11
   <212> PRT
   <213> Human cytomegalovirus
<400> 35
   Gln Glu Phe Phe Trp Asp Ala Asn Asp Ile Tyr
   1 5 10
<210> 36
   <211> 9
   <212> PRT
   <213> Human cytomegalovirus
<400> 36
   Tyr Glu Gln His Lys Ile Thr Ser Tyr
   1 5
<210> 37
   <211> 9
   <212> PRT
   <213> Human cytomegalovirus
<400> 37
   Gln Glu Pro Met Ser Ile Tyr Val Tyr
   1 5
<210> 38
   <211> 10
   <212> PRT
   <213> Human cytomegalovirus
<400> 38
   Ser Glu His Pro Thr Phe Thr Ser Gln Tyr
   1 5 10
<210> 39
   <211> 9
   <212> PRT
   <213> Human cytomegalovirus
<400> 39
   Gln Ala Ile Arg Glu Thr Val Glu Leu
   1               5
<210> 40
   <211> 9
   <212> PRT
   <213> Human cytomegalovirus
<400> 40
   Thr Arg Ala Thr Lys Met Gln Val Ile
   1               5
<210> 41
   <211> 8
   <212> PRT
   <213> Human cytomegalovirus
<400> 41
   Asp Ala Leu Pro Gly Pro Cys Ile
   1               5
<210> 42
   <211> 9
   <212> PRT
   <213> Human cytomegalovirus
<400> 42
   Cys Glu Asp Val Pro Ser Gly Lys Leu
   1               5
<210> 43
   <211> 9
   <212> PRT
   <213> Human cytomegalovirus
<400> 43
   His Glu Arg Asn Gly Phe Thr Val Leu
   1               5
<210> 44
   <211> 13
   <212> PRT
   <213> Human cytomegalovirus
<400> 44
   Pro Thr Phe Thr Ser Gln Tyr Arg Ile Gln Gly Lys Leu
   1               5                   10
<210> 45
   <211> 9
   <212> PRT
   <213> Human cytomegalovirus
<400> 45
   Gln Met Trp Gln Ala Arg Leu Thr Val
   1 5
<210> 46
   <211> 12
   <212> PRT
   <213> Human cytomegalovirus
<400> 46
   His Glu Leu Leu Val Leu Val Lys Lys Ala Gln Leu
   1 5 10
<210> 47
   <211> 12
   <212> PRT
   <213> Human cytomegalovirus
<400> 47
   Asp Asp Tyr Ser Asn Thr His Ser Thr Arg Tyr Val
   1 5 10
<210> 48
   <211> 9
   <212> PRT
   <213> Human immunodeficiency virus
<400> 48
   Ser Leu Tyr Asn Thr Val Ala Thr Leu
   1 5
<210> 49
   <211> 9
   <212> PRT
   <213> Human herpesvirus 4
<400> 49
   Gly Leu Cys Thr Leu Val Ala Met Leu
   1 5
<210> 50
   <211> 9
   <212> PRT
   <213> Influenza A virus
<400> 50
   Gly Ile Leu Gly Phe Val Phe Thr Leu
   1 5
<210> 51
   <211> 9
   <212> PRT
   <213> Hepatitis B virus
<400> 51
   Ser Thr Asn Arg Gln Ser Gly Arg Gln
   1 5
<210> 52
   <211> 9
   <212> PRT
   <213> Human T-cell lymphotropic virus type 1
<400> 52
   Leu Leu Phe Gly Tyr Pro Val Tyr Val
   1 5
<210> 53
   <211> 9
   <212> PRT
   <213> Artificial Sequence
<220>
   <223> V-jun Sarcoma Virus 17 Oncogene Homolog (JUN)
<400> 53
   Phe Ala Glu Gly Phe Val Arg Ala Leu
   1 5
<210> 54
   <211> 9
   <212> PRT
   <213> Human adenovirus type 3
<400> 54
   Leu Ile Val Ile Gly Ile Leu Ile Leu
   1 5
<210> 55
   <211> 8
   <212> PRT
   <213> Hepatitis C virus
<400> 55
   Ile Leu His Thr Pro Gly Cys Val
   1 5
<210> 56
   <211> 9
   <212> PRT
   <213> Dengue virus
<400> 56
   Trp Tyr Ala Gln Ile Gln Pro His Trp
   1               5
<210> 57
   <211> 9
   <212> PRT
   <213> Dengue virus
<400> 57
   Ala Phe Ser Gly Val Ser Trp Thr Met
   1               5
<210> 58
   <211> 9
   <212> PRT
   <213> Dengue virus
<400> 58
   Ile Leu Ile Gly Val Val Ile Thr Trp
   1               5
<210> 59
   <211> 9
   <212> PRT
   <213> Dengue virus
<400> 59
   Met Met Ile Pro Thr Val Val Ala Phe
   1               5
<210> 60
   <211> 9
   <212> PRT
   <213> Dengue virus
<400> 60
   Pro Phe Pro Gln Ser Asn Ala Pro Ile
   1               5
<210> 61
   <211> 9
   <212> PRT
   <213> Dengue virus
<400> 61
   Leu Leu Leu Thr Leu Leu Ala Thr Val
   1               5
<210> 62
   <211> 9
   <212> PRT
   <213> Dengue virus
<400> 62
   Ile Val Leu Glu His Gly Ser Cys Val
   1 5
<210> 63
   <211> 9
   <212> PRT
   <213> Dengue virus
<400> 63
   Leu Leu Phe Lys Thr Glu Asn Gly Val
   1 5
<210> 64
   <211> 9
   <212> PRT
   <213> Dengue virus
<400> 64
   Pro Leu Asn Glu Ala Ile Met Ala Val
   1 5
<210> 65
   <211> 9
   <212> PRT
   <213> Dengue virus
<400> 65
   Asn Leu Val Arg Leu Gln Ser Gly Val
   1 5
<210> 66
   <211> 9
   <212> PRT
   <213> Dengue virus
<400> 66
   Leu Val Ile Ser Gly Leu Phe Pro Val
   1 5
<210> 67
   <211> 9
   <212> PRT
   <213> Dengue virus
<400> 67
   Leu Leu Leu Val Ala His Tyr Ala Ile
   1 5
<210> 68
   <211> 9
   <212> PRT
   <213> Dengue virus
<400> 68
   Leu Ala Leu Leu Ala Ala Phe Lys Val
   1               5
<210> 69
   <211> 9
   <212> PRT
   <213> Dengue virus
<400> 69
   Val Ile Leu Ala Gly Pro Met Pro Val
   1               5
<210> 70
   <211> 9
   <212> PRT
   <213> Dengue virus
<400> 70
   His Val Leu Gly Arg Leu Ile Thr Val
   1               5
<210> 71
   <211> 99
   <212> PRT
   <213> Homo sapiens
<400> 71
<210> 72
   <211> 274
   <212> PRT
   <213> Homo sapiens
<400> 72
<210> 73
   <211> 2
   <212> PRT
   <213> Artificial Sequence
<220>
   <223> linker peptide 1
<400> 73
   Gly Ser
   1
<210> 74
   <211> 3
   <212> PRT
   <213> Artificial Sequence
<220>
   <223> linker peptide 2
<400> 74
   Gly Gly Ser
   1
<210> 75
   <211> 4
   <212> PRT
   <213> Artificial Sequence
<220>
   <223> linker peptide 3
<400> 75
   Gly Gly Gly Ser
   1
<210> 76
   <211> 8
   <212> PRT
   <213> Artificial Sequence
<220>
   <223> linker peptide 4
<400> 76
   Gly Gly Gly Ser Gly Gly Gly Ser
   1               5
<210> 77
   <211> 12
   <212> PRT
   <213> Artificial Sequence
<220>
   <223> linker peptide 5
<400> 77
   Gly Gly Gly Ser Gly Gly Gly Ser Gly Gly Gly Ser
   1               5                   10
<210> 78
   <211> 16
   <212> PRT
   <213> Artificial Sequence
<220>
   <223> linker peptide 6
<400> 78
   Gly Gly Gly Ser Gly Gly Gly Ser Gly Gly Gly Ser Gly Gly Gly Ser
   1               5                   10                  15
<210> 79
   <211> 20
   <212> PRT
   <213> Artificial Sequence
<220>
   <223> linker peptide 7
<400> 79
   Gly Gly Gly Ser Gly Gly Gly Ser Gly Gly Gly Ser Gly Gly Gly Ser
   1               5                   10                  15
   Gly Gly Gly Ser
               20
<210> 80
   <211> 5
   <212> PRT
   <213> Artificial Sequence
<220>
   <223> linker peptide 8
<400> 80
   Gly Gly Gly Gly Ser
   1 5
<210> 81
   <211> 10
   <212> PRT
   <213> Artificial Sequence
<220>
   <223> linker peptide 9
<400> 81
   Gly Gly Gly Gly Ser Gly Gly Gly Gly Ser
   1 5 10
<210> 82
   <211> 15
   <212> PRT
   <213> Artificial Sequence
<220>
   <223> linker peptide 10
<400> 82
   Gly Gly Gly Gly Ser Gly Gly Gly Gly Ser Gly Gly Gly Gly Ser
   1 5 10 15
<210> 83
   <211> 20
   <212> PRT
   <213> Artificial Sequence
<220>
   <223> linker peptide 11
<400> 83
   Gly Gly Gly Gly Ser Gly Gly Gly Gly Ser Gly Gly Gly Gly Ser Gly
   1 5 10 15
   Gly Gly Gly Ser
   20
<210> 84
   <211> 25
   <212> PRT
   <213> Artificial Sequence
<220>
   <223> linker peptide 12
<400> 84
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser
               20                  25
<210> 85
   <211> 107
   <212> PRT
   <213> Homo sapiens
<400> 85
   Ala Pro Glu Leu Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Trp Asp Val Ser His Glu Asp Pro Glu Val Lys Phe Asn Trp Tyr Val
           35                  40                  45
   Asp Gly Val Glu Val His Asn Ala Lys Thr Lys Pro Arg Glu Glu Gln
       50                  55                  60
   Glu Ser Thr Tyr Arg Trp Ser Val Leu Thr Val Leu His Gln Asp Trp
   65                  70                  75                  80
   Leu Asn Gly Lys Glu Tyr Lys Cys Lys Val Ser Asn Lys Ala Leu Pro
                   85                  90                  95
   Ala Pro Ile Glu Lys Thr Ile Ser Lys Ala Lys
               100                 105
<210> 86
   <211> 106
   <212> PRT
   <213> Homo sapiens
<400> 86
<210> 87
   <211> 232
   <212> PRT
   <213> Homo sapiens
<400> 87
   Glu Pro Lys Ser Ala Asp Lys Thr His Thr Cys Pro Pro Cys Pro Ala
   1 5 10 15
   Pro Glu Leu Leu Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Ser Arg Asp Glu Leu Thr
   130 135 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 88
   <211> 232
   <212> PRT
   <213> Artificial Sequence
<220>
   <223> variant human Fc-region of the IgG1 isotype with the mutations L234A, L235A
<400> 88
   Glu Pro Lys Ser Ala Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 89
   <211> 232
   <212> PRT
   <213> Artificial Sequence
<220>
   <223> variant human Fc-region of the IgG1 isotype with a hole mutation
<400> 89
   Glu Pro Lys Ser Ala Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Cys Thr Leu Pro Pro Ser Arg Asp Glu Leu Thr
       130                 135                 140
   Lys Asn Gln Val Ser Leu Ser Cys Ala Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Val
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 90
   <211> 232
   <212> PRT
   <213> Artificial Sequence
<220>
   <223> variant human Fc-region of the IgG1 isotype with a knob mutation
<400> 90
   Glu Pro Lys Ser Ala Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Cys Arg Asp Glu Leu Thr
       130                 135                 140
   Lys Asn Gln Val Ser Leu Trp Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 91
   <211> 232
   <212> PRT
   <213> Artificial Sequence
<220>
   <223> variant human Fc-region of the IgG1 isotype with a L234A, L235A and hole mutation
<400> 91
   Glu Pro Lys Ser Ala Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Cys Thr Leu Pro Pro Ser Arg Asp Glu Leu Thr
   130 135 140
   Lys Asn Gln Val Ser Leu Ser Cys Ala Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Val
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 92
   <211> 232
   <212> PRT
   <213> Artificial Sequence
<220>
   <223> variant human Fc-region of the IgG1 isotype with a L234A, L235A and knob mutation
<400> 92
   Glu Pro Lys Ser Ala Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Cys Arg Asp Glu Leu Thr
       130                 135                 140
   Lys Asn Gln Val Ser Leu Trp Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 93
   <211> 232
   <212> PRT
   <213> Artificial Sequence
<220>
   <223> variant human Fc-region of the IgG1 isotype with a P329G mutation
<400> 93
   Glu Pro Lys Ser Ala Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Gly Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 94
   <211> 232
   <212> PRT
   <213> Artificial Sequence
<220>
   <223> variant human Fc-region of the IgG1 isotype with a L234A, L235A and P329G mutation
<400> 94
   Glu Pro Lys Ser Ala Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Gly Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 95
   <211> 232
   <212> PRT
   <213> Artificial Sequence
<220>
   <223> variant human Fc-region of the IgG1 isotype with a P239G and hole mutation
<400> 95
   Glu Pro Lys Ser Ala Asp Lys Thr His Thr Cys Pro Pro Cys Pro Ala
   1 5 10 15
   Pro Glu Leu Leu Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Gly Ala Pro Ile Glu Lys Thr Ile Ser Lys Ala Lys Gly Gln Pro
   115 120 125
   Arg Glu Pro Gln Val Cys Thr Leu Pro Pro Ser Arg Asp Glu Leu Thr
   130 135 140
   Lys Asn Gln Val Ser Leu Ser Cys Ala Val Lys Gly Phe Tyr Pro Ser
   145 150 155 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Val
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 96
   <211> 232
   <212> PRT
   <213> Artificial Sequence
<220>
   <223> variant human Fc-region of the IgG1 isotype with a P329G and knob mutation
<400> 96
   Glu Pro Lys Ser Ala Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Gly Ala Pro Ile Glu Lys Thr Ile Ser Lys Ala Lys Gly Gln Pro
           115                 120                 125
   Arg Glu Pro Gln Val Tyr Thr Leu Pro Pro Cys Arg Asp Glu Leu Thr
   130 135 140
   Lys Asn Gln Val Ser Leu Trp Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 97
   <211> 232
   <212> PRT
   <213> Artificial Sequence
<220>
   <223> variant human Fc-region of the IgG1 isotype with a L234A, L235A, P329G and hole mutation
<400> 97
   Glu Pro Lys Ser Ala Asp Lys Thr His Thr Cys Pro Pro Cys Pro Ala
   1 5 10 15
   Pro Glu Ala Ala Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
                   100                 105                 110
   Leu Gly Ala Pro Ile Glu Lys Thr Ile Ser Lys Ala Lys Gly Gln Pro
           115                 120                 125
   Arg Glu Pro Gln Val Cys Thr Leu Pro Pro Ser Arg Asp Glu Leu Thr
       130                 135                 140
   Lys Asn Gln Val Ser Leu Ser Cys Ala Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Val
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 98
   <211> 232
   <212> PRT
   <213> Artificial Sequence
<220>
   <223> variant human Fc-region of the IgG1 isotype with a L234A, L235A, P329G and knob mutation
<400> 98
   Glu Pro Lys Ser Ala Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Gly Ala Pro Ile Glu Lys Thr Ile Ser Lys Ala Lys Gly Gln Pro
   115 120 125
   Arg Glu Pro Gln Val Tyr Thr Leu Pro Pro Cys Arg Asp Glu Leu Thr
   130 135 140
   Lys Asn Gln Val Ser Leu Trp Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 99
   <211> 229
   <212> PRT
   <213> Homo sapiens
<400> 99
   Glu Ser Lys Tyr Gly Pro Pro Cys Pro Ser Cys Pro Ala Pro Glu Phe
   1 5 10 15
   Leu Gly Gly Pro Ser Val Phe Leu Phe Pro Pro Lys Pro Lys Asp Thr
   20 25 30
   Leu Met Ile Ser Arg Thr Pro Glu Val Thr Cys Val Val Val Asp Val
   35 40 45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Pro Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Thr Cys Leu Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
                   165                 170                 175
   Pro Pro Val Leu Asp Ser Asp Gly Ser Phe Phe Leu Tyr Ser Arg Leu
               180                 185                 190
   Thr Val Asp Lys Ser Arg Trp Gln Glu Gly Asn Val Phe Ser Cys Ser
           195                 200                 205
   Val Met His Glu Ala Leu His Asn His Tyr Thr Gln Lys Ser Leu Ser
       210                 215                 220
   Leu Ser Leu Gly Lys
   225
<210> 100
   <211> 229
   <212> PRT
   <213> Artificial Sequence
<220>
   <223> variant human Fc-region of the IgG4 isotype with a S228P and L235E mutation
<400> 100
   Glu Ser Lys Tyr Gly Pro Pro Cys Pro Pro Cys Pro Ala Pro Glu Phe
   1               5                   10                  15
   Glu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Pro Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Thr Cys Leu Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
                   165                 170                 175
   Pro Pro Val Leu Asp Ser Asp Gly Ser Phe Phe Leu Tyr Ser Arg Leu
               180                 185                 190
   Thr Val Asp Lys Ser Arg Trp Gln Glu Gly Asn Val Phe Ser Cys Ser
           195                 200                 205
   Val Met His Glu Ala Leu His Asn His Tyr Thr Gln Lys Ser Leu Ser
       210                 215                 220
   Leu Ser Leu Gly Lys
   225
<210> 101
   <211> 229
   <212> PRT
   <213> Artificial Sequence
<220>
   <223> variant human Fc-region of the IgG4 isotype with a S228P, L235E and P329G mutation
<400> 101
   Glu Ser Lys Tyr Gly Pro Pro Cys Pro Pro Cys Pro Ala Pro Glu Phe
   1               5                   10                  15
   Glu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Gly Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Thr Cys Leu Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
                   165                 170                 175
   Pro Pro Val Leu Asp Ser Asp Gly Ser Phe Phe Leu Tyr Ser Arg Leu
               180                 185                 190
   Thr Val Asp Lys Ser Arg Trp Gln Glu Gly Asn Val Phe Ser Cys Ser
           195                 200                 205
   Val Met His Glu Ala Leu His Asn His Tyr Thr Gln Lys Ser Leu Ser
       210                 215                 220
   Leu Ser Leu Gly Lys
   225
<210> 102
   <211> 229
   <212> PRT
   <213> Artificial Sequence
<220>
   <223> variant human Fc-region of the IgG4 isotype with a S228P and L235E mutation
<400> 102
   Glu Ser Lys Tyr Gly Pro Pro Cys Pro Pro Cys Pro Ala Pro Glu Phe
   1               5                   10                  15
   Glu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Gly Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Ser Cys Ala Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
                   165                 170                 175
   Pro Pro Val Leu Asp Ser Asp Gly Ser Phe Phe Leu Val Ser Arg Leu
               180                 185                 190
   Thr Val Asp Lys Ser Arg Trp Gln Glu Gly Asn Val Phe Ser Cys Ser
           195                 200                 205
   Val Met His Glu Ala Leu His Asn His Tyr Thr Gln Lys Ser Leu Ser
       210                 215                 220
   Leu Ser Leu Gly Lys
   225
<210> 103
   <211> 229
   <212> PRT
   <213> Artificial Sequence
<220>
   <223> variant human Fc-region of the IgG4 isotype with a S228P, L235E and P329G mutation
<400> 103
   Glu Ser Lys Tyr Gly Pro Pro Cys Pro Pro Cys Pro Ala Pro Glu Phe
   1               5                   10                  15
   Glu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Gly Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Trp Cys Leu Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
   165 170 175
   Pro Pro Val Leu Asp Ser Asp Gly Ser Phe Phe Leu Tyr Ser Arg Leu
   180 185 190
   Thr Val Asp Lys Ser Arg Trp Gln Glu Gly Asn Val Phe Ser Cys Ser
   195 200 205
   Val Met His Glu Ala Leu His Asn His Tyr Thr Gln Lys Ser Leu Ser
   210 215 220
   Leu Ser Leu Gly Lys
   225
<210> 104
   <211> 11
   <212> PRT
   <213> Artificial Sequence
<220>
   <223> HVR-L1
<400> 104
   Ser Ala Ser Gln Gly Ile Arg Asn Tyr Leu Asn
   1 5 10
<210> 105
   <211> 7
   <212> PRT
   <213> Artificial Sequence
<220>
   <223> HVR-L2
<400> 105
   Tyr Thr Ser Ser Leu His Ser
   1 5
<210> 106
   <211> 9
   <212> PRT
   <213> Artificial Sequence
<220>
   <223> HVR-L3
<400> 106
   Gln Gln Tyr Ser Lys Leu Pro Trp Thr
   1 5
<210> 107
   <211> 11
   <212> PRT
   <213> Artificial Sequence
<220>
   <223> HVR-L1
<400> 107
   Arg Ala Ser Gln Gly Ile Arg Asn Tyr Leu Asn
   1               5                   10
<210> 108
   <211> 11
   <212> PRT
   <213> Artificial Sequence
<220>
   <223> HVR-H1
<400> 108
   Gly Tyr Ser Ile Thr Ser Gly Tyr Tyr Trp Asn
   1               5                   10
<210> 109
   <211> 16
   <212> PRT
   <213> Artificial Sequence
<220>
   <223> HVR-H2
<400> 109
   Tyr Ile Thr Tyr Asp Gly Ser Asn Asn Tyr Asn Pro Ser Leu Lys Asn
   1               5                   10                  15
<210> 110
   <211> 3
   <212> PRT
   <213> Artificial Sequence
<220>
   <223> HVR-H3
<400> 110
   Phe Asp Tyr
   1
<210> 111
   <211> 11
   <212> PRT
   <213> Artificial Sequence
<220>
   <223> HVR-H1
<400> 111
   Gly Gly Ser Ile Thr Ser Gly Tyr Tyr Trp Asn
   1               5                   10
<210> 112
   <211> 16
   <212> PRT
   <213> Artificial Sequence
<220>
   <223> HVR-H2
<400> 112
   Tyr Ile Thr Tyr Asp Gly Ser Asn Asn Tyr Asn Pro Ser Leu Lys Ser
   1               5                   10                  15
<210> 113
   <211> 107
   <212> PRT
   <213> Artificial Sequence
<220>
   <223> LC007 Chimeric Antibody VL
<400> 113
   Asp Ile Val Leu Thr Gln Ser Pro Ser Ser Leu Ser Ala Ser Leu Gly
   1               5                   10                  15
   Asp Arg Val Thr Ile Ser Cys Ser Ala Ser Gln Gly Ile Arg Asn Tyr
               20                  25                  30
   Leu Asn Trp Tyr Gln Gln Arg Pro Asp Gly Thr Val Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Lys Leu Pro Trp
                   85                  90                  95
   Thr Phe Gly Gly Gly Thr Lys Leu Glu Ile Lys
               100                 105
<210> 114
   <211> 112
   <212> PRT
   <213> Artificial Sequence
<220>
   <223> LC007 Chimeric Antibody VH
<400> 114
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
<210> 115
   <211> 107
   <212> PRT
   <213> Artificial Sequence
<220>
   <223> LC007 Humanized Antibody ML2 VL
<400> 115
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Lys Leu Pro Trp
                     85                  90                  95
   Thr Phe Gly Gln Gly Thr Lys Val Glu Ile Lys
               100                 105
<210> 116
   <211> 112
   <212> PRT
   <213> Artificial Sequence
<220>
   <223> LC007 Humanized Antibody M4-3 VH
<400> 116
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
<210> 117
   <211> 862
   <212> PRT
   <213> Artificial Sequence
<220>
   <223> MHC-I-VH (MCSP)-IgGl Fc-region L234A, L235A, P329G, T366S, L368A, Y407V mutant amino acid sequence
<400> 117
   Asn Leu Val Pro Met Val Ala Thr Val Gly Gly Gly Gly Ser Gly Gly
   1               5                   10                  15
   Gly Gly Ser Gly Gly Gly Gly Ser Ile Gln Arg Thr Pro Lys Ile Gln
               20                  25                  30
   Val Tyr Ser Arg His Pro Ala Glu Asn Gly Lys Ser Asn Phe Leu Asn
           35                  40                  45
   Cys Tyr Val Ser Gly Phe His Pro Ser Asp Ile Glu Val Asp Leu Leu
       50                  55                  60
   Lys Asn Gly Glu Arg Ile Glu Lys Val Glu His Ser Asp Leu Ser Phe
   65                  70                  75                  80
   Ser Lys Asp Trp Ser Phe Tyr Leu Leu Tyr Tyr Thr Glu Phe Thr Pro
                   85                  90                  95
   Thr Glu Lys Asp Glu Tyr Ala Cys Arg Val Asn His Val Thr Leu Ser
               100                 105                 110
   Gln Pro Lys Ile Val Lys Trp Asp Arg Asp Met Gly Gly Gly Gly Ser
           115                 120                 125
   Gly Gly Gly Gly Ser Gly Gly Gly Gly Ser Gly Gly Gly Gly Ser Gly
       130                 135                 140
   Ser His Ser Met Arg Tyr Phe Phe Thr Ser Val Ser Arg Pro Gly Arg
   145                 150                 155                 160
   Gly Glu Pro Arg Phe Ile Ala Val Gly Tyr Val Asp Asp Thr Gln Phe
                   165                 170                 175
   Val Arg Phe Asp Ser Asp Ala Ala Ser Gln Arg Met Glu Pro Arg Ala
               180                 185                 190
   Pro Trp Ile Glu Gln Glu Gly Pro Glu Tyr Trp Asp Gly Glu Thr Arg
           195                 200                 205
   Lys Val Lys Ala His Ser Gln Thr His Arg Val Asp Leu Gly Thr Leu
       210                 215                 220
   Arg Gly Tyr Tyr Asn Gln Ser Glu Ala Gly Ser His Thr Val Gln Arg
   225                 230                 235                 240
   Met Tyr Gly Cys Asp Val Gly Ser Asp Trp Arg Phe Leu Arg Gly Tyr
                   245                 250                 255
   His Gln Tyr Ala Tyr Asp Gly Lys Asp Tyr Ile Ala Leu Lys Glu Asp
               260                 265                 270
   Leu Arg Ser Trp Thr Ala Ala Asp Met Ala Ala Gln Thr Thr Lys His
           275                 280                 285
   Lys Trp Glu Ala Ala His Val Ala Glu Gln Leu Arg Ala Tyr Leu Glu
       290                 295                 300
   Gly Thr Cys Val Glu Trp Leu Arg Arg Tyr Leu Glu Asn Gly Lys Glu
   305                 310                 315                 320
   Thr Leu Gln Arg Thr Asp Ala Pro Lys Thr His Met Thr His His Ala
                   325                 330                 335
   Val Ser Asp His Glu Ala Thr Leu Arg Cys Trp Ala Leu Ser Phe Tyr
               340                 345                 350
   Pro Ala Glu Ile Thr Leu Thr Trp Gln Arg Asp Gly Glu Asp Gln Thr
           355                 360                 365
   Gln Asp Thr Glu Leu Val Glu Thr Arg Pro Ala Gly Asp Gly Thr Phe
       370                 375                 380
   Gln Lys Trp Ala Ala Val Val Val Pro Ser Gly Gln Glu Gln Arg Tyr
   385                 390                 395                 400
   Thr Cys His Val Gln His Glu Gly Leu Pro Lys Pro Leu Thr Leu Arg
                   405                 410                 415
   Trp Gly Ser Gly Gln Val Gln Leu Gln Glu Ser Gly Pro Gly Leu Val
               420                 425                 430
   Lys Pro Ser Gln Thr Leu Ser Leu Thr Cys Thr Val Ser Gly Gly Ser
           435                 440                 445
   Ile Thr Ser Gly Tyr Tyr Trp Asn Trp Ile Arg Gln His Pro Gly Lys
       450                 455                 460
   Gly Leu Glu Trp Ile Gly Tyr Ile Thr Tyr Asp Gly Ser Asn Asn Tyr
   465                 470                 475                 480
   Asn Pro Ser Leu Lys Ser Arg Val Thr Ile Ser Arg Asp Thr Ser Lys
                   485                 490                 495
   Asn Gln Phe Ser Leu Lys Leu Ser Ser Val Thr Ala Ala Asp Thr Ala
               500                 505                 510
   Val Tyr Tyr Cys Ala Asp Phe Asp Tyr Trp Gly Gln Gly Thr Leu Val
           515                 520                 525
   Thr Val Ser Ser Ala Ser Thr Lys Gly Pro Ser Val Phe Pro Leu Ala
   530 535 540
   Pro Ser Ser Lys Ser Thr Ser Gly Gly Thr Ala Ala Leu Gly Cys Leu
   545 550 555 560
   Val Lys Asp Tyr Phe Pro Glu Pro Val Thr Val Ser Trp Asn Ser Gly
   565 570 575
   Ala Leu Thr Ser Gly Val His Thr Phe Pro Ala Val Leu Gln Ser Ser
   580 585 590
   Gly Leu Tyr Ser Leu Ser Ser Val Val Thr Val Pro Ser Ser Ser Leu
   595 600 605
   Gly Thr Gln Thr Tyr Ile Cys Asn Val Asn His Lys Pro Ser Asn Thr
   610 615 620
   Lys Val Asp Lys Lys Val Glu Pro Lys Ser Cys Asp Lys Thr His Thr
   625 630 635 640
   Cys Pro Pro Cys Pro Ala Pro Glu Ala Ala Gly Gly Pro Ser Val Phe
   645 650 655
   Leu Phe Pro Pro Lys Pro Lys Asp Thr Leu Met Ile Ser Arg Thr Pro
   660 665 670
   Glu Val Thr Cys Val Val Val Asp Val Ser His Glu Asp Pro Glu Val
   675 680 685
   Lys Phe Asn Trp Tyr Val Asp Gly Val Glu Val His Asn Ala Lys Thr
   690 695 700
   Lys Pro Arg Glu Glu Gln Tyr Asn Ser Thr Tyr Arg Val Val Ser Val
   705 710 715 720
   Leu Thr Val Leu His Gln Asp Trp Leu Asn Gly Lys Glu Tyr Lys Cys
   725 730 735
   Lys Val Ser Asn Lys Ala Leu Gly Ala Pro Ile Glu Lys Thr Ile Ser
   740 745 750
   Lys Ala Lys Gly Gln Pro Arg Glu Pro Gln Val Tyr Thr Leu Pro Pro
   755 760 765
   Ser Arg Asp Glu Leu Thr Lys Asn Gln Val Ser Leu Ser Cys Ala Val
   770 775 780
   Lys Gly Phe Tyr Pro Ser Asp Ile Ala Val Glu Trp Glu Ser Asn Gly
   785                 790                 795                 800
   Gln Pro Glu Asn Asn Tyr Lys Thr Thr Pro Pro Val Leu Asp Ser Asp
                   805                 810                 815
   Gly Ser Phe Phe Leu Val Ser Lys Leu Thr Val Asp Lys Ser Arg Trp
               820                 825                 830
   Gln Gln Gly Asn Val Phe Ser Cys Ser Val Met His Glu Ala Leu His
           835                 840                 845
   Asn His Tyr Thr Gln Lys Ser Leu Ser Leu Ser Pro Gly Lys
       850                 855                 860
<210> 118
   <211> 442
   <212> PRT
   <213> Artificial Sequence
<220>
   <223> VH(MCSP)-IgG1 Fc-region L234A, L235A, P329G, T366W mutant amino acid sequence
<400> 118
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Gly Ala Pro Ile Glu Lys Thr Ile Ser Lys Ala Lys Gly
                   325                 330                 335
   Gln Pro Arg Glu Pro Gln Val Tyr Thr Leu Pro Pro Ser Arg Asp Glu
               340                 345                 350
   Leu Thr Lys Asn Gln Val Ser Leu Trp Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 119
   <211> 214
   <212> PRT
   <213> Artificial Sequence
<220>
   <223> VL(MCSP)-CL amino acid sequence
<400> 119
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Tyr
   20 25 30
   Leu Asn Trp Tyr Gln Gln Lys Pro Gly Lys Ala Pro Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Lys Leu Pro Trp
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 120
   <211> 215
   <212> PRT
   <213> Artificial Sequence
<220>
   <223> Ig Light Chain
<400> 120
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Lys Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Lys Trp Pro Pro
                   85                  90                  95
   Trp Thr Phe Gly Gln Gly Thr Lys Val Glu Ser Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 121
   <211> 448
   <212> PRT
   <213> Artificial Sequence
<220>
   <223> Ig Heavy Chain (IgG1-L234A, L235A mutant)
<400> 121
   Gln Val Glu Leu Val Glu Ser Gly Gly Gly Val Val Gln Pro Gly Arg
   1               5                   10                  15
   Ser Gln Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ile Ile Trp Phe Asp Gly Ser Ser Thr Tyr Tyr Ala Asp Ser Val
       50                  55                  60
   Arg Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Phe Cys
                   85                  90                  95
   Ala Arg Glu Leu Gly Arg Arg Tyr Phe Asp Leu Trp Gly Arg Gly Thr
               100                 105                 110
   Leu Val Ser Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Ala Ala Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Asp Glu Leu Thr Lys Asn Gln Val Ser Leu Thr Cys
           355                 360                 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 122
   <211> 448
   <212> PRT
   <213> Artificial Sequence
<220>
   <223> Ig Heavy Chain (IgG1-L234A, L235A mutant with knob variation)
<400> 122
   Gln Val Glu Leu Val Glu Ser Gly Gly Gly Val Val Gln Pro Gly Arg
   1               5                   10                  15
   Ser Gln Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ile Ile Trp Phe Asp Gly Ser Ser Thr Tyr Tyr Ala Asp Ser Val
       50                  55                  60
   Arg Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Phe Cys
                   85                  90                  95
   Ala Arg Glu Leu Gly Arg Arg Tyr Phe Asp Leu Trp Gly Arg Gly Thr
               100                 105                 110
   Leu Val Ser Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Ala Ala Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Cys Arg Asp Glu Leu Thr Lys Asn Gln Val Ser Leu Trp Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 123
   <211> 448
   <212> PRT
   <213> Artificial Sequence
<220>
   <223> Ig Heavy Chain (IgG1-L234A, L235A mutant with hole variation)
<400> 123
   Gln Val Glu Leu Val Glu Ser Gly Gly Gly Val Val Gln Pro Gly Arg
   1               5                   10                  15
   Ser Gln Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ile Ile Trp Phe Asp Gly Ser Ser Thr Tyr Tyr Ala Asp Ser Val
       50                  55                  60
   Arg Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Phe Cys
                   85                  90                  95
   Ala Arg Glu Leu Gly Arg Arg Tyr Phe Asp Leu Trp Gly Arg Gly Thr
               100                 105                 110
   Leu Val Ser Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Gln Thr Tyr Ile Cys Asn Val Asn His Lys Pro Ser
   195 200 205
   Asn Thr Lys Val Asp Lys Lys Val Glu Pro Lys Ser Cys Asp Lys Thr
   210 215 220
   His Thr Cys Pro Pro Cys Pro Ala Pro Glu Ala Ala Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Cys Thr Leu
   340 345 350
   Pro Pro Ser Arg Asp Glu Leu Thr Lys Asn Gln Val Ser Leu Ser Cys
   355 360 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 124
   <211> 232
   <212> PRT
   <213> Artificial Sequence
<220>
   <223> Ig heavy chain Fc region (IgG1-L234A, L235A mutant Fc-region)
<400> 124
   Glu Pro Lys Ser Ala Asp Lys Thr His Thr Cys Pro Pro Cys Pro Ala
   1 5 10 15
   Pro Glu Ala Ala Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Cys Arg Asp Glu Leu Thr
   130 135 140
   Lys Asn Gln Val Ser Leu Trp Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 125
   <211> 246
   <212> PRT
   <213> Artificial Sequence
<220>
   <223> scFv
<400> 125
   Gln Val Glu Leu Val Glu Ser Gly Gly Gly Val Val Gln Pro Gly Arg
   1               5                   10                  15
   Ser Gln Arg Leu Ser Cys Ala Ala Ser Gly Phe Thr Phe Ser Ser Tyr
               20                  25                  30
   Gly Met His Trp Val Arg Gln Ala Pro Gly Lys Cys Leu Glu Trp Val
           35                  40                  45
   Ala Ile Ile Trp Phe Asp Gly Ser Ser Thr Tyr Tyr Ala Asp Ser Val
       50                  55                  60
   Arg Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Phe Cys
                   85                  90                  95
   Ala Arg Glu Leu Gly Arg Arg Tyr Phe Asp Leu Trp Gly Arg Gly Thr
               100                 105                 110
   Leu Val Ser Val Ser Ser Gly Gly Gly Gly Ser Gly Gly Gly Gly Ser
           115                 120                 125
   Gly Gly Gly Gly Ser Gly Gly Gly Gly Ser Glu Ile Val Leu Thr Gln
       130                 135                 140
   Ser Pro Ala Thr Leu Ser Leu Ser Pro Gly Glu Arg Ala Thr Leu Ser
   145 150 155 160
   Cys Arg Ala Ser Gln Ser Val Ser Ser Tyr Leu Ala Trp Tyr Gln Gln
   165 170 175
   Lys Pro Gly Gln Ala Pro Arg Leu Leu Ile Tyr Asp Ala Ser Lys Arg
   180 185 190
   Ala Thr Gly Ile Pro Ala Arg Phe Ser Gly Ser Gly Ser Gly Thr Asp
   195 200 205
   Phe Thr Leu Thr Ile Ser Ser Leu Glu Pro Glu Asp Phe Ala Val Tyr
   210 215 220
   Tyr Cys Gln Gln Arg Ser Lys Trp Pro Pro Trp Thr Phe Gly Cys Gly
   225 230 235 240
   Thr Lys Val Glu Ser Lys
   245
<210> 126
   <211> 214
   <212> PRT
   <213> Artificial Sequence
<220>
   <223> Murine anti-MCSP monoclonal light chain antibody amino acid sequence (kappa)
<400> 126
   Asp Ile Val Leu Thr Gln Ser Pro Ser Ser Leu Ser Ala Ser Leu Gly
   1 5 10 15
   Asp Arg Val Thr Ile Ser Cys Ser Ala Ser Gln Gly Ile Arg Asn Tyr
   20 25 30
   Leu Asn Trp Tyr Gln Gln Arg Pro Asp Gly Thr Val Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Tyr Ser Leu Thr Ile Ser Asn Leu Glu Pro
   65 70 75 80
   Glu Asp Ile Ala Thr Tyr Tyr Cys Gln Gln Tyr Ser Lys Leu Pro Trp
   85 90 95
   Thr Phe Gly Gly Gly Thr Lys Leu Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 127
   <211> 214
   <212> PRT
   <213> Artificial Sequence
<220>
   <223> Humanized anti-MCSP monoclonal light chain antibody amino acid sequence (kappa)
<400> 127
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Tyr
   20 25 30
   Leu Asn Trp Tyr Gln Gln Lys Pro Gly Lys Ala Pro Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Lys Leu Pro Trp
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 128
   <211> 442
   <212> PRT
   <213> Artificial Sequence
<220>
   <223> Murine anti-MCSP monoclonal heavy chain antibody amino acid sequence (IgG1 L234A, L235A mutant)
<400> 128
   Glu Val Gln Leu Gln Glu Ser Gly Pro Gly Leu Val Lys Pro Ser Gln
   1 5 10 15
   Ser Leu Ser Leu Thr Cys Ser Val Thr Gly Tyr Ser Ile Thr Ser Gly
   20 25 30
   Tyr Tyr Trp Asn Trp Ile Arg Gln Phe Pro Gly Asn Lys Leu Glu Trp
   35 40 45
   Met Gly Tyr Ile Thr Tyr Asp Gly Ser Asn Asn Tyr Asn Pro Ser Leu
   50 55 60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
   325 330 335
   Gln Pro Arg Glu Pro Gln Val Tyr Thr Leu Pro Pro Ser Arg Asp Glu
   340 345 350
   Leu Thr Lys Asn Gln Val Ser Leu Thr Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 129
   <211> 442
   <212> PRT
   <213> Artificial Sequence
<220>
   <223> Humanized anti-MCSP monoclonal heavy chain antibody amino acid sequence (IgG1 L234A, L235A mutant)
<400> 129
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Thr Ser Gly
   20 25 30
   Tyr Tyr Trp Asn Trp Ile Arg Gln His Pro Gly Lys Gly Leu Glu Trp
   35 40 45
   Ile Gly Tyr Ile Thr Tyr Asp Gly Ser Asn Asn Tyr Asn Pro Ser Leu
   50 55 60
   Lys Ser Arg Val Thr Ile Ser Arg Asp Thr Ser Lys Asn Gln Phe Ser
   65 70 75 80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
   325 330 335
   Gln Pro Arg Glu Pro Gln Val Tyr Thr Leu Pro Pro Ser Arg Asp Glu
   340 345 350
   Leu Thr Lys Asn Gln Val Ser Leu Thr Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 130
   <211> 442
   <212> PRT
   <213> Artificial Sequence
<220>
   <223> Murine anti-MCSP monoclonal heavy chain antibody amino acid sequence (IgG1 L234A, L235A mutant and knob variant)
<400> 130
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
   85 90 95
   Ala Asp Phe Asp Tyr Trp Gly Gln Gly Thr Thr Leu Thr Val Ser Ser
   100 105 110
   Ala Ser Thr Lys Gly Pro Ser Val Phe Pro Leu Ala Pro Ser Ser Lys
   115 120 125
   Ser Thr Ser Gly Gly Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
   180 185 190
   Tyr Ile Cys Asn Val Asn His Lys Pro Ser Asn Thr Lys Val Asp Lys
   195 200 205
   Lys Val Glu Pro Lys Ser Cys Asp Lys Thr His Thr Cys Pro Pro Cys
   210 215 220
   Pro Ala Pro Glu Ala Ala Gly Gly Pro Ser Val Phe Leu Phe Pro Pro
   225 230 235 240
   Lys Pro Lys Asp Thr Leu Met Ile Ser Arg Thr Pro Glu Val Thr Cys
   245 250 255
   Val Val Val Asp Val Ser His Glu Asp Pro Glu Val Lys Phe Asn Trp
   260 265 270
   Tyr Val Asp Gly Val Glu Val His Asn Ala Lys Thr Lys Pro Arg Glu
   275 280 285
   Glu Gln Tyr Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Ala Leu Pro Ala Pro Ile Glu Lys Thr Ile Ser Lys Ala Lys Gly
   325 330 335
   Gln Pro Arg Glu Pro Gln Val Tyr Thr Leu Pro Pro Cys Arg Asp Glu
               340                 345                 350
   Leu Thr Lys Asn Gln Val Ser Leu Trp Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440
<210> 131
   <211> 442
   <212> PRT
   <213> Artificial Sequence
<220>
   <223> Humanized anti-MCSP monoclonal heavy chain antibody amino acid sequence (IgG1 L234A, L235A mutant and knob variant)
<400> 131
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Tyr Thr Leu Pro Pro Cys Arg Asp Glu
               340                 345                 350
   Leu Thr Lys Asn Gln Val Ser Leu Trp Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 132
   <211> 442
   <212> PRT
   <213> Artificial Sequence
<220>
   <223> Murine anti-MCSP monoclonal heavy chain antibody amino acid sequence (IgG1 L234A, L235A mutant and hole variant)
<400> 132
   Glu Val Gln Leu Gln Glu Ser Gly Pro Gly Leu Val Lys Pro Ser Gln
   1 5 10 15
   Ser Leu Ser Leu Thr Cys Ser Val Thr Gly Tyr Ser Ile Thr Ser Gly
   20 25 30
   Tyr Tyr Trp Asn Trp Ile Arg Gln Phe Pro Gly Asn Lys Leu Glu Trp
   35 40 45
   Met Gly Tyr Ile Thr Tyr Asp Gly Ser Asn Asn Tyr Asn Pro Ser Leu
   50 55 60
   Lys Asn Arg Ile Ser Ile Thr Arg Asp Thr Ser Lys Asn Gln Phe Phe
   65 70 75 80
   Leu Lys Leu Asn Ser Val Thr Thr Glu Asp Thr Ala Thr Tyr Tyr Cys
   85 90 95
   Ala Asp Phe Asp Tyr Trp Gly Gln Gly Thr Thr Leu Thr Val Ser Ser
   100 105 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Cys Thr Leu Pro Pro Ser Arg Asp Glu
               340                 345                 350
   Leu Thr Lys Asn Gln Val Ser Leu Ser Cys Ala Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Val Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440
<210> 133
   <211> 442
   <212> PRT
   <213> Artificial Sequence
<220>
   <223> Humanized anti-MCSP monoclonal heavy chain antibody amino acid sequence (IgG1 L234A, L235A mutant and hole variant)
<400> 133
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Cys Thr Leu Pro Pro Ser Arg Asp Glu
               340                 345                 350
   Leu Thr Lys Asn Gln Val Ser Leu Ser Cys Ala Val Lys Gly Phe Tyr
           355                 360                 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Val Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 134
   <211> 239
   <212> PRT
   <213> Artificial Sequence
<220>
   <223> Disulfide-stabilized single chain Fv of murine anti-MCSP monoclonal antibody
<400> 134
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Gly Gly Gly Gly Ser Gly Gly Gly Gly Ser Gly Gly Gly Gly Ser Gly
           115                 120                 125
   Gly Gly Gly Ser Asp Ile Val Leu Thr Gln Ser Pro Ser Ser Leu Ser
       130                 135                 140
   Ala Ser Leu Gly Asp Arg Val Thr Ile Ser Cys Ser Ala Ser Gln Gly
   145                 150                 155                 160
   Ile Arg Asn Tyr Leu Asn Trp Tyr Gln Gln Arg Pro Asp Gly Thr Val
                   165                 170                 175
   Lys Leu Leu Ile Tyr Tyr Thr Ser Ser Leu His Ser Gly Val Pro Ser
               180                 185                 190
   Arg Phe Ser Gly Ser Gly Ser Gly Thr Asp Tyr Ser Leu Thr Ile Ser
           195                 200                 205
   Asn Leu Glu Pro Glu Asp Ile Ala Thr Tyr Tyr Cys Gln Gln Tyr Ser
       210                 215                 220
   Lys Leu Pro Trp Thr Phe Gly Gly Gly Thr Lys Leu Glu Ile Lys
   225                 230                 235
<210> 135
   <211> 239
   <212> PRT
   <213> Artificial Sequence
<220>
   <223> Disulfide-stabilized single chain Fv of humanized anti-MCSP monoclonal antibody
<400> 135
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
   100 105 110
   Gly Gly Gly Gly Ser Gly Gly Gly Gly Ser Gly Gly Gly Gly Ser Gly
   115 120 125
   Gly Gly Gly Ser Asp Ile Gln Met Thr Gln Ser Pro Ser Ser Leu Ser
   130 135 140
   Ala Ser Val Gly Asp Arg Val Thr Ile Thr Cys Arg Ala Ser Gln Gly
   145 150 155 160
   Ile Arg Asn Tyr Leu Asn Trp Tyr Gln Gln Lys Pro Gly Lys Ala Pro
   165 170 175
   Lys Leu Leu Ile Tyr Tyr Thr Ser Ser Leu His Ser Gly Val Pro Ser
   180 185 190
   Arg Phe Ser Gly Ser Gly Ser Gly Thr Asp Phe Thr Leu Thr Ile Ser
   195 200 205
   Ser Leu Gln Pro Glu Asp Phe Ala Thr Tyr Tyr Cys Gln Gln Tyr Ser
   210 215 220
   Lys Leu Pro Trp Thr Phe Gly Gln Gly Thr Lys Val Glu Ile Lys
   225 230 235
<210> 136
   <211> 3
   <212> PRT
   <213> Artificial Sequence
<220>
   <223> linker peptide 13
<400> 136
   Gly Ser Gly
   1
<210> 137
   <211> 862
   <212> PRT
   <213> Artificial Sequence
<220>
   <223> disulfide stabilized MHC-I-VH (MCSP)-IgGl Fc-region L234A, L235A, P329G, T366S, L368A, Y407V mutant amino acid sequence
<400> 137
<210> 138
   <211> 2322
   <212> PRT
   <213> Homo sapiens
<400> 138
   Met Gln Ser Gly Pro Arg Pro Pro Leu Pro Ala Pro Gly Leu Ala Leu
   1               5                   10                  15
   Ala Leu Thr Leu Thr Met Leu Ala Arg Leu Ala Ser Ala Ala Ser Phe
               20                  25                  30
   Phe Gly Glu Asn His Leu Glu Val Pro Val Ala Thr Ala Leu Thr Asp
           35                  40                  45
   Ile Asp Leu Gln Leu Gln Phe Ser Thr Ser Gln Pro Glu Ala Leu Leu
       50                  55                  60
   Leu Leu Ala Ala Gly Pro Ala Asp His Leu Leu Leu Gln Leu Tyr Ser
   65                  70                  75                  80
   Gly Arg Leu Gln Val Arg Leu Val Leu Gly Gln Glu Glu Leu Arg Leu
                   85                  90                  95
   Gln Thr Pro Ala Glu Thr Leu Leu Ser Asp Ser Ile Pro His Thr Val
               100                 105                 110
   Val Leu Thr Val Val Glu Gly Trp Ala Thr Leu Ser Val Asp Gly Phe
           115                 120                 125
   Leu Asn Ala Ser Ser Ala Val Pro Gly Ala Pro Leu Glu Val Pro Tyr
       130                 135                 140
   Gly Leu Phe Val Gly Gly Thr Gly Thr Leu Gly Leu Pro Tyr Leu Arg
   145                 150                 155                 160
   Gly Thr Ser Arg Pro Leu Arg Gly Cys Leu His Ala Ala Thr Leu Asn
                   165                 170                 175
   Gly Arg Ser Leu Leu Arg Pro Leu Thr Pro Asp Val His Glu Gly Cys
               180                 185                 190
   Ala Glu Glu Phe Ser Ala Ser Asp Asp Val Ala Leu Gly Phe Ser Gly
           195                 200                 205
   Pro His Ser Leu Ala Ala Phe Pro Ala Trp Gly Thr Gln Asp Glu Gly
       210                 215                 220
   Thr Leu Glu Phe Thr Leu Thr Thr Gln Ser Arg Gln Ala Pro Leu Ala
   225                 230                 235                 240
   Phe Gln Ala Gly Gly Arg Arg Gly Asp Phe Ile Tyr Val Asp Ile Phe
                   245                 250                 255
   Glu Gly His Leu Arg Ala Val Val Glu Lys Gly Gln Gly Thr Val Leu
               260                 265                 270
   Leu His Asn Ser Val Pro Val Ala Asp Gly Gln Pro His Glu Val Ser
           275                 280                 285
   Val His Ile Asn Ala His Arg Leu Glu Ile Ser Val Asp Gln Tyr Pro
       290                 295                 300
   Thr His Thr Ser Asn Arg Gly Val Leu Ser Tyr Leu Glu Pro Arg Gly
   305                 310                 315                 320
   Ser Leu Leu Leu Gly Gly Leu Asp Ala Glu Ala Ser Arg His Leu Gln
                   325                 330                 335
   Glu His Arg Leu Gly Leu Thr Pro Glu Ala Thr Asn Ala Ser Leu Leu
               340                 345                 350
   Gly Cys Met Glu Asp Leu Ser Val Asn Gly Gln Arg Arg Gly Leu Arg
           355                 360                 365
   Glu Ala Leu Leu Thr Arg Asn Met Ala Ala Gly Cys Arg Leu Glu Glu
       370                 375                 380
   Glu Glu Tyr Glu Asp Asp Ala Tyr Gly His Tyr Glu Ala Phe Ser Thr
   385 390 395 400
   Leu Ala Pro Glu Ala Trp Pro Ala Met Glu Leu Pro Glu Pro Cys Val
   405 410 415
   Pro Glu Pro Gly Leu Pro Pro Val Phe Ala Asn Phe Thr Gln Leu Leu
   420 425 430
   Thr Ile Ser Pro Leu Val Val Ala Glu Gly Gly Thr Ala Trp Leu Glu
   435 440 445
   Trp Arg His Val Gln Pro Thr Leu Asp Leu Met Glu Ala Glu Leu Arg
   450 455 460
   Lys Ser Gln Val Leu Phe Ser Val Thr Arg Gly Ala Arg His Gly Glu
   465 470 475 480
   Leu Glu Leu Asp Ile Pro Gly Ala Gln Ala Arg Lys Met Phe Thr Leu
   485 490 495
   Leu Asp Val Val Asn Arg Lys Ala Arg Phe Ile His Asp Gly Ser Glu
   500 505 510
   Asp Thr Ser Asp Gln Leu Val Leu Glu Val Ser Val Thr Ala Arg Val
   515 520 525
   Pro Met Pro Ser Cys Leu Arg Arg Gly Gln Thr Tyr Leu Leu Pro Ile
   530 535 540
   Gln Val Asn Pro Val Asn Asp Pro Pro His Ile Ile Phe Pro His Gly
   545 550 555 560
   Ser Leu Met Val Ile Leu Glu His Thr Gln Lys Pro Leu Gly Pro Glu
   565 570 575
   Val Phe Gln Ala Tyr Asp Pro Asp Ser Ala Cys Glu Gly Leu Thr Phe
   580 585 590
   Gln Val Leu Gly Thr Ser Ser Gly Leu Pro Val Glu Arg Arg Asp Gln
   595 600 605
   Pro Gly Glu Pro Ala Thr Glu Phe Ser Cys Arg Glu Leu Glu Ala Gly
   610 615 620
   Ser Leu Val Tyr Val His Arg Gly Gly Pro Ala Gln Asp Leu Thr Phe
   625 630 635 640
   Arg Val Ser Asp Gly Leu Gln Ala Ser Pro Pro Ala Thr Leu Lys Val
   645 650 655
   Val Ala Ile Arg Pro Ala Ile Gln Ile His Arg Ser Thr Gly Leu Arg
   660 665 670
   Leu Ala Gln Gly Ser Ala Met Pro Ile Leu Pro Ala Asn Leu Ser Val
   675 680 685
   Glu Thr Asn Ala Val Gly Gln Asp Val Ser Val Leu Phe Arg Val Thr
   690 695 700
   Gly Ala Leu Gln Phe Gly Glu Leu Gln Lys Gln Gly Ala Gly Gly Val
   705 710 715 720
   Glu Gly Ala Glu Trp Trp Ala Thr Gln Ala Phe His Gln Arg Asp Val
   725 730 735
   Glu Gln Gly Arg Val Arg Tyr Leu Ser Thr Asp Pro Gln His His Ala
   740 745 750
   Tyr Asp Thr Val Glu Asn Leu Ala Leu Glu Val Gln Val Gly Gln Glu
   755 760 765
   Ile Leu Ser Asn Leu Ser Phe Pro Val Thr Ile Gln Arg Ala Thr Val
   770 775 780
   Trp Met Leu Arg Leu Glu Pro Leu His Thr Gln Asn Thr Gln Gln Glu
   785 790 795 800
   Thr Leu Thr Thr Ala His Leu Glu Ala Thr Leu Glu Glu Ala Gly Pro
   805 810 815
   Ser Pro Pro Thr Phe His Tyr Glu Val Val Gln Ala Pro Arg Lys Gly
   820 825 830
   Asn Leu Gln Leu Gln Gly Thr Arg Leu Ser Asp Gly Gln Gly Phe Thr
   835 840 845
   Gln Asp Asp Ile Gln Ala Gly Arg Val Thr Tyr Gly Ala Thr Ala Arg
   850 855 860
   Ala Ser Glu Ala Val Glu Asp Thr Phe Arg Phe Arg Val Thr Ala Pro
   865 870 875 880
   Pro Tyr Phe Ser Pro Leu Tyr Thr Phe Pro Ile His Ile Gly Gly Asp
                   885                 890                 895
   Pro Asp Ala Pro Val Leu Thr Asn Val Leu Leu Val Val Pro Glu Gly
               900                 905                 910
   Gly Glu Gly Val Leu Ser Ala Asp His Leu Phe Val Lys Ser Leu Asn
           915                 920                 925
   Ser Ala Ser Tyr Leu Tyr Glu Val Met Glu Arg Pro Arg His Gly Arg
       930                 935                 940
   Leu Ala Trp Arg Gly Thr Gln Asp Lys Thr Thr Met Val Thr Ser Phe
   945                 950                 955                 960
   Thr Asn Glu Asp Leu Leu Arg Gly Arg Leu Val Tyr Gln His Asp Asp
                   965                 970                 975
   Ser Glu Thr Thr Glu Asp Asp Ile Pro Phe Val Ala Thr Arg Gln Gly
               980                 985                 990
   Glu Ser Ser Gly Asp Met Ala Trp  Glu Glu Val Arg Gly  Val Phe Arg
           995                 1000                1005
   Val Ala  Ile Gln Pro Val Asn  Asp His Ala Pro Val  Gln Thr Ile
       1010                 1015                 1020
   Ser Arg  Ile Phe His Val Ala  Arg Gly Gly Arg Arg  Leu Leu Thr
       1025                 1030                 1035
   Thr Asp  Asp Val Ala Phe Ser  Asp Ala Asp Ser Gly  Phe Ala Asp
       1040                 1045                 1050
   Ala Gln  Leu Val Leu Thr Arg  Lys Asp Leu Leu Phe  Gly Ser Ile
       1055                 1060                 1065
   Val Ala  Val Asp Glu Pro Thr  Arg Pro Ile Tyr Arg  Phe Thr Gln
       1070                 1075                 1080
   Glu Asp  Leu Arg Lys Arg Arg  Val Leu Phe Val His  Ser Gly Ala
       1085                 1090                 1095
   Asp Arg  Gly Trp Ile Gln Leu  Gln Val Ser Asp Gly  Gln His Gln
       1100                 1105                 1110
   Ala Thr  Ala Leu Leu Glu Val  Gln Ala Ser Glu Pro  Tyr Leu Arg
       1115                 1120                 1125
   Val Ala  Asn Gly Ser Ser Leu  Val Val Pro Gln Gly  Gly Gln Gly
       1130                 1135                 1140
   Thr Ile  Asp Thr Ala Val Leu  His Leu Asp Thr Asn  Leu Asp Ile
       1145                 1150                 1155
   Arg Ser  Gly Asp Glu Val His  Tyr His Val Thr Ala  Gly Pro Arg
       1160                 1165                 1170
   Trp Gly  Gln Leu Val Arg Ala  Gly Gln Pro Ala Thr  Ala Phe Ser
       1175                 1180                 1185
   Gln Gln  Asp Leu Leu Asp Gly  Ala Val Leu Tyr Ser  His Asn Gly
       1190                 1195                 1200
   Ser Leu  Ser Pro Arg Asp Thr  Met Ala Phe Ser Val  Glu Ala Gly
       1205                 1210                 1215
   Pro Val  His Thr Asp Ala Thr  Leu Gln Val Thr Ile  Ala Leu Glu
       1220                 1225                 1230
   Gly Pro  Leu Ala Pro Leu Lys  Leu Val Arg His Lys  Lys Ile Tyr
       1235                 1240                 1245
   Val Phe  Gln Gly Glu Ala Ala  Glu Ile Arg Arg Asp  Gln Leu Glu
       1250                 1255                 1260
   Ala Ala  Gln Glu Ala Val Pro  Pro Ala Asp Ile Val  Phe Ser Val
       1265                 1270                 1275
   Lys Ser  Pro Pro Ser Ala Gly  Tyr Leu Val Met Val  Ser Arg Gly
       1280                 1285                 1290
   Ala Leu  Ala Asp Glu Pro Pro  Ser Leu Asp Pro Val  Gln Ser Phe
       1295                 1300                 1305
   Ser Gln  Glu Ala Val Asp Thr  Gly Arg Val Leu Tyr  Leu His Ser
       1310                 1315                 1320
   Arg Pro  Glu Ala Trp Ser Asp  Ala Phe Ser Leu Asp  Val Ala Ser
       1325                 1330                 1335
   Gly Leu  Gly Ala Pro Leu Glu  Gly Val Leu Val Glu  Leu Glu Val
       1340                 1345                 1350
   Leu Pro  Ala Ala Ile Pro Leu  Glu Ala Gln Asn Phe  Ser Val Pro
       1355                 1360                 1365
   Glu Gly  Gly Ser Leu Thr Leu  Ala Pro Pro Leu Leu  Arg Val Ser
       1370                 1375                 1380
   Gly Pro  Tyr Phe Pro Thr Leu  Leu Gly Leu Ser Leu  Gln Val Leu
       1385                 1390                 1395
   Glu Pro  Pro Gln His Gly Ala  Leu Gln Lys Glu Asp  Gly Pro Gln
       1400                 1405                 1410
   Ala Arg  Thr Leu Ser Ala Phe  Ser Trp Arg Met Val  Glu Glu Gln
       1415                 1420                 1425
   Leu Ile  Arg Tyr Val His Asp  Gly Ser Glu Thr Leu  Thr Asp Ser
       1430                 1435                 1440
   Phe Val  Leu Met Ala Asn Ala  Ser Glu Met Asp Arg  Gln Ser His
       1445                 1450                 1455
   Pro Val  Ala Phe Thr Val Thr  Val Leu Pro Val Asn  Asp Gln Pro
       1460                 1465                 1470
   Pro Ile  Leu Thr Thr Asn Thr  Gly Leu Gln Met Trp  Glu Gly Ala
       1475                 1480                 1485
   Thr Ala  Pro Ile Pro Ala Glu  Ala Leu Arg Ser Thr  Asp Gly Asp
       1490                 1495                 1500
   Ser Gly  Ser Glu Asp Leu Val  Tyr Thr Ile Glu Gln  Pro Ser Asn
       1505                 1510                 1515
   Gly Arg  Val Val Leu Arg Gly  Ala Pro Gly Thr Glu  Val Arg Ser
       1520                 1525                 1530
   Phe Thr  Gln Ala Gln Leu Asp  Gly Gly Leu Val Leu  Phe Ser His
       1535                 1540                 1545
   Arg Gly  Thr Leu Asp Gly Gly  Phe Arg Phe Arg Leu  Ser Asp Gly
       1550                 1555                 1560
   Glu His  Thr Ser Pro Gly His  Phe Phe Arg Val Thr  Ala Gln Lys
       1565                 1570                 1575
   Gln Val  Leu Leu Ser Leu Lys  Gly Ser Gln Thr Leu  Thr Val Cys
       1580                 1585                 1590
   Pro Gly  Ser Val Gln Pro Leu  Ser Ser Gln Thr Leu  Arg Ala Ser
       1595                 1600                 1605
   Ser Ser Ala Gly Thr Asp Pro Gln Leu Leu Leu Tyr Arg Val Val
       1610                1615                1620
   Arg Gly Pro Gln Leu Gly Arg Leu Phe His Ala Gln Gln Asp Ser
       1625                1630                1635
   Thr Gly Glu Ala Leu Val Asn Phe Thr Gln Ala Glu Val Tyr Ala
       1640                1645                1650
   Gly Asn Ile Leu Tyr Glu His Glu Met Pro Pro Glu Pro Phe Trp
       1655                1660                1665
   Glu Ala His Asp Thr Leu Glu Leu Gln Leu Ser Ser Pro Pro Ala
       1670                1675                1680
   Arg Asp Val Ala Ala Thr Leu Ala Val Ala Val Ser Phe Glu Ala
       1685                1690                1695
   Ala Cys Pro Gln Arg Pro Ser His Leu Trp Lys Asn Lys Gly Leu
       1700                1705                1710
   Trp Val Pro Glu Gly Gln Arg Ala Arg Ile Thr Val Ala Ala Leu
       1715                1720                1725
   Asp Ala Ser Asn Leu Leu Ala Ser Val Pro Ser Pro Gln Arg Ser
       1730                1735                1740
   Glu His Asp Val Leu Phe Gln Val Thr Gln Phe Pro Ser Arg Gly
       1745                1750                1755
   Gln Leu Leu Val Ser Glu Glu Pro Leu His Ala Gly Gln Pro His
       1760                1765                1770
   Phe Leu Gln Ser Gln Leu Ala Ala Gly Gln Leu Val Tyr Ala His
       1775                1780                1785
   Gly Gly Gly Gly Thr Gln Gln Asp Gly Phe His Phe Arg Ala His
       1790                1795                1800
   Leu Gln Gly Pro Ala Gly Ala Ser Val Ala Gly Pro Gln Thr Ser
       1805                1810                1815
   Glu Ala Phe Ala Ile Thr Val Arg Asp Val Asn Glu Arg Pro Pro
       1820                1825                1830
   Gln Pro Gln Ala Ser Val Pro Leu Arg Leu Thr Arg Gly Ser Arg
   1835 1840 1845
   Ala Pro Ile Ser Arg Ala Gln Leu Ser Val Val Asp Pro Asp Ser
   1850 1855 1860
   Ala Pro Gly Glu Ile Glu Tyr Glu Val Gln Arg Ala Pro His Asn
   1865 1870 1875
   Gly Phe Leu Ser Leu Val Gly Gly Gly Leu Gly Pro Val Thr Arg
   1880 1885 1890
   Phe Thr Gln Ala Asp Val Asp Ser Gly Arg Leu Ala Phe Val Ala
   1895 1900 1905
   Asn Gly Ser Ser Val Ala Gly Ile Phe Gln Leu Ser Met Ser Asp
   1910 1915 1920
   Gly Ala Ser Pro Pro Leu Pro Met Ser Leu Ala Val Asp Ile Leu
   1925 1930 1935
   Pro Ser Ala Ile Glu Val Gln Leu Arg Ala Pro Leu Glu Val Pro
   1940 1945 1950
   Gln Ala Leu Gly Arg Ser Ser Leu Ser Gln Gln Gln Leu Arg Val
   1955 1960 1965
   Val Ser Asp Arg Glu Glu Pro Glu Ala Ala Tyr Arg Leu Ile Gln
   1970 1975 1980
   Gly Pro Gln Tyr Gly His Leu Leu Val Gly Gly Arg Pro Thr Ser
   1985 1990 1995
   Ala Phe Ser Gln Phe Gln Ile Asp Gln Gly Glu Val Val Phe Ala
   2000 2005 2010
   Phe Thr Asn Phe Ser Ser Ser His Asp His Phe Arg Val Leu Ala
   2015 2020 2025
   Leu Ala Arg Gly Val Asn Ala Ser Ala Val Val Asn Val Thr Val
   2030 2035 2040
   Arg Ala Leu Leu His Val Trp Ala Gly Gly Pro Trp Pro Gln Gly
   2045 2050 2055
   Ala Thr Leu Arg Leu Asp Pro Thr Val Leu Asp Ala Gly Glu Leu
   2060 2065 2070
   Ala Asn Arg Thr Gly Ser Val Pro Arg Phe Arg Leu Leu Glu Gly
       2075                2080                2085
   Pro Arg His Gly Arg Val Val Arg Val Pro Arg Ala Arg Thr Glu
       2090                2095                2100
   Pro Gly Gly Ser Gln Leu Val Glu Gln Phe Thr Gln Gln Asp Leu
       2105                2110                2115
   Glu Asp Gly Arg Leu Gly Leu Glu Val Gly Arg Pro Glu Gly Arg
       2120                2125                2130
   Ala Pro Gly Pro Ala Gly Asp Ser Leu Thr Leu Glu Leu Trp Ala
       2135                2140                2145
   Gln Gly Val Pro Pro Ala Val Ala Ser Leu Asp Phe Ala Thr Glu
       2150                2155                2160
   Pro Tyr Asn Ala Ala Arg Pro Tyr Ser Val Ala Leu Leu Ser Val
       2165                2170                2175
   Pro Glu Ala Ala Arg Thr Glu Ala Gly Lys Pro Glu Ser Ser Thr
       2180                2185                2190
   Pro Thr Gly Glu Pro Gly Pro Met Ala Ser Ser Pro Glu Pro Ala
       2195                2200                2205
   Val Ala Lys Gly Gly Phe Leu Ser Phe Leu Glu Ala Asn Met Phe
       2210                2215                2220
   Ser Val Ile Ile Pro Met Cys Leu Val Leu Leu Leu Leu Ala Leu
       2225                2230                2235
   Ile Leu Pro Leu Leu Phe Tyr Leu Arg Lys Arg Asn Lys Thr Gly
       2240                2245                2250
   Lys His Asp Val Gln Val Leu Thr Ala Lys Pro Arg Asn Gly Leu
       2255                2260                2265
   Ala Gly Asp Thr Glu Thr Phe Arg Lys Val Glu Pro Gly Gln Ala
       2270                2275                2280
   Ile Pro Leu Thr Ala Val Pro Gly Gln Gly Pro Pro Pro Gly Gly
       2285                2290                2295
   Gln Pro Asp Pro Glu Leu Leu Gln Phe Cys Arg Thr Pro Asn Pro
       2300                2305                2310
   Ala Leu  Lys Asn Gly Gln Tyr  Trp Val
       2315                 2320